# EUROPEAN PATENT SPECIFICATION

(11) **EP 0 750 044 B1**
(45) Date of publication and mention of the grant of the patent: **07.08.2002**
(21) Application number: 96202037.6
(22) Date of filing: 05.11.1992
(51) Int. Cl.: C12N 15/85, A61K 48/00, C12N 5/10

(54) **DNA constructs and transfection of cells therewith for homologous recombination**
DNA-Konstrukte und ihre Verwendung zur Transfektion von Zellen durch homologe Rekombination
Constructions d'ADN et leur utilisation dans la transfection par recombinaison homologue de cellules

(30) Priority: 05.11.1991 US 787840; 05.11.1991 US 789188; 10.07.1992 US 911533
(43) Date of publication of application: 27.12.1996
(62) Divisional of application: 92924367.3
(73) Proprietor: TRANSKARYOTIC THERAPIES, INC., Cambridge, MA 02139 (US)
(72) Inventor: Selden, Richard F., Wellesley, Massachusetts 02181 (US); Heartlein, Michael W., Boxborough, Massachusetts 01719 (US); Treco, Douglas A., Arlington, Massachusetts 02174 (US)
(74) Representative: White, Martin Paul

(56) References cited:
- WO-A-90/12002
- WO-A-91/06667
- WO-A-91/09955
- Methods in Enzymology, Volume 185, 1990, Michael Kriegler, "Assembly of Enhancers, Promoters, and Splice Signals to Control Expression of Transferred Genes"
- DNA and Cell Biology, Volume 9, No. 7, 1990, Anne M. Sittler et al, "Tissue-Specific Expression of the Rat Growth Hormone Gene Is Due to the Interaction of Multiple Promoter, Not Enhancer, Elements"
- Nature, Volume 311, September 1984, Robert E. Hammer et al, "Partial correction of murine hereditary growth disorder by germ-line incorporation of a new gene"
- Science, Volume 222, November 1983, Richard D. Palmiter et al, "Metallothionein-Human GH Fusion Genes Stimulate Growth of Mice"
- Blood, Volume 77, No. 2, January 1991, Shigehiko Imagawa et al, "Regulatory Elements of the Erythropoietin Gene"
- Dialog Information Services, Biosis, Dialog accession no. 7115105, Biosis, accesion no. 88037850, Kramerova I.A. et al: "Expression of the Cloned Human Erythropoietin Gene in Cho Cells", Biopolim Kletka 5 (2). 1989. 47-51
- Science, Volume 244, June 1989, Tadanori Yoshimatsu et al, "Control of Gene Expression by Artificial Introns in Saccharomyces cerevisiae"
- Nucleic Acids Research, Volume 18, No. 16, 1990, Akila Mayeda et al, "Beta-globin transcripts carrying a single intron with three adjacent nucleotides of 5' exon are efficiently spliced in vitro irrespective of intron position or surrounding exon sequences"

## Description

Efforts to develop human gene therapies have their roots in the 1950s, when early successes with kidney transplantation led to speculation that it might be possible to transplant cells from a normal individual into a patient suffering from a genetic disease. Soon after the discovery of the enzymatic defects in Gaucher's and Niemann-Pick disease, scientists considered organ and bone marrow transplantation and enzyme supplementation to treat rare genetic disorders (Brady, R., NEJM 275:312 (1966)). By the late 1960s and early 1970s, several investigators speculated that it also might be possible to introduce genes into a patient's own cells, and the cloning of the first human genes only a few years later intensified work in the field.

Until recently, almost all of the theoretical and experimental work on human gene therapy was centered on extremely rare genetic diseases, and gene therapy has come to mean, to many in the field, the modification of a patient's genes to treat a genetic disease. However, gene therapy has far wider applications than simply treatment of a genetic disease. Gene therapy is perhaps more appropriately described as medical intervention in which cells, either from the individual to be treated or another appropriate source, are modified genetically to treat or cure any condition, regardless of etiology, that will be ameliorated by the long-term delivery of a therapeutic protein. Gene therapy can therefore be thought of as an in vivo protein production and delivery system, and almost all diseases that are currently treated by the administration of proteins are candidates for treatment using gene therapy.

Gene therapy can be divided into two areas: germ cell and somatic cell gene therapy. Germ cell gene therapy refers to the modification of sperm cells, egg cells, zygotes, or early stage embryos. On the basis of both ethical and practical criteria, germ cell gene therapy is inappropriate for human use. In contrast to germ cell gene therapy, somatic cell gene therapy would affect only the person under treatment (somatic cells are cells that are not capable of developing into whole individuals and include all of the body's cells with the exception of the germ cells). As such, somatic cell gene therapy is a reasonable approach to the treatment and cure of certain disorders in human beings.

In a somatic cell gene therapy system, somatic cells (e.g., fibroblasts, hepatocytes, or endothelial cells) are removed from the patient, cultured in vitro, transfected with the gene(s) of therapeutic interest, characterized, and reintroduced into the patient. The means by which these five steps are carried out are the distinguishing features of a given gene therapy system.

Presently-available approaches to gene therapy make use of infectious vectors, such as retroviral vectors, which include the genetic material to be expressed. Such approaches have limitations, such as the potential of generating replication-competent virus during vector production; recombination between the therapeutic virus and endogenous retroviral genomes, potentially generating infectious agents with novel cell specificities, host ranges, or increased virulence and cytotoxicity; independent integration into large numbers of cells, increasing the risk of a tumorigenic insertional event; limited cloning capacity in the retrovirus (which restricts therapeutic applicability) and short-lived in vivo expression of the product of interest. A better approach to providing gene products, particularly one which avoids the risks associated with presently available methods and provides long-term production, would be valuable. Proteins of therapeutic interest are generally produced by introducing exogenous DNA encoding the protein of therapeutic interest into appropriate cells.

According to one aspect of the present invention there is provided a DNA construct that can alter the expression of a targeted gene in a vertebrate cell following homologous recombination with a target site present in DNA of the cell, the DNA construct comprising:
(a) a targeting sequence homologous to the target site,
(b) an exogenous regulatory region for altering said expression,
(c) an exon located downstream of said exogenous regulatory region, and
(d) a splice donor site at the 3' end of the exon; wherein the construct is such so that following said homologous recombination the DNA of the cell includes the construct-derived exon in addition to all endogenous exons of the targeted gene and, following transcription and splicing to remove RNA between an RNA sequence transcribed from the construct-derived exon and an RNA sequence transcribed from endogenous exon 2 of the targeted gene, RNA can be provided in which said RNA sequence transcribed from the construct-derived exon is positioned adjacent to said RNA sequence transcribed from endogenous exon 2 of the targeted gene.

The construct can be used in an *in vitro* method to transfect a vertebrate cell. The cell can be used to express a gene product.

One method of the present invention comprises:
1) providing a population of primary cells,
2) introducing into said cells or secondary cells derived therefrom a construct of the present invention and allowing homologous recombination to occur, so as to produce primary or secondary transfected cells,
3) maintaining said transfected cells under conditions appropriate for their propagation,
4) identifying a transfected primary or secondary cell.

The present invention further comprises a vertebrate cell comprising DNA having all of the endogenous exons of a given gene, as well as an exogenous exon, a splice donor site at the 3' end of the exogenous exon and an exogenous regulatory region; wherein transcription of said gene is under control of the exogenous regulatory region and, following transcription and splicing to remove RNA between an RNA sequence transcribed from the exogenous exon and an RNA sequence transcribed from endogenous exon 2 of the given gene, RNA can be provided in which an RNA sequence transcribed from the exogenous exon is positioned adjacent to the RNA sequence transcribed from endogenous exon 2 of the given gene.

If desired, the vertebrate cell may be produced via a method of the present invention. The cell may, for example, be a primary cell, a secondary cell or an immortal cell. Specific cell types include a transfected HT1080 cell, a HeLa cell, an MCF-7cell, a breast cancer cell, a K-562 leukemia cell, a KB carcinoma cell, or a 2780AD ovarian carcinoma cell.

The present invention also provides a device comprising a plurality of cells of the present invention and a barrier that permits a gene product of said cells to pass into a recipient's circulation or tissues, but which prevents an immune response from said recipient causing rejection of said cells. The device may for example be a device for intraomental implantation.

A construct, cell or device of the present invention can be used in therapy (e.g. via intraomental implantation). It can be used to prepare a medicament for treating a disorder that is treatable by altering the expression of a targeted gene. Thus expression of a gene product may be increased or decreased.

The present invention includes the preparation of medicaments for gene therapy (e.g. via intraomental implantation).

From the foregoing description it will be appreciated that the present invention allows gene or DNA targeting in cells of vertebrate, particularly mammalian, origin. That is, it allows DNA to be introduced into primary or secondary cells of vertebrate origin through homologous recombnination or targeting of the DNA, which is introduced into genomic DNA of the primary or secondary cells at a preselected site. The preselected site determines the targeting sequences used. The homologously recombinant primary or secondary cells produced by the present method are referred to as homologously recombinant (HR) primary or secondary cells.

The present invention also allows the turning on or activating a gene present in primary cells, secondary cells or immortalized cells of vertebrate origin, which is normally not expressed in the cells or is not expressed at significant levels in the cells.
Homologous recombination or targeting can be used to replace or disable the regulatory region normally associated with the gene with a regulatory sequence which causes the gene to be expressed at levels higher than evident in the corresponding nontransfected cell. The present invention, therefore, allows proteins to be made by turning on or activating an endogenous gene which encodes the desired product in transfected primary, secondary or immortalized cells.

As used herein, the term primary cell includes cells present in a suspension of cells isolated from a vertebrate tissue source (prior to their being plated i.e., attached to a tissue culture substrate such as a dish or flask), cells present in an explant derived from tissue, both of the previous types of cells plated for the first time, and cell suspensions derived from these plated cells. The term secondary cell or cell strain refers to cells at all subsequent steps in culturing. That is, the first time a plated primary cell is removed from the culture substrate and replated (passaged), it is referred to herein as a secondary cell, as are all cells in subsequent passages. Secondary cells are cell strains which consist of secondary cells which have been passaged one or more time. A cell strain consists of secondary cells that: 1) have been passaged one or more times; 2) exhibit a finite number of mean population doubling in culture; 3) exhibit the properties of contact-inhibited, anchorage dependent growth (anchorage-dependence does not apply to cells that are propagated in suspension culture); and 4) are not immortalized. A "clonal cell strain" is defined as a cell strain that is derived from a single founder cell. A "heterogenous cell strain" is defined as a cell strain that is derived from two or more founder cells.

The present invention includes primary and secondary somatic cells, such as fibroblasts, keratinocytes, epithelial cells, endothelial cells, glial cells, neural cells, formed elements of the blood, muscle cells, other somatic cells which can be cultured and somatic cell precursors, which have been transfected via a construct of the present invention with exogenous DNA which is stably integrated into their genomes or is expressed in the cells episomally. The resulting cells are referred to, respectively, as transfected primary cells and transfected secondary cells. The exogenous DNA is DNA which undergoes homologous recombination with genomic DNA of recipient cells and results in alteration of (increase or decrease in) expression of an endogenous gene.

Primary cells modified to express or render available exogenous DNA are referred to herein as transfected primary cells, which include cells removed from tissue and placed on culture medium for the first time. Secondary cells modified to express or render available exogenous DNA are referred to herein as transfected secondary cells.

Introduction of the exogenous DNA results in disablement of the endogenous sequences which control expression of the endogenous gene, either by replacing all or a portion of the endogenous (genomic) sequence or disrupting the endogenous sequence.

Primary and secondary cells transfected by the subject method fall into three types or categories: 1) cells which do not, as obtained, make or contain the therapeutic product, 2) cells which make or contain the therapeutic product but in lower quantities than normal (in quantities less than the physiologically normal lower level) or in defective form, and e) cells which make the therapeutic product at physiologically normal levels, but are to be augmented or enhanced in their content or production.

Exogenous DNA can be introduced into primary or secondary cells by a variety of techniques. For example, a construct of the present invention can be introduced into primary or secondary cells by electroporation, microinjection, or other means (e.g., calcium phosphate precipitation, modified calcium phosphate precipitation, polybrene precipitation, liposome fusion, receptor-mediated DNA delivery). Alternatively, a vector, such as a retroviral vector, which includes exogenous DNA can be used and cells can be genetically modified as a result of infection with the vector.

In addition to the exogenous DNA, transfected primary and secondary cells may optionally contain DNA encoding a selectable marker, which is expressed and confers upon recipients a selectable phenotype, such as antibiotic resistance, resistance to a cytotoxic agent, nutritional prototrophy or expression of a surface protein. Its presence makes it possible to identify and select cells containing the exogenous DNA. A variety of selectable marker genes can be used, such as neo, gpt, dhfr, ada, pac, hyg, mdr and hisD.

Transfected cells of the present invention are useful, as populations of transfected primary cells, transfected clonal cell strains, transfected heterogenous cell strains, and as cell mixtures in which at least one representative cell of one of the three preceding categories of transfected cells is present, as a delivery system for treating an individual with an abnormal or undesirable condition which responds to delivery of a therapeutic product, which is either: 1) a therapeutic protein (e.g., a protein which is absent, underproduced relative to the individual's physiologic needs, defective or inefficiently or inappropriately utilized in the individual; a protein with novel functions, such as enzymatic or transport function) or 2) a therapeutic nucleic acid (e.g., RNA which has intrinsic enzymatic activity). In the method of providing a therapeutic protein or nucleic acid, transfected primary cells, clonal cell strains or heterogenous cell strains can be administered to an individual in whom the abnormal or undesirable condition is to be treated or prevented, in sufficient quantity and by an appropriate route, to express or make available the exogenous DNA at physiologically relevant levels. A physiologically relevant level is one with either approximates the level at which the product is produced in the body or results in improvement of the abnormal or undesirable condition. Cells administered via this method are cells transfected with exogenous DNA which is introduced into a preselected site in genomic DNA through homologous recombination and function to cause recipient cells to produce a product which is normally not expressed in the cells or to produce the product of a higher level than occurs in the corresponding nontransfected cell. A regulatory region (e.g., a promoter) can therefore be introduced which replaces or disables a regulatory sequence normally associated with a gene, and results in expression of the gene at a higher level than occurs in the corresponding nontransfected cell.

### Brief Description of the Drawings

Figure 1 is a schematic representation of plasmid pXGH5, which includes the human growth hormone (hGH) gene under the control of the mouse metallothionein promoter.

Figure 2 is a schematic representation of plasmid pcDNEO, which includes the neo coding region (BamHI-BglII fragment) from plasmid PSV2neo inserted into the BamHI site of plasmid pcD; the Amp-R and pBR322ori sequences from pBR322; and the polyA, 16S splice junctions and early promoter regions from SV40.

Figure 3 is a schematic representation of plasmid pXGH301 which includes the human growth hormone gene and the neo gene.

Figure 4 is a flow chart of a method that can be used to provide a vertebrate with an implant comprising a stably transfected cell strain.

Figure 5 is a schematic representation of plasmid pXEP01. The solid black arc represents the pUC12 backbone and the arrow denotes the direction of transcription of the ampicillin resistance gene. The stippled arc represents the mouse metallothionein promoter (pmMT1). The unfilled arc interrupted by black boxes represents the human erythropoietin EPO gene (the black boxes denote exons and the arrow indicates the direction hEPO transcription). The relative positions of restriction endonuclease recognition sites are indicated.

Figure 6 is a schematic representation of plasmid pE3neoEPO. The positions of the human erythropoeietin gene and the neo and amp resistance genes are indicated. Arrows indicate the directions of transcription of the various genes. pmMT1 denotes the mouse metallothionein promoter (driving hEPO expression) and pTK denotes the Herpes Simplex Virus thymidine kinase promoter (driving neo expression). The dotted regions of the map mark the positions of human HGPRT sequences. The relative positions of restriction endonuclease recognition sites are indicated.

Figure 7 is a schematic diagram of a strategy for transcriptionally activating the hEPO gene.

Figure 8 is a schematic diagram of a strategy for transcriptionally activating the hEPO gene.

Figure 9 shows the results of an assessment of long-term in vitro hGH production by transfected primary human skin fibroblasts (two strains, HF96-11 and HF96-23).

Figure 10 is a graphic representation of human growth hormone (hGH) expression by transfected primary rabbit skin fibroblasts in vitro.

Figure 11 shows the results of an assay to detect serum levels of hGH over time in mice implanted with transfected rabbit fibroblasts expressing hGH.

Figure 12 is a graphic representation of human growth hormone (hGH) expression in cells recovered from subrenal capsule implants.

Figure 13a shows hematocrit (HCT) levels in control mice and mice implanted with transfected rabbit fibroblasts expressing hEPO.

Figure 13b shows results of an assay to detect hEPO in the serum of mice implanted with transfected rabbit fibroblasts expressing hEPO.

In the following description and accompanying examples various possibilities for providing transfected vertebrate cells are discussed. In some cases passages do not specifically relate to the invention as set out in the accompanying claims, but have been retained for information purposes.

As described herein, primary or secondary cells of vertebrate, particularly mammalian, origin have been transfected with exogenous DNA encoding a therapeutic product and shown to produce the encoded therapeutic protein stably and reproducibly, both in vitro and in vivo, over extended periods of time. In addition, the transfected primary and secondary cells have been shown to express the encoded product in vivo at physiologically relevant levels, to be recoverable after implantation and, upon reculturing, to grow and display their preimplantation properties. This demonstration is in sharp contrast to what one of skill in the art would predict, because, for example, even experts in the field see the finite life span of normal somatic cells and the inability to isolate or grow the relevant transplantable cells as precluding their use for gene therapy unless the cells are genetically modified using retroviruses. Miller, A.D., Blood, 76:271-278 (1990). However, the transplantation of retrovirally-treated fibroblasts has been shown to provide, at best, only transient metabolic improvements, and is seen to have serious limitations as a therapeutic system. Normal (non immortal) fibroblasts are characterized as being "much more difficult to transfect than continuous cell lines by using calcium phosphate precipitation techniques." Miller, A.D., Blood, 76:271-278 (1990). Furthermore, in considering non- retroviral techniques for gene therapy, it is typical of experts in the field to believe "... the efficiency of gene delivery is dismal... A physician would have to obtain an impossible number of cells from patients to guarantee the appropriate alteration of the millions required for therapy." (Verma, I.M., Scient. Amer., November 1990, pages 68-84).

Surprisingly, Applicants have been able to produce transfected primary and secondary cells which include exogenous DNA encoding a desired product, (i.e., a translation product which is a therapeutic protein or an antigen against which antibodies are produced) and stably express the exogenous DNA. It is also possible, using the method described herein, to produce transfected primary and secondary cells which include exogenous DNA encoding other translation products (novel proteins not made in nature) or transcription products (e.g., anti-sense RNA or ribozymes) or exogenous DNA which itself is a therapeutic product (e.g., exogenous DNA which binds a regulatory protein present in the transfected cell).

The method includes the steps of: 1) providing a population of primary cells, obtained from the individual to whom the transfected primary cells will be administered or from another source; 2) introducing into the primary cells or into secondary cells derived from primary cells a DNA construct which includes exogenous DNA as described above and the necessary additional DNA sequences described above, producing transfected primary or secondary cells; 3) maintaining transfected primary or secondary cells under conditions appropriate for their propagation; 4) identifying a transfected primary or secondary cell; and 5) producing a colony from the transfected primary or secondary cell identified in (4) by maintaining it under appropriate culture conditions and for sufficient time for its propagation, thereby producing a cell strain derived from the (founder) cell identified in (4). In one embodiment of the method, exogenous DNA is introduced into genomic DNA by homologous recombination between DNA sequences present in the DNA construct and genomic DNA.

In one embodiment of the present method of producing a clonal population of transfected secondary cells, a cell suspension containing primary or secondary cells is combined with exogenous DNA encoding a therapeutic product and DNA encoding a selectable marker, such as the neo gene. The two DNA sequences are present on the same DNA construct or on two separate DNA constructs. The resulting combination is subjected to electroporation, generally at 250-300 volts with a capacitance of 960 µFarads and an appropriate time constant (e.g., 14 to 20 m sec) for cells to take up the DNA construct. In an alternative embodiment, microinjection is used to introduce the DNA construct into primary or secondary cells. In either embodiment, introduction of the exogenous DNA results in production of transfected primary or secondary cells.

In the method of producing heterogenous cell strains, the same steps are carried out as described for production of a clonal cell strain, except that a single transfected primary or secondary cell is not isolated and used as the founder cell. Instead, two or more transfected primary or secondary cells are cultured to produce a heterogenous cell strain.

A method of producing antibodies specific for the protein encoded by the exogenous DNA can be provided. In the method, transfected primary or secondary cells expressing an antigen against which antibodies are desired are introduced into an animal recipient (e.g., rabbit, mouse, pig, dog, cat, goat, guinea pig, sheep, non-human primate). The animal recipient produces antibodies against the antigen expressed, which may be an entire protein antigen or a peptide encoded by a fragment of the intact gene which encodes the entire antigen. Polyclonal sera is obtained from the animals. It is also possible to produce monclonal antibodies through the use of transfected primary or secondary cells. Splenocytes are removed from an animal recipient of transfected primary or secondary cells expressing the antigen against which monoclonal antibodies are desired. The splenocytes are fused with myeloma cells, using known methods, such as that of Koprowski et al. (U.S. Patent No. 4,172,124) or Kohler et al., (Nature 256:495-497 (1975)) to produce hybridoma cells which produce the desired monclonal antibody. The polyclonal antisera and monclonal antibodies produced can be used for the same purposes (e.g., diagnostic, preventive, therapeutic purposes) as antibodies produced by other methods.

The present invention has wide applicability in treating abnormal or undesired conditions and can be used to provide a variety of products to an individual. For example, it can be used to provide secreted proteins (with either predominantly systemic or predominantly local effects), membrane proteins (e.g., for imparting new or enhanced cellular responsiveness, facilitating removal of a toxic product or marking or targeting a cell) or intracellular proteins (e.g., for affecting gene expression or producing autolytic effects). In addition, it can be used to provide antigens against which immune response occurs in an individual (to prevent disease as by vaccination or to suppress an existing condition). The present invention is particularly advantageous in treating abnormal or undesired conditions in that it: 1) is curative (one gene therapy treatment has the potential to last a patient's lifetime); 2) allows precise dosing (the patient's cells continuously determine and deliver the optimal dose of the required protein based on physiologic demands, and the stably transfected cell strains can be characterized extensively in vitro prior to implantation, leading to accurate predictions of long term function in vivo); 3) is simple to apply in treating patients; 4) eliminates issues concerning patient compliance (following a one-time gene therapy treatment, daily protein injections are no longer necessary); 5) reduces treatment costs (since the therapeutic protein is sytnthesized by the patient's own cells, investment in costly protein production and purification is unnecessary); and 6) is safe (the invention does not need to use infectious agents such as retroviruses to genetically engineer the patient's cells, thereby overcoming the safety and efficacy issues that have hampered other gene therapy systems).

As further described herein, primary or secondary cells of vertebrate, particularly mammalian, origin have been transfected with exogenous DNA encoding EPO and shown to produce the encoded EPO reproducibly, both in vitro and in vivo, over extended periods of time. In addition, the transfected primary and secondary cells have been shown express EPO in vivo at physiologically relevant levels. The EPO expressed has been shown to have the glycosylation pattern typical of EPO purified from human urine or recombinant human EPO.

In addition, as further described herein, primary or secondary cells of vertebrate, particularly mammalian, origin have been transfected with exogenous DNA encoding hGH and shown to produce the encoded hGH reproducibly, both in vitro and in vivo, over extended periods of time. In addition, the transfected primary and secondary cells have been shown to express hGH in vivo at physiologically relevant levels.

Applicants have also developed methods for producing transfected primary or secondary cells which stably express exogenous DNA encoding EPO, clonal cell strains and heterogenous cell strains of such transfected cells, methods of producing the clonal and heterogenous cell strains, and methods of using transfected cells expressing EPO to deliver the encoded product to an individual mammal at physiologically relevant levels. The constructs and methods herein described are useful, for example, for treating an individual (human)whose EPO production and/or function is in need of being increased or enhanced [e.g., is compromised or less than normal, or normal but the individual would benefit from enhancement, at least temporarily, of red blood cell production (e.g., during predialysis or dialysis therapy, during treatment of AIDS with AZT, after surgery, or during chemotherapy)].

As also described herein, it is possible to transfect primary or secondary cells of vertebrate, particularly mammalian, origin with exogenous DNA encoding insulinotropin and to use them to provide insulinotropin to an individual in whom insulin production, function and/or sensitivity is compromised. As used herein, the term insulinotropin includes, e.g. derivatives of glucagon-like peptide 1 (GLP-1) such as GLP(7-37), GLP(7-36), GLP-1(7-35) and GLP-1(7-34) as well as their carboxy-terminal amidated derivatives produced by in vivo amidating enzymes and derivatives which have amino acid alterations or other alterations which result in substantially the same biological activity or stability in the blood as that of a truncated GLP-1 or enhanced biological activity or stability.

As further described herein, Applicants have also demonstrated that DNA can be introduced into primary or secondary vertebrate cells in a DNA construct or plasmid and integrated into the genome of the transfected primary or secondary cells by homologous recombination. That is, they have demonstrated gene targeting in primary and secondary mammalian cells. They have further demonstrated that the exogenous DNA has the desired function in the homologously recombinant (HR) cells and that correctly targeted cells can be identified on the basis of a detectable phenotype conferred by a selectable marker gene.

In addition, the present invention relates to a method of protein production using transfected primary, secondary or immortalized cells. The method involves transfecting primary cells, secondary cells or immortalized cells with exogenous DNA which is sufficient to target to and activate an endogenous gene which encodes a therapeutic product. For example, Examples 18f, 19 and 21 describe protein production by targeting and activation of a selected endogenous gene.

Applicants describe (Example 3) construction of plasmids containing a selectable marker gene (plasmid pcDNEO), a gene encoding a therapeutic product (plasmid pXGH5) or both (pXGH301). They also describe construction of a plasmid useful for targeting to a particular locus (the HPRT locus) in the human genome and selection based upon a drug resistant phenotype (Example 18a). This plasmid is designated pE3Neo and its integration into the cellular genomes at the HPRT locus produces cells which have an hprt⁻, 6-TG resistant phenotype and are also G418 resistant. As described, they have shown that pE3Neo functions properly in gene targeting in an established human fibroblast cell line (Example 18b), by demonstrating localization of the DNA introduced into established cells within exon 3 of the HPRT gene.

In addition, Applicants demonstrate gene targeting in primary and secondary human skin fibroblasts using pE3Neo (Example 18c) and describe construction of a plasmid for targeted insertion of a gene encoding a therapeutic product (human growth hormone [hGH]) into the human genome (Example 18d). The subject application further demonstrates that modification of DNA termini enhances targeting of DNA into genomic DNA (Examples 18c and 18e) and construction of a variety of targeting plasmids. For instance, Applicants describe targeting plasmids for placing a human gene under the control of a murine promoter known to function in human cells (Examples 18f and 18i); for targeting to sequences flanking a gene and isolation of targeted secondary fibroblasts using a variety of screening and selection approaches (Examples 18g, 18h, 18j and 18k); for placing a human gene not normally expressed in the primary or secondary cells under the control of a promoter of nonhuman or human origin, to produce homologously recombinant primary or secondary cells which express the encoded product (Examples 18f-18k).

Two further embodiments are envisioned, (Example 19) in which the normal regulatory sequences upstream of a gene (e.g. the human EPO gene) are altered to allow expression of a gene product in primary or secondary cell strains which do not normally express that product in detectable quantities in their untransfected state. In one embodiment, the product of the targeting events is a chimeric transcription unit, in which a regulatory element and an operatively linked exon are positioned upstream of the desired endogenous gene to be activated. The product of transcription, splicing, and translation produces a chimeric protein in which amino acids encoded by exon 1 of the exogenous gene are fused to amino acids encoded by exons 2 and downstream exons in the endogenous gene. In a second embodiment, the product of the targeting event replaces the regulatory and exon 1 sequences of the endogenous gene with corresponding exogenous sequences. The product of transcription, splicing, and translation produces a chimeric protein similar to that described above. Typically, secretion of such proteins involves membrane translocation and removal of the signal peptide, in this case producing a normal protein lacking the chimeric signal peptide. In both cases the chimeric protein is now under the control of a desired regulatory element.

Examples 18f-18h and 19 illustrate embodiments in which the normal regulatory sequences upstream of the human EPO gene are altered to allow expression of hEPO in primary or secondary fibroblast strains which do not express EPO in detectable quantities in their untransfected state. In one embodiment the product of targeting leaves the normal EPO protein intact, but under the control of the mouse metallothionein promoter. Examples 18i and 18j demonstrate the use of similar targeting constructs to activate the endogenous growth hormone gene in primary or secondary human fibroblasts. In other embodiments described for activating EPO expression in human fibroblasts, the products of targeting events are chimeric transcription units, in which the first exon of the human growth hormone gene is positioned upstream of EPO exons 2-5. The product of transcription (controlled by the mouse metallothionein promoter), splicing, and translation is a protein in which amino acids 1-4 of the hEPO signal peptide are replaced with amino acid residues 1-3 of hGH. The chimeric portion of this protein, the signal peptide, is removed prior to secretion from cells.

The Examples provide methods for activating endogenous genes by gene targeting which do not require manipulation or other uses of the hEPO and hGH protein coding regions. By these methods, normally inactive genes may be activated in cells that have properties desirable for in vivo protein delivery methods (e.g. gene therapy) and in vitro protein production (e.g., pharmaceutics).

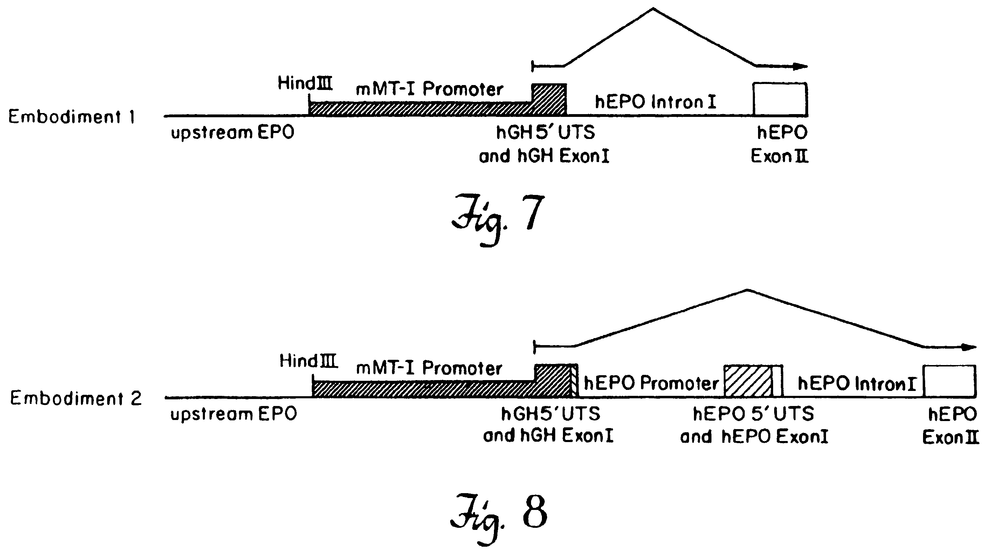

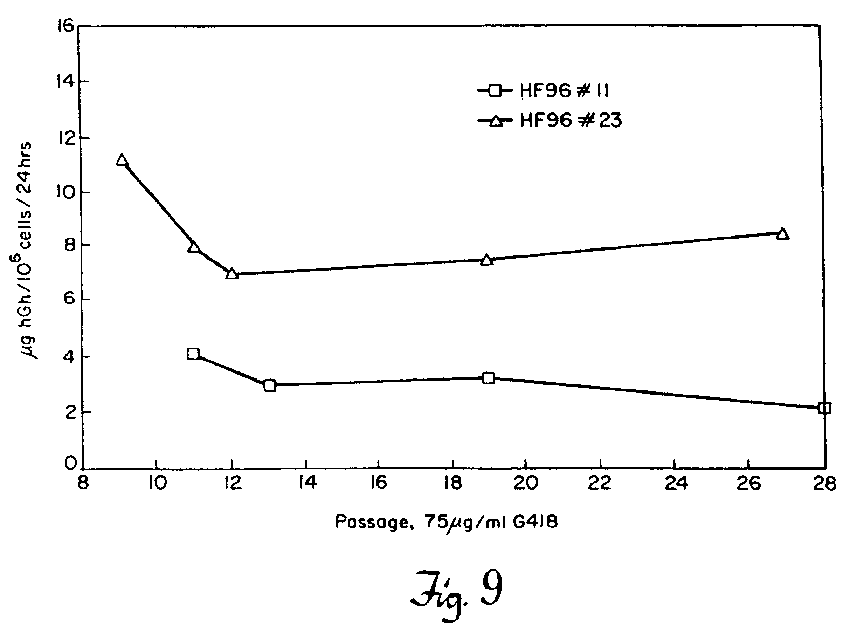

Figures 7 and 8 illustrate two strategies for transcriptionally activating the hEPO gene. The thin lines represent hEPO sequences; thick lines, mouse metallothionein I promoter; stippled box, 5' untranslated region of hGH; solid box, hGH exon 1; striped box, 10 bp linker from hEPO intron 1; cross-hatched box, 5' untranslated region of hEPO; and open boxes, hEPO coding sequences; HIII, HindIII site.

Using the methods and DNA constructs or plasmids taught herein or modifications thereof which are apparent to one of ordinary skill in the art, exogenous DNA which encodes a therapeutic product (e.g., protein, ribozyme, nucleic acid) can be inserted at preselected sites in the genome of vertebrate (e.g., mammalian, both human and nonhuman) primary or secondary cells.

The methods and DNA constructs described can be used for a wide variety of purposes. The method can be used to alter primary or secondary cells of vertebrate origin in order to repair, alter, delete or replace DNA already present in the recipient primary or secondary cell; to introduce into primary or secondary cells a gene or DNA sequence (at a preselected site) which encodes a therapeutic product or other desired product or is itself a therapeutic or other product; to add to or replace regulatory sequences present in the primary or secondary cell recipients; to knock out or remove an entire gene or gene portion present in primary or secondary cells; and to produce universal donor cells.

The transfected primary or secondary cells may also include DNA encoding a selectable marker which confers a selectable phenotype upon them, facilitating their identification and isolation. Applicants have also developed methods for producing transfected primary or secondary cells which stably express exogenous DNA, clonal cell strains and heterogenous cell strains of such transfected cells, methods of producing the clonal and heterogenous cell strains, and methods of treating or preventing an abnormal or undesirable condition through the use of populations of transfected primary or secondary cells of the present invention.

### Transfected Cells

Primary and secondary cells to be transfected by the present method can be obtained from a variety of tissues and include all cell types which can be maintained in culture. For example, primary and secondary cells which can be transfected by the present method include fibroblasts, keratinocytes, epithelial cells (e.g., mammary epithelial cells, intestinal epithelial cells), endothelial cells, glial cells, neural cells, formed elements of the blood (e.g., lymphocytes, bone marrow cells), muscle cells and precursors of these somatic cell types. Primary cells are preferably obtained from the individual to whom the transfected primary or secondary cells are administered. However, primary cells may be obtained from a donor (other than the recipient) of the same species or another species (e.g., mouse, rat, rabbit, cat, dog, pig, cow, bird, sheep, goat, horse).

Transfected primary and secondary cells have been produced, with or without phenotypic selection, as described in Examples 5-7, and shown to express exogenous DNA encoding a therapeutic product including, e.g., EPO and insulinotropin.

Immortalized cells can also be transfected by the present method and used for either gene therapy or protein production. Examples of immortalized human cell lines useful for protein production by the present method include, but are not limited to, HT1080, HeLa, MCF-7 breast cancer cells, K-562 leukemia cells, KB carcinoma cells and 2780AD ovarian carcinoma cells.

### Exogenous DNA

Exogenous DNA incorporated into primary or secondary cells can be: 1) DNA which encodes a translation or transcription product whose expression in primary or secondary cells is desired, such as a translation or transcription product useful to treat an existing condition or prevent it from occurring (eg., EPO or insulinotropin) and 2) DNA which does not encode a gene product but is itself useful, such as DNA useful to treat an existing condition or prevent it from occurring or 3) DNA which undergoes homologous recombination with genomic DNA of recipient cells and results in alteration of (increase or decrease in) expression of an endogenous gene.

DNA transfected into primary or secondary cells can encode an entire desired product, or can encode, for example, the active or functional portion(s) of the product. The product can be, for example, a hormone, a cytokine, an antigen, an antibody, an enzyme, a clotting factor, a transport protein, a receptor, a regulatory protein, a structural protein, an anti-sense RNA, a ribozyme or a protein or a nucleic acid which does not occur in nature (i.e., a novel protein or novel nucleic acid). The DNA can be obtained from a source in which it occurs in nature or can be produced, using genetic engineering techniques or synthetic processes. The DNA transfected into primary or secondary cells can encode one or more therapeutic products. After transfection into primary or secondary cells, the exogenous DNA is stably incorporated into the recipient cell's genome (along with the additional sequences present in the DNA construct used), from which it is expressed or otherwise functions. Alternatively, the exogenous DNA may exist episomally within the transfected primary or secondary cells.

DNA encoding the desired product can be introduced into cells under the control of an inducible promoter, with the result that cells produced or as introduced into an individual do not express the product but can be induced to do so (i.e., production is induced after the transfected cells are produced but before implantation or after implantation). DNA encoding the desired product can, of course, be introduced into cells in such a manner that it is expressed upon introduction (i.e., without induction).

### Selectable Markers

A variety of selectable markers can be incorporated into primary or secondary cells. For example, a selectable marker which confers a selectable phenotype such as drug resistance, nutritional auxotrophy, resistance to a cytotoxic agent or expression of a surface protein, can be used. Selectable marker genes which can be used include neo, gpt, dhfr, ada, pac, hyg and hisd. The selectable phenotype conferred makes it possible to identify and isolate recipient primary or secondary cells.

Selectable markers can be divided into two categories: positive selectable and negative selectable. In positive selection, cells expressing the positive selectable marker are capable of surviving treatment with a selective agent (such as neo, gpt, dhfr, ada, pac, hyg, mdrl and hisD). In negative selection, cells expressing the negative selectable marker are destroyed in the presence of the selective agent (e.g., tk, gpt).

### DNA Constructs

DNA constructs, which include exogenous DNA and, optionally, DNA encoding a selectable marker, along with additional sequences necessary for expression of the exogenous DNA in recipient primary or secondary cells, can be used to transfect primary or secondary cells in which the encoded product is to be produced. The DNA construct can also include targeting sequences for homologous recombination with host cell DNA. DNA constructs which include exogenous DNA sequences which do not encode a gene product (and are the therapeutic product) and, optionally, include DNA encoding a selectable marker, can be used to transfect primary and secondary cells. Alternatively, infectious vectors, such as retroviral, herpes, adenovirus, adenovirus-associated, mumps and poliovirus vectors, can be used for this purpose.

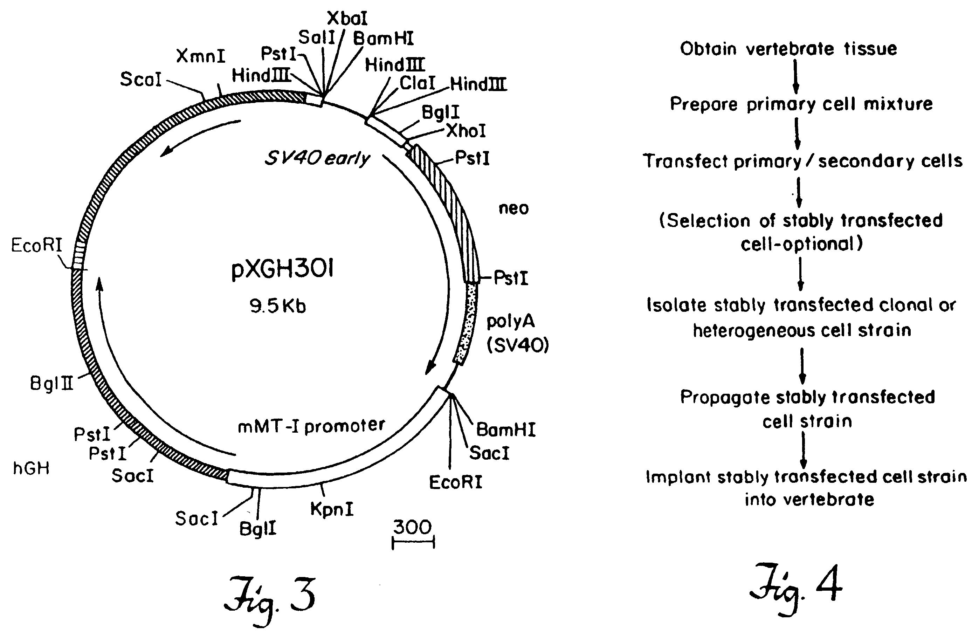

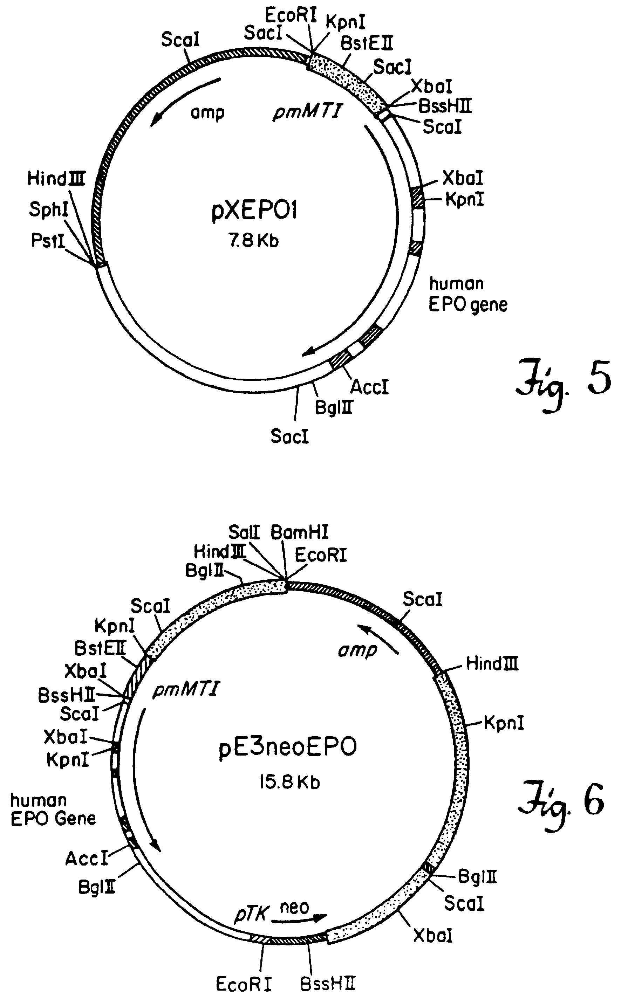

In one embodiment, a DNA construct which includes the exogenous DNA and additional sequences, such as sequences necessary for expression of the exogenous DNA, can be used (e.g., plasmid pXGH5 or plasmid pXEP01). A DNA construct can include an inducible promoter which controls expression of the exogenous DNA, making inducible expression possible. Optionally, the DNA construct may include a bacterial origin of replication and bacterial antibiotic resistance markers, which allow for large-scale plasmid propagation in bacteria. A DNA construct which includes DNA encoding a selectable marker, along with additional sequences, such as a promoter, polyadenylation site and splice junctions, can be used to confer a selectable phenotype upon transfected primary or secondary cells (e.g., plasmid pcDNEO). The two DNA constructs are co-transfected into primary or secondary cells, using methods described herein. Alternatively, one DNA construct which includes exogenous DNA, a selectable marker gene and additional sequences (e.g., those necessary for expression of the exogenous DNA and for expression of the selectable marker gene) can be used. Such a DNA construct (e.g., plasmid PXGH301, which includes the hGH gene and the neo gene, or plasmid pE3neoEPO which includes the EPO gene and the neo gene; these plasmids are described in Figures 3 and 6, respectively). Similar constructs, which include exogenous DNA encoding insulinotropin and additional sequences (e.g., sequences necessary for insulinotropin expression) can be produced (e.g., plasmid pXGLP1; see Example 11). These constructs can also include DNA encoding a selectable marker, as well as other sequences, such as a promoter, a polyadenylation site, and splice junctions.

In those instances in which DNA is injected directly into an individual, such as by injection into muscles, the DNA construct can include the exogenous DNA and regulatory sequences necessary and sufficient for expression of the encoded product (e.g., EPO) upon entry of the DNA construct into recipient cells.

In another embodiment, DNA constructs, which include exogenous DNA encoding a desired product, targeting sequences for homologous recombination and, optionally, DNA encoding one or more selectable markers are used to transfect primary or secondary cells in which homologous recombination is to occur. In this embodiment, DNA sequences necessary for expression of the exogenous DNA will generally be present as well. DNA constructs which include exogenous DNA sequences which do not encode a gene product (and are the desired product) and, optionally, include DNA encoding a selectable marker, can also be used to transfect primary and secondary cells.

The exogenous DNA, targeting sequences and selectable marker can be introduced into cells on a single DNA construct or on separate constructs. The total length of the DNA construct will vary according to the number of components (exogenous DNA, targeting sequences, selectable marker gene) and the length of each. The entire construct length will generally be at least 20 nucleotides. In a construct in which the exogenous DNA has sufficient homology with genomic DNA to undergo homologous recombination, the construct will include a single component, the exogenous DNA. In this embodiment, the exogenous DNA, because of its homology, serves also to target integration into genomic DNA and additional targeting sequences are unnecessary. Such a construct is useful to knock out, replace or repair a resident DNA sequence, such as an entire gene, a gene portion, a regulatory element or portion thereof or regions of DNA which, when removed, place regulatory and structural sequences in functional proximity. It is also useful when the exogenous DNA is a selectable marker.

In a third embodiment, the DNA construct includes exogenous DNA and one or more separate targeting sequences, generally located at both ends of the exogenous DNA sequence. Targeting sequences are DNA sequences normally present in the primary or secondary cell genome in the genome of the cells as obtained [e.g., an essential gene, a nonessential gene or noncoding DNA, or present in the genome through a previous modification]. Such a construct is useful to integrate exogenous DNA encoding a therapeutic product, such as a hormone, a cytokine, an antigen, an antibody, an enzyme, a clotting factor, a transport protein, a receptor, a regulatory protein, a structural protein, an anti-sense RNA, a ribozyme or a protein or a nucleic acid which does not occur in nature. In particular, exogenous DNA can encode one of the following: Factor VIII, Factor IX, erythropoietin, alpha-1 antitrypsin, calcitonin, glucocerebrosidase, growth hormone, low density lipoprotein (LDL) receptor, apolipoprotein E, IL-2 receptor and its antagonists, insulin, globin, immunoglobulins, catalytic antibodies, the interleukins, insulin-like growth factors, superoxide dismutase, immune responder modifiers, parathyroid hormone, interferons, nerve growth factors, tissue plasminogen activators, and colony stimulating factors. Such a construct is also useful to integrate exogenous DNA which is a therapeutic product, such as DNA sequences sufficient for sequestration of a protein or nucleic acid in the transfected primary or secondary cell, DNA sequences which bind to a cellular regulatory protein, DNA sequences which alter the secondary or tertiary chromosomal structure and DNA sequences which are transcriptional regulatory elements into genomic DNA of primary or secondary cells.

In a fourth embodiment, the DNA construct can include exogenous DNA, targeting DNA sequences and DNA encoding at least one selectable marker. In this fourth embodiment, the order of construct components can be: targeting sequences-exogenous DNA-DNA encoding a selectable marker(s)-targeting sequences. In this embodiment, one or more selectable markers are included in the construct, which makes selection based on a selectable phenotype possible. Cells that stably integrate the construct will survive treatment with the selective agent; a subset of the stably transfected cells will be HR cells, which can be identified by a variety of techniques, including PCR, Southern hybridization and phenotypic screening.

In a fifth embodiment, the order of components in the DNA construct can be: targeting sequence-selectable marker 1 - targeting sequence - selectable marker 2. In this embodiment selectable marker 2 displays the property negative selection. That is, the gene product of selectable marker 2 can be selected against by growth in an appropriate media formulation containing an agent (typically a drug or metabolite analog) which kills cells expressing selectable marker 2. Recombination between the targeting sequences flanking selectable marker 1 with homologous sequences in the host cell genome results in the targeted integration of selectable marker 1, while selectable marker 2 is not integrated. Such recombination events generate cells which are stably transfected with selectable marker 1 but not stably transfected with selectable marker 2, and such cells can be selected for by growth in the media containing the selective agent which selects for selectable marker 1 and the selective agent which selects against selectable marker 2.

In all embodiments of the DNA construct, exogenous DNA can encode one or more products, can be one or more therapeutic products or one or more of each, thus making it possible to deliver multiple products.

### Transfection of Primary or Secondary Cells and Production of Clonal or Heterogenous Cell Strains

A method is represented schematically in Figure 4. As shown, vertebrate tissue is first obtained; this is carried out using known procedures, such as punch biopsy or other surgical methods of obtaining a tissue source of the primary cell type of interest. For example, punch biopsy is used to obtain skin as a source of fibroblasts or keratinocytes. A mixture of primary cells is obtained from the tissue, using known methods, such as enzymatic digestion or explanting. If enzymatic digestion is used, enzymes such as collagenase, hyaluronidase, dispase, pronase, trypsin, elastase and chymotrypsin can be used.

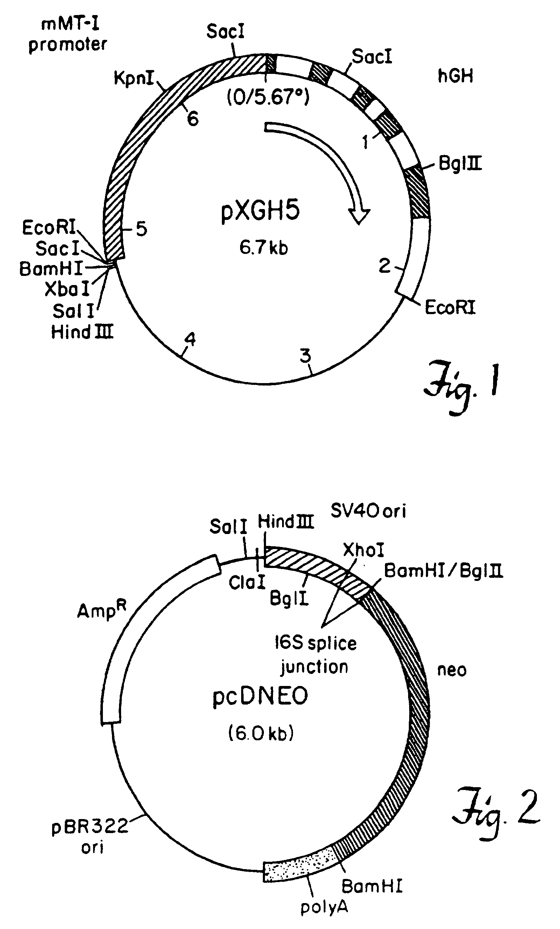

The resulting primary cell mixture can be transfected directly or it can be cultured first, removed from the culture plate and resuspended before transfection is carried out. Primary cells or secondary cells are combined with exogenous DNA to be stably integrated into their genomes and, optionally, DNA encoding a selectable marker, and treated in order to accomplish transfection. The exogenous DNA and selectable marker-encoding DNA are each on a separate construct (e.g., pXGH5 and pcDNEO, see Figures 1 and 2) or on a single construct (e.g., pXGH301 and pE3neoEPO, see Figure 3 and Figure 6). An appropriate quantity of DNA is used to ensure that at least one stably transfected cell containing and appropriately expressing exogenous DNA is produced. In general, 0.1 to 500 µg DNA is used.

Using the present methods to introduce only a selectable marker gene, between 170 (1 in 588 starting cells treated by electroporation, Example 6) and 2000 (1 in 49 starting cells treated by microinjection, Example 5) stably transfected cells are generated per 100,000 starting cells. Using the present methods to introduce a therapeutic gene as well as a selectable marker gene, between 7 (1 in 14,705 starting cells treated by electroporation, Example 6) and 950 (1 in 105 starting cells treated by microinjection, Example 5) stably transfected cells are generated per 100,000 starting cells. Of these stable transfectants, from 43 to 90% express the gene of therapeutic interest. Since only a single appropriately expressing cell is required, it is clearly possible to use substantially fewer starting cells. Conversely, using transfection techniques which are substantially less efficient than the present methods, it would not be possible to obtain even a single such cell unless large amount of the individual's tissue is used as the source of starting cells.

In one embodiment of the present method of producing transfected primary or secondary cells, transfection is effected by electroporation, as described in the Examples. Electroporation is carried out at appropriate voltage and capacitance (and corresponding time constant) to result in entry of the DNA construct(s) into the primary or secondary cells. Electroporation can be carried out over a wide range of voltages (e.g., 50 to 2000 volts) and corresponding capacitance. As described herein, electroporation is very efficient if carried out at an electroporation voltage in the range of 250-300 volts and a capacitance of 960 µFarads. Total DNA of approximately 0.1 to 500 µg is generally used. As described in the Examples, total DNA of 60 µg and voltage of 250-300 volts with capacitance of 960 µFarads for a time constant 14-20 of msec. has been used and shown to be efficient.

In another embodiment of the present method, primary or secondary cells are transfected using microinjection. See, for example, Example 5. Alternatively, known methods such as calcium phosphate precipitation, modified calcium phosphate precipitation and polybrene precipitation, liposome fusion and receptor-mediated gene delivery can be used to transfect cells. A stably transfected cell is isolated and cultured and subcultivated, under culturing conditions and for sufficient time, to propagate the stably transfected secondary cells and produce a clonal cell strain of transfected secondary cells. Alternatively, more than one transfected cell is cultured and subcultured, resulting in production of a heterogenous cell strain.

Transfected primary or secondary cells undergo a sufficient number of doublings to produce either a clonal cell strain or a heterogenous cell strain of sufficient size to provide the therapeutic product (e.g., EPO) to an individual in effective amounts. In general, for example, 0.1 cm² of skin is biopsied and assumed to contain 100,000 cells; one cell is used to produce a clonal cell strain and undergoes approximately 27 doublings to produce 100 million transfected secondary cells. If a heterogenous cell strain is to be produced from an original transfected population of approximately 100,000 cells, only 10 doublings are needed to produce 100 million transfected cells.

The number of required cells in a transfected clonal or heterogenous cell strain is variable and depends on a variety of factors, which include but are not limited to, the use of the transfected cells, the functional level of the exogenous DNA in the transfected cells, the site of implantation of the transfected cells (for example, the number of cells that can be used is limited by the anatomical site of implantation), and the age, surface area, and clinical condition of the patient. To put these factors in perspective, to deliver therapeutic levels of human growth hormone in an otherwise healthy 60 kg patient with isolated growth hormone deficiency, approximately one to five hundred million transfected fibroblasts would be necessary. This represents approximately the volume of cells prosent on the very tip of the patient's thumb.

### Episomal Expression of Exogenous DNA

DNA sequences that are present within the cell yet do not integrate into the genome are referred to as episomes. Recombinant episomes may be useful in at least three settings: 1) if a given cell type is incapable of stably integrating the exogenous DNA; 2) if a given cell type is adversely affected by the integration of DNA; and 3) if a given cell type is capable of improved therapeutic function with an episomal rather than integrated DNA.

Using the transfection and culturing approaches to gene therapy described in the Examples, exogenous DNA in the form of episomes can be introduced into vertebrate primary and secondary cells. Plasmid pXGH301 can be converted into such an episome by the addition DNA sequences for the Epstein-Barr virus origin of replication and nuclear antigen [Yates, J.L. Nature 319:780-7883 (1985)]. Alternatively, vertebrate autonomously replicating sequences can be introduced into the construct (Weidle, U.H. Gene 73(2):427-437 (1988). These and other episomally derived sequences can also be included in DNA constructs without selectable markers, such as pXGH5. The episomal exogenous DNA is then introduced into primary or secondary vertebrate cells as described in this application (if a selective marker is included in the episome a selective agent is used to treat the transfected cells).

### Implantation of Clonal Cell Strains or Heterogenous Cell Strains of Transfected Secondary Cells

The transfected cells produced by the methods described above and in the Examples that follow, are introduced into an individual to whom the therapeutic product is to be delivered, using known methods. The clonal cell strain or heterogenous cell strain is then introduced into an individual, using known methods, using various routes of administration and at various sites (e.g., renal subcapsular, subcutaneous, central nervous system (including intrathecal), intravascular, intrahepatic, intrasplanchnic, intraperitoneal (including intraomental), or intramuscular implantation). Once implanted in the individual, the transfected cells produce the therapeutic product encoded by the exogenous DNA or are affected by the exogenous DNA itself. For example, an individual who has been diagnosed with Hemophilia B, a bleeding disorder that is caused by a deficiency in Factor IX, a protein normally found in the blood, is a candidate for a gene therapy cure. The patient has a small skin biopsy performed; this is a simple procedure which can be performed on an out-patient basis. The piece of skin, approximately the size of a matchhead, is taken, for example, from under the arm and requires about one minute to remove. The sample is processed, resulting in isolation of the patient's cells (in this case, fibroblasts) and genetically engineered to produce the missing Factor IX. Based on the age, weight, and clinical condition of the patient, the required number of cells are grown in large-scale culture. The entire process usually requires 4-6 weeks and, at the end of that time, the appropriate number of genetically-engineered cells are introduced into the individual, once again as an out-patient (e.g., by injecting them back under the patient's skin). The patient is now capable of producing his or her own Factor IX and is no longer a hemophiliac.

A similar approach can be used to treat other conditions or diseases. For example, short stature can be treated by administering human growth hormone to an individual by implanting primary or secondary cells which express human growth hormone, or anemia can be treated by implanting primary or secondary cells which express EPO.

In addition to the previous examples, transfected cells, produced as described above, which contain insulinotropin-encoding DNA are delivered into an individual in whom insulin production, secretion, function and/or sensitivity is compromised. They are introduced into the individual by known methods and at various sites of administration (e.g., renal, subcapsular, subcutaneous, central nervous system (including intrathecal), intravascular, intrahepatic, intrasplanchnic, intraperitoneal (including intraomental) or intramuscular implantation). Once implanted in the individual, the transfectedcells produce insulinotropin encoded by the exogenous DNA. For example, an individual in whom insulin production, secretion or sensitivity is impaired can receive therapy or preventive treatment through the implantation of transfected cells expressing exogenous DNA encoding insulinotropin produced as described herein. The cells to be genetically engineered are obtained as described above, processed in a similar manner to produce sufficient numbers of cells, and introduced back into the individual.

As these examples suggest, the cells used will generally be patient-specific genetically-engineered cells. It is possible, however, to obtain cells from another individual of the same species or from a different species. Use of such cells might require administration of an immunosuppressant, alteration of histocompatibility antigens, or use of a barrier device to prevent rejection of the implanted cells.

In one embodiment, a barrier device is used to prevent rejection of implanted cells obtained from a source other than the recipient (e.g., from another human or from a non-human mammal such as a cow, dog, pig, goat, sheep or rodent). In this embodiment, transfected cells of the present invention are placed within the barrier device, which is made of a material (e.g., a membrane such as Amicon XM-50) which permits the product encoded by the exogenous DNA to pass into the recipient's circulation or tissues but prevents contact between the cells and the recipient's immune system and thus prevents an immune resonse to (and possible rejection of) the cells by the recipient. Alternatively, DNA encoding hGH, EPO or insulinotropin can be introduced into an individual by direct injection, such as into muscle or other appropriate site. In this embodiment, the DNA construct includes exogenous DNA encoding the therapeutic product (e.g., EPO, insulinotropin) and sufficient regulatory sequences for expression of the exogenous DNA in recipient cells. After injection into the individual, the DNA construct is taken up by some of the recipient cells. The DNA can be injected alone or in a formulation which includes a physiologially compatible carrier (e.g., a physiological buffer) and, optionally, other components, such as agents which allow more efficient entry of the DNA construct into cells, stabilize the DNA or protect the DNA from degradation.

For many diseases, this will be a one-time treatment and, for others, multiple gene therapy treatments will be required.

### Uses of Transfected Primary and Secondary Cells and Cell Strains

Transfected primary or secondary cells or cell strains as described herein have wide applicability as a vehicle or delivery system for therapeutic products, such as enzymes, hormones, cytokines, antigens, antibodies, clotting factors, anti-sense RNA, regulatory proteins, transcription proteins, receptors, structural proteins, ribozymes, novel (non-naturally occurring) proteins and nucleic acid products, and engineered DNA. For example, transfected primary or secondary cells can be used to supply a therapeutic protein, including, but not limited to, Factor VIII, Factor IX, erythropoietin, alpha-1 antitrypsin, calcitonin, glucocerebrosidase, growth hormone, low density lipoprotein (LDL), apolipoprotein E, receptor IL-2 receptor and its antagonists, insulin, globin, immunoglobulins, catalytic antibodies, the interleukins, insulin-like growth factors, superoxide dismutase, immune responder modifiers, parathyroid hormone and interferon, nerve growth factors, tissue plasminogen activators, and colony stimulating factors.
Alternatively, transfected primary and secondary cells can be used to immunize an individual (i.e., as a vaccine).

The wide variety of uses of cell strains can perhaps most conveniently be summarized as shown below. The cell strains can be used to deliver the following therapeutic products.
1. a secreted protein with predominantly systemic effects;
2. a secreted protein with predominantly local effects;
3. a membrane protein imparting new or enhanced cellular responsiveness;
4. membrane protein facilitating removal of a toxic product;
5. a membrane protein marking or targeting a cell;
6. an intracellular protein;
7. an intracellular protein directly affecting gene expression;
8. an intracellular protein with autolytic effects;
9. gene product-engineered DNA which binds to or sequesters a regulatory protein;
10. a ribozyme; and
11. antisense-engineered RNA to inhibit gene expression.

The transfected primary or secondary cells of the present invention can be used to administer therapeutic proteins (e.g., hormones, enzymes, clotting factors) which are presently administered intravenously, intra-muscularly or subcutaneously, which require patient cooperation and, often, medical staff participation. When transfected primary or secondary cells are used, there is no need for extensive purification of the polypeptide before it is administered to an individual, as is generally necessary with an isolated polypeptide. In addition, the transfected primary or secondary cells produce the therapeutic product as it would normally be produced.

An advantage to the use of the transfected primary or secondary cells is that by controlling the number of cells introduced into an individual, one can control the amount of the product delivered to the body. In addition, in some cases, it is possible to remove the transfected cells if there is no longer a need for the product. A further advantage of treatment by use of the transfected primary or secondary cells is that production of the therapeutic product can be regulated, such as through the administration of zinc, steroids or an agent which affects translation or transcription of a protein, product or nucleic acid product or affects the stability of a nucleic acid product.

Glucagon-like peptide 1 (GLP-1) and glucagon-like peptide 1 derivatives (GLP-1 derivatives) are additional molecules that can be delivered therapeutically using the in vivo protein production and delivery system described in the present invention. GLP-1 derivatives include truncated derivatives GLP-1(7-37), GLP-1(7-36), GLP-1(7-35) GLP-1(7-34) and other truncated carboxy-terminal amidated derivatives and derivatives of GLP-1 which have amino acid substitutions, deletions, additions or other alterations (e.g., addition of a non-amino acid component) which result in biological activity or stability in the blood which is substantially the same as that of a truncated GLP-1 derivative or enhanced biological activity or stability in the blood (greater than that of a truncated GLP-1 derivative). As used herein, the term GLP-1 derivative includes all of the above-described molecules. The term GLP-1 related peptide, as used herein, includes GLP-1 and GLP-1 derivatives. GLP-1 derivatives, also known as insulinotropins or incretins, are normally secreted into the circulation by cells in the gastrointestinal tract. In vivo studies have demonstrated that these peptides function to stimulate insulin secretion and inhibit glucagon secretion from the endocrine pancreas, as well as increase insulin sensitivity in peripheral tissues [Goke, R. et al. (1991) Eur. J. Clin. Inv. 21:135-144; Gutnia). M. et al. (1992) New Engl. J. Med. 326:1316=-1322]. Patients with non-insulin dependent diabetes mellitus (NIDDM) are often treated with high levels of insulin to compensate for their decreased insulin sensitivity. Thus, the stimulation of insulin release and the increase in insulin sensitivity by GLP-1 derivatives would be beneficial for NIDDM patients. Of particular importance is the fact that the insulinotropin-induced stimulation of insulin secretion is strongly dependent on glucose levels, suggesting that these peptides act in response to increases in blood glucose in vivo to potentiate insulin release and, ultimately, lower blood glucose.

### Replacement of a Regulatory Sequence of a Gene by Homologous Recombination

As taught herein, gene targeting can be used to replace a gene's existing regulatory region with a regulatory sequence isolated from a different gene or a novel regulatory sequence synthesized by genetic engineering methods. Such regulatory sequences may be comprised of promoters, enhancers, Scaffold-attachment regions, negative regulatory elements, transcriptional initiation sites, regulatory protein binding sites or combinations of said sequences. (Alternatively, sequences which affect the structure or stability of the RNA or protein produced may be replaced, removed, added, or otherwise modified by targeting, including polyadenylation signals, mRNA stability elements, splice sites, leader sequences for enhancing or modifying transport or secretion properties of the protein, or other sequences which alter or improve the function or stability of protein or RNA molecules).

Several embodiments are possible. First, the targeting event may be a simple insertion of the regulatory sequence, placing the gene under the control of the new regulatory sequence (for example, inserting a new promoter or enhancer or both upstream of a gene). Second, the targeting event may be a simple deletion of a regulatory element, such as the deletion of a tissue-specific negative regulatory element. Third, the targeting event may replace an existing element; for example, a tissue-specific enhancer can be replaced by an enhancer that has broader or different cell-type specificity than the naturally-occurring elements. In this embodiment the naturally occurring sequences are deleted and new sequences are added. In all cases, the identification of the targeting event may be facilitated by the use of one or more selectable marker genes that are contiguous with the targeting DNA, allowing for the selection of cells in which the exogenous DNA has integrated into the host cell genome. The identification of the targeting event may also be facilitated by the use of one or more marker genes exhibiting the property of negative selection, such that the negatively selectable marker is linked to the exogenous DNA, but configured such that the negatively selectable marker flanks the targeting sequence, and such that a correct homologous recombination event with sequences in the host cell genome does not result in the stable integration of the negatively selectable marker. Markers useful for this purpose include the Herpes Simplex Virus thymidine kinase (TK) gene or the bacterial xanthine-guanine phosphoribosyl-transferase (gpt) gene.

The present invention will now be illustrated by the following examples, which are not intended to be limiting in any way.

### EXAMPLES

### EXAMPLE 1. ISOLATION OF FIBROBLASTS

### a. Source of Fibroblasts

Human fibroblasts can be obtained from a variety of tissues, including biopsy specimens derived from liver, kidney, lung and skin. The procedures presented here are optimized for the isolation of skin fibroblasts, which are readily obtained from individuals of any age with minimal discomfort and risk (embryonic and fetal fibroblasts may be isolated using this protocol as well). Minor modifications to the protocol can be made if the isolation of fibroblasts from other tissues is desired.

Human skin is obtained following circumcision or punch biopsy. The specimen consists of three major components: the epidermal and dermal layers of the skin itself, and a fascial layer that adheres to the dermal layer. Fibroblasts can be isolated from either the dermal or fascial layers.

### b. Isolation of Human Fascial Fibroblasts

Approximately 3 cm² tissue is placed into approximately 10 ml of wash solution (Hank's Balanced Salt Solution containing 100 units/ml penicillin G, 100 µg/ml streptomycin sulfate, and 0.5 µg/ml Fungisone) and subjected to gentle agitation for a total of three 10-minute washes at room temperature. The tissue is then transferred to a 100 mm tissue culture dish containing 10 ml digestion solution (wash solution containing 0.1 units/ml collagenase A, 2.4 units/ml grade II Dispase).

Under a dissecting microscope, the skin is adjusted such that the epidermis is facing down. The fascial tissue is separated from the dermal and epidermal tissue by blunt dissection. The fascial tissue is then cut into small fragments (less than 1 mm) and incubated on a rotating platform for 30 min at 37°C. The enzyme/cell suspension is removed and saved, an additional 10 cc of digestion solution is added to the remaining fragments of tissue, and the tissue is reincubated for 30 min at 37°C. The enzyme/cell suspensions are pooled, passed through a 15-gauge needle several times, and passed through a Cellector Sieve (Sigma) fitted with a 150-mesh screen. The cell suspension is centrifuged at 1100 rpm for 15 min at room temperature. The supernatant is aspirated and the disaggregated cells resuspended in 10 ml of nutrient medium (see below). Fibroblast cultures are initiated on tissue culture treated flasks (Corning) at a density of approximately 40,000 cells/cm².

### c. Isolation of Human Dermal Fibroblasts

Fascia is removed from skin biopsy or circumcision specimen as described above and the skin is cut into small fragments less than 0.5 cm². The tissue is incubated with 0.25% trypsin for 60 min at 37°C (alternatively, the tissue can be incubated in trypsin for 18 hrs at 4°C). Under the dissecting microscope, the dermis and epidermis are separated. Dermal fibroblasts are then isolated as described above for fascial fibroblasts.

### d. Isolation of Rabbit Fibroblasts

The procedure is essentially as described above. Skin should be removed from areas that have been shaved and washed with a germicidal solution surgically prepared using accepted procedures.

### EXAMPLE 2. CULTURING OF FIBROBLASTS

### a. Culturing of Human Fibroblasts

When confluent, the primary culture is trypsinized using standard methods and seeded at approximately 10,000 cells/cm². The cells are cultured at 37°C in humidified air containing 5% CO₂. Human fibroblast nutrient medium (containing DMEM, high glucose with sodium pyruvate, 10-15% calf serum, 20 mM HEPES, 20 mM L-glutamine, 50 units/ml penicillin G, and 10 µg/ml streptomycin sulfate) is changed twice weekly.

### b. Culturing of Rabbit Fibroblasts.

The cells are trypsinized and cultured as described for human fibroblasts. Rabbit fibroblast nutrient medium consists of a 1:1 solution of MCDB-110 (Sigma) with 20% calf serum and conditioned medium. Conditioned medium is essentially human fibroblast nutrient medium (with 15% calf serum) removed from rabbit fibroblasts grown in mass culture for 2-3 days.

### EXAMPLE 3. CONSTRUCTION OF A PLASMID (pXGH301) CONTAINING BOTH THE HUMAN GROWTH HORMONE AND NEOMYCIN RESISTANCE GENES

pXGH301 was constructed by a two-step procedure. The SaII-ClaI fragment from pBR322 (positions 23-651 in pBR322) was isolated and inserted into SaII-ClaI digested pcD NEO, introducing a BamHI site upstream of the SV40 early promoter region of pcDNEO. This plasmid, pBNEO was digested with BamHI, and the 2.1 kb fragment containing the neo gene under the control of the SV40 early promoter, was isolated and inserted into BamHI digested pXGH5. A plasmid with a single insertion of the 2.1 kb BamHI fragment was isolated in which neo and hGH are transcribed in the same direction relative to each other. This plasmid was designated pXGH301 (Figure 3).

### EXAMPLE 4. CONSTRUCTION OF A PLASMID (pXEPO1) CONTAINING THE HUMAN ERYTHROPOIETIN GENE UNDER THE CONTROL OF THE MOUSE METALLOTHIONEIN PROMOTER

The expression plasmid pXEPO1 has the hEPO gene under the transcriptional control of the mouse metallothionein (mMT) promoter. pXEPOl is constructed as follows: Plasmid pUC19 (ATCC #37254) is digested with KpnI and BamHI and ligated to a 0.7 kb KpnI-BgIII fragment containing the mouse metallothionein promoter [Hamer, D.H. and Walling, M., J. Mol. Appl. Gen., 1:273-288 (1982). This fragment can also be isolated by known methods from mouse genomic DNA using PCR primers designed from analysis of mMT sequences available from Genbank; i.e. MUSMTI, MUSMTIP, MUSMTIPRM]. The resulting clone is designated pXQM2.

The hEPO gene was isolated by from a bacteriophage lambda clone containing the entire hEPO gene. This bacteriophage was isolated by screening a human Sau3A--partial genomic DNA library (Stratagene) constructed in the lambda vector LAMBDA DASH with 0.77 kb fragment of the human gene. This 0.77 kb fragment was amplified from human genomic DNA using the primers shown below in the polymerase chain reaction (PCR). 
HUMAN EPO PCR PRIMERS:
Oligo hEPO-1:   5'GTTTGCTCAGCTTGGTGCTTG (SEQ ID NO 1)
(positions 2214-2234 in the Genbank HUMERPA sequence)
 
Oligo hEPO-2:   5'TCAAGTTGGCCCTGTGACAT (SEQ ID NO 2)
(positions 2986-2967 in the Genbank HUMERPA sequence)
 (positions 2986-2967 in the Genbank HUMERPA sequence)

The amplified fragment, encompassing exons 4 and 5 of the human EPO gene, was radiolabelled and used to screen the human genomic DNA library. Phage with a 5.4 kb HindIII-BamHI fragment containing the entire human EPO gene were assumed to contain the entire gene, based on published DNA sequence and restriction enzyme mapping data [Lin, F-K., et al., Proc. Natl. Acad. Sci. USA, 82:7580-7584 (1985)].

A 4.8 kb BstEII-BamHI fragment (BstEII site is at position 580 in Genbank HUMERPA sequence; the BamHI site is 4.8 kb.3' of this site, outside of the sequenced region) was isolated from the bacteriophage clone. The purified fragment is made blunt-ended by treatment with the Klenow fragment of E. coli DNA polymerase and ligated to HincII digested pXQM2, which cuts in the pUC19-derived polylinker adjacent to the 3' side of the subcloned mMT promoter. One orientation, in which the ablated BstEII site is proximal to the mMT promoter, was identified by restriction mapping and designated pXEPO1 (Figure 5).

### EXAMPLE 5. STABLE TRANSFECTION OF PRIMARY HUMAN FIBROBLASTS BY MICROINJECTION

Direct injection of DNA into cell nuclei is another method for stably transfecting cells. The ability of primary and secondary human foreskin fibroblasts to be stably transfected by this method has not been previously reported. The 8 kb HindIII fragment from plasmid RV6.9h (Zheng, H. et al., Proc. Natl. Acad. Sci. USA 88:18 8067-8071 (1991)) was purified by gel electrophoresis and passage through an anion exchange column (QIAGEN Inc.). DNA at (10 µg/ml) was injected into primary or secondary human foreskin fibroblasts using 0.1 µm outer diameter glass needles. 41 G418^{r} clones were isolated after injection of 2,000 cells (1 in 49 starting cells).

hGH expressing clones were also generated by microinjection. Plasmid pXGH301 was linearized by Scal digestion (which cuts once within the amp^{r} gene in the pUC12 backbone), purified by passage through an anion exchange column (QIAGEN Inc.), and injected into secondary human foreskin fibroblasts using 0.1 µm outer diameter glass needles. Several DNA concentrations were used, ranging from 2.5-20 µg pXGH301/ml. Twenty G418 resistant clones were isolated after microinjection into 2,100 cells (1 in 105 starting cells). The fraction of G418 cells, is approximately 1% of all cells treated. Nine of 10 clones analyzed were expressing hGH, with average hGH expression being 0.6 µg/10 cells/24 hr for clones isolated in this experiment, and 3 clones were expanded for studying long-term expression of hGH. All 3 were expressing hGH stably, with hGH still being produced through 33, 44, and 73 mpd for the 3 strains, respectively.

The methods described above may be used to obtain stable transfection of primary human fibroblasts with other DNA constructs, for example pE3neoEPO.

### EXAMPLE 6. TRANSFECTION OF PRIMARY AND SECONDARY FIBROBLASTS WITH EXOGENOUS DNA AND A SELECTABLE MARKER GENE BY ELECTROPORATION

Exponentially growing or early stationary phase fibroblasts are trypsinized and rinsed from the plastic surface with nutrient medium. An aliquot of the cell suspension is removed for counting, and the remaining cells are subjected to centrifugation. The supernatant is aspirated and the pellet is resuspended in 5 ml of electroporation buffer (20 mM HEPES pH 7.3, 137 mM NaCl, 5 mM KCl, 0.7 mM Na2HP04, 6 mM dextrose). The cells are recentrifuged, the supernatant aspirated, and the cells resuspended in electroporation buffer containing 1 mg/ml acetylated bovine serum albumin. The final cell suspension contains approximately 3 x 10⁶ cells/ml. Electroporation should be performed immediately following resuspension.

Supercoiled plasmid DNA is added to a sterile cuvette with a 0.4 cm electrode gap (Bio-Rad). The final DNA concentration is generally at least 120 µg/ml. 0.5 ml of the cell suspension (containing approximately 1.5 x 10⁶ cells) is then added to the cuvette, and the cell suspension and DNA solutions are gently mixed. Electroporation is performed with a Gene-Pulser apparatus (Bio-Rad). Capacitance and voltage are set at 960 µF and 250-300 V, respectively. As voltage increases, cell survival decreases, but the percentage of surviving cells that stably incorporate the introduced DNA into their genome increases dramatically. Given these parameters, a pulse time of approximately 14-20 mSec should be observed.

Electroporated cells are maintained at room temperature for approximately 5 min, and the contents of the cuvette are then gently removed with a sterile transfer pipette. The cells are added directly to 10 ml of prewarmed nutrient media (as above with 15% calf serum) in a 10 cm dish and incubated as described above. The following day, the media is aspirated and replaced with 10 ml of fresh media and incubated for a further 16-24 hrs. Subculture of cells to determine cloning efficiency and to select for G418-resistant colonies is performed the following day. Cells are trypsinized, counted and plated; typically, fibroblasts are plated at 10³ cells/10 cm dish for the determination of cloning efficiency and at 1-2 x 10⁴ cells/10 cm dish for G418 selection.

Human fibroblasts are selected for G418 resistance in medium consisting of 300-400 µg/ml G418 (Geneticin, disulfate salt with a potency of approximately 50%; Gibco) in fibroblasts nutrient media (with 15% calf serum). Cloning efficiency is determined in the absence of G418. The plated cells are incubated for 12-14 days, at which time colonies are fixed with formalin, stained with crystal violet and counted (for cloning efficiency plates) or isolated using cloning cylinders (for G418 plates). Electroporation and selection of rabbit fibroblasts is performed essentially as described for human fibroblasts, with the exception of the selection conditions used. Rabbit fibroblasts are selected for G418 resistance in medium containing 1 gm/ml G418.

Fibroblasts were isolated from freshly excised human foreskins. Cultures were seeded at 50,000 cells/cm in DMEM + 10% calf serum. When cultures became confluent fibroblasts were harvested by trypsinization and transfected by electroporation. Electroporation conditions were evaluated by transfection with the plasmid pcDNEO. A representative electroporation experiment using near optimal conditions (60 µg of plasmid pcDNEO at an electroporation voltage of 250 volts and a capacitance setting of 960 µFarads) resulted in one G418 coloney per 588 treated cells (0.17% of all cells treated), or one G418 colony per 71 clonable cells (1.4%).

When nine separate electroporation experiments at near optimal conditions (60 µg of plasmid pcDneo at an electroporation voltage of 300 volts and a capacitance setting of 960 µFarads) were performed, an average of one G418 colony per 1,899 treated cells (0.05%) was observed, with a range of 1/882 to 1/7,500 treated cells. This corresponds to an average of one G418 colony per 38 clonable cells (2.6%).

Low passage primary human fibroblasts were converted to hGH expressing cells by co-transfection with plasmids; pcDNEO and pXGH5. Typically, 60 µg of an equimolar mixture of the two plasmids were transfected at near optimal conditions (electroporation voltage of 300 volts and a capacitance setting of 960 µFarads). The results of such an experiment resulted in one G418 colony per 14,705 treated cells.

hGH expression data for these and other cells isolated under identical transfection conditions are summarized below. Ultimately, 98% of all G418^{r} colonies could be expanded to generate mass cultures.

| | |
|---|---|
| Number of G418^{r} Clones Analyzed | 154 |
| Number of G418^{r}/hGH Expressing Clones | 65 |
| Average hGH Expression Level | 2.3 µg hGH/10⁶ Cells/24 hr |
| Maximum hGH Expression Level | 23.0 µg hGH/10⁶ Cells/24 hr |

Stable transfectants also have been generated by electroporation of primary or secondary human fibroblasts with pXGH301, a DNA construct in which the neo and hGH genes are present on the same plasmid molecule (Example 3). For example, 1.5 x 10⁶ cells were electroporated with 60 µg pXGH301 at 300 volts and 960 µFarads. G418 resistant colonies were isolated from transfected secondary fibroblasts at a frequency of 652 G418 resistant colonies per 1.5 x 10 treated cells (1 per 2299 treated cells). Approximately 59% of these colonies express hGH.

### EXAMPLE 7. ISOLATION OF TRANSFECTANTS IN THE ABSENCE OF SELECTION

Stable transfection of primary fibroblasts with the plasmid pXGH5 renders recipient fibroblasts capable of secreting human growth hormone (hGH) into the surrounding medium. Therefore, a radioimmunoassay for hGH (or for any expressed protein) can be used as a simple and rapid screen for transfectants as an alternative to the use of selective markers and selective agents. In addition, it should be possible to determine the true frequency of stable integration of exogenous DNA using a screening method such as PCR which does not necessarily rely on gene expression.

The results of experiments to be discussed below demonstrated that it was indeed possible to isolate transfectants stably expressing hGH without selection of a cotransfected plasmid. These experiments have been successful for primary human foreskin fibroblasts and primary rabbit skin fibroblasts. The frequency of stably expressing transfectants was approximately 1 in 10 colonies.

### 1. Human Foreskin Tissue

Approximately 2.0 x 10⁶ human cells that were freshly dissociated from tissue were electroporated with 60 µg of pXGH5 at 300 volts, 960 µFarads. The cells were plated immediately in a 100 mm tissue culture dish containing 10 ml of prewarmed medium and incubated at 37°C in a humidified 5% CO₂ atmosphere. Two days following transfection, 5 x 10³ cells were subcultured into a 24 well cloning plate (Bellco Glass Co.). Each well of the 24 well plate contained 16 smaller wells (384 wells/plate). Eight days later 10 of 24 large wells were screened for hGH expression via radioimmune assay. All ten wells exhibited significant hGH expression. The media was aspirated, replaced with fresh media and assayed for hGH 24 hours later. Again all ten wells were expressing significant levels of hGH. Colony counts were performed for each of the ten wells at this time. An average of 11.5 colonies per large well was observed. If one assumes a minimum of one stable transfectant per well, a lower limit frequency of transfection of approximately 8-9% of clonable cells can be calculated.

Individual colonies in each of the 16 small wells within one of these larger wells were trypsinized and transferred to 16 wells of a 96 well plate. Three days later each of these wells was assayed for hGH expression. One of the 16 was found to contain cells expressing hGH. The cells in this well were expanded in culture. These cells, HF26-19M, were producing 260 ng hGH/10⁶ cells/24 hr after 42 mpd in culture.

The above experiment was repeated using another primary human foreskin fibroblast culture. From this experiment a second clone expressing hGH was isolated. After 24 mpd in culture these cells, HF24-GHl, were producing 60 ng hGH/10 cells/24 hr. hGH production continued until the cells reached 45 mpd, at which point the cells were frozen away. The lower limit transfection frequency was similar to the first experiment (6-7% of clonable cells).

### 2. Primary Rabbit Fibroblasts

Primary rabbit skin cells were transfected with pXGH5. The electroporation conditions were identical to the human tissue electroporation described above. 1 x 10³ cells were subcultured into a 384 well plate. Seven days later, hGH assays were performed for 10 of the 24 larger wells. Nine of the ten wells were expressing hGH. One of these wells was chosen for colony isolation. This well contained nine colonies dispersed among the 16 small wells. Cells in each of the 16 small wells were trypsinized and transferred to 16 wells of a 96 well plate. subsequent hGH assays showed that one of these 16 wells was expressing hGH. This clone was expanded in culture. hGH production was at 58 ng/10 cells/24 hr after 35 mpd in culture. The estimated transfection frequency for this experiment was 1 colony expressing hGH out of nine total colonies (11%).

### EXAMPLE 8. LONG TERM IN VITRO hGH PRODUCTION BY CELL STRAINS DERIVED FROM TRANSFECTED PRIMARY HUMAN SKIN FIBROBLASTS

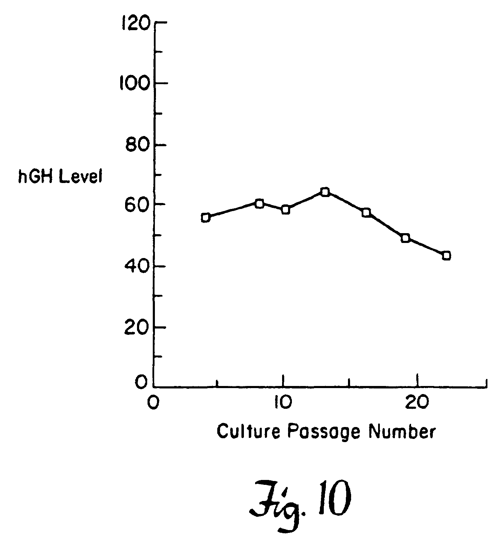

Fibroblasts were isolated from freshly excised human skin fibroblasts and cultured in DMEM + 10% calf serum. Electroporation (250 volts, 960 µFarads) with 60 µg of an equimolar mixture of pcDNEO and pXGH5 was performed and treated cells were selected in G418-containing medium (300 µg/ml G418). Colonies were isolated and expanded using standard methods, and the resulting primary cell strains were subcultured repeatedly in order to monitor the stability of hGH expression as a function of time in culture. Data derived from two such strains, HF96-11 and HF96-23, are shown in Figure 9. Cells were maintained in a low level of G418 (75 µg/ml G418) in DMEM + 10% calf serum and subcultured at a seeding density of 10,000 cells/cm². Culture medium was changed 24 hr prior to harvesting the cells for passaging. At the time of passage an aliquot of the culture medium was removed for hGH assay and the cells were then harvested, counted, and reseeded. hGH concentration in the medium was determined using a commercially available immunoassay. hGH levels (expressed as µg hGH/10⁶ cells/24 hr) were plotted for various culture passage numbers from 9 to 28 passages. Results of these assays show that hGH expression remains remarkably constant throughout this extended in vitro culture interval (for example, data derived from two different strains show that HF96-11 at passage 28 reached 69 mpd, while HF96-23 at passage 27 reached 76 mpd).

### EXAMPLE 9. LONG TERM IN VITRO hGH PRODUCTION BY CELL STRAINS DERIVED FROM TRANSFECTED PRIMARY AND SECONDARY RABBIT SKIN FIBROBLASTS

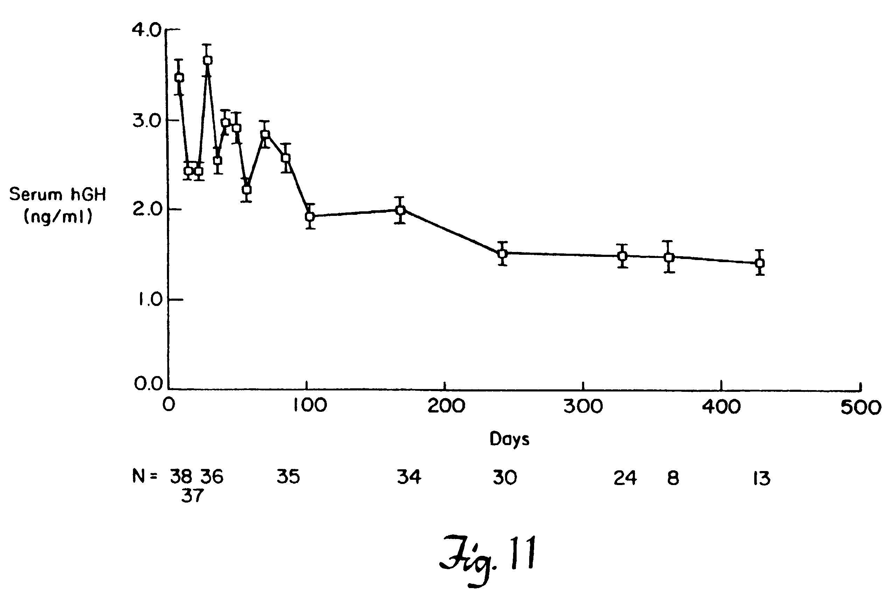

Fibroblasts were isolated from freshly excised rabbit skin and cultured in DMEM + 10% calf serum. Electroporation (250 volts, 960 µFarads) with 60 µg of an equimolar mixture of pcD NEO and pXGH5 was performed and treated cells were selected in G418-containing medium (1 mg/ml G418). Colonies were isolated using cloning cylinders and expanded using standard methods, and the resulting primary cell strains were subcultured repeatedly in order to monitor the stability of hGH expression as a function of time in culture. Data derived from one such strain, RF40/1-3 are shown in Figure 10. RF40/1-3 cells were maintained in the absence of selection in rabbit fibroblast nutrient medium and subcultured at a seeding density of 10,000 cells/cm². Culture medium was changed 24 hr prior to harvesting the cells for passaging. At the time of passage an aliquot of the culture medium was removed for hGH assay and the cells were then harvested, counted, and reseeded. hGH concentration in the medium was determined using a commercially available immunoassay. (Nichols Institute) hGH levels (expressed as µg hGH/10⁶ cells/24 hr) are plotted for culture passage numbers, 4, 8, 10, 13, 16, 19, and 22, corresponding to culture mean population doubling (mpd) levels of approximately 28 through 84. The results of these assays demonstrate that hGH expression remains remarkably constant throughout this extended in vitro culture interval and virtually identical to the levels observed at approximately 20 mpd in culture (47 µg hGH/10⁶ cells/24 hr).

### EXAMPLE 10. LONG-TERM EXPRESSION OF HUMAN GROWTH HORMONE IN MICE

The nude mouse provides a valuable system to study implants of genetically engineered cells for their ability to deliver therapeutically useful proteins to an animal's general circulation. The relative immune-incompetence of these animals may allow certain primary and secondary rabbit fibroblasts to survive in vivo for extended periods.

For implantation of cells into the subrenal capsule, mice are given intraperitoneal injection of Avertin (a solution of 2% w/v 2.2.2 tribromoethanol and 2% v/v 2-methyl, 2-butanol.) at a dose of 0.017 ml/g body weight. The kidney (generally the left kidney) is approached through an 8-10 mm incision made approximately 3 mm below the rib cage. The skin, abdominal musculature, peritoneum, and perirenal fascia are retracted to expose the kidney. A small forcep is used to pull the kidney out of the abdominal cavity. A 27-gauge hypodermic needle is used to make a small opening in the renal capsule. Using a 20-gauge I.V. catheter, cells to be implanted (typically 3 million cells in a volume of 5-10 µl) are withdrawn into a 1 ml syringe and slowly ejected under the renal capsule. Care is taken to ensure that the cells are released distal to the opening in the renal capsule. The incision is closed with one staple through the musculature and the skin. Blood is collected after placing the mouse in a large beaker containing methoxyflurane until light anesthesia is achieved. The tip of a Pasteur pipette is placed between the eye and the periorbital space to collect blood from the orbital sinus. Serum hGH levels are determined using a commercially available kit (Nichol's Institute).

In our initial experiment, a nude mouse was implanted with 5 million transfected rabbit fibroblast cells (strain RF20-11). This mouse has displayed detectable hGH in its serum for one year. The time course of expression is shown below.

| Month | 1 | 2 | 3 | 4 | 6 | 12 |
|---|---|---|---|---|---|---|
| Serum hGH (ng/ml): | 0.7 | 0.7 | 0.8 | 0.6 | 0.7 | 0.6 |

A larger experiment was then performed in which animals were implanted with a rabbit fibroblast strain (RF40/1-3) expressing 96 µg/10 cells/24 hr. Results from this experiment are shown in Figure 11. The bars represent standard errors, and N indicates the number of mice bled at each time point. These results demonstrate that average serum hGH levels remained relatively constant for over 14 months, averaging from 1-3 ng/ml since implantation. No side effects of any type arising from the implanted cells have been observed over this time period.

### EXAMPLE 11. RECULTURING hGH PRODUCING CELLS FROM SUBRENAL CAPSULE IMPLANTS

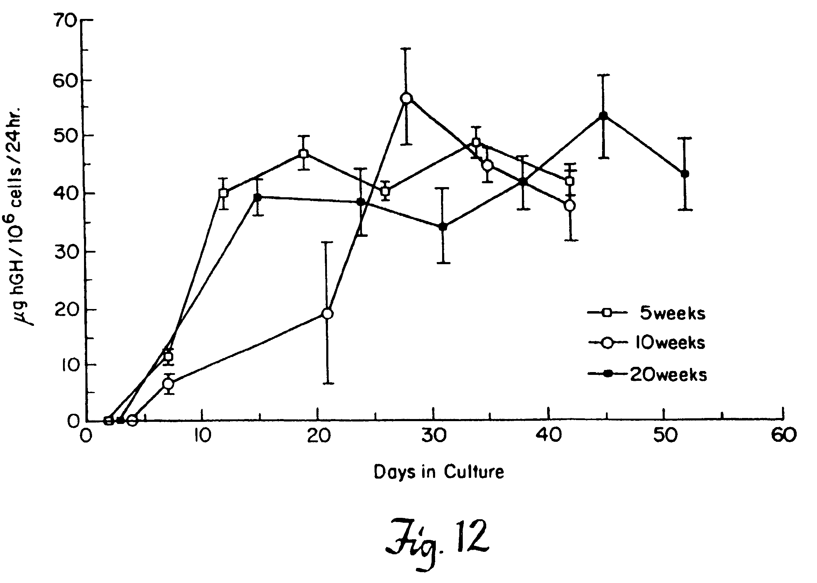

A stringent test of the viability of implanted cells is their ability to grow and display their preimplantation properties when removed from animals and recultured in vitro. RF40/1-3 cells (3 x 10⁶ cells per mouse) expressing 24.5 µg hGH/10⁶ cells/24 hr were implanted under the subrenal capsule of nude mice. These cells had undergone 49 mpd in culture prior to implantation. After either 5 or 10 weeks in vivo, the animals were sacrificed, kidneys were harvested, and cells in the vicinity of the implant (clearly visible on the surface of the kidney) were microdissected out. The resulting tissue was subjected to a scaled-down version of a standard enzymatic cell dispersion protocol (Example 6), and the single cell suspension was placed in G418-containing medium. Cell culture data was obtained from 5 recultured implants from mice having 5-week-old implants, and 4 recultured implants from mice having 10-week-old implants. The recovered cells were propagated for 19 mpd in culture and the hGH levels were analyzed at various times after placing in culture, ranging from about 10 to about 50 days in culture. Results from these experiments are shown in Figure 12. Each point in Figure 12 represents the average (with standard deviation) of at least four separate recovery experiments. In experiments involving both the 5-week-old implants and the 10-week-old implants, G418^{r} cells expressing high levels of hGH were recovered, with hGH expression being relatively stable over the 19 in vitro population doublings post-recovery.

### EXAMPLE 12. EXPRESSION OF HUMAN GROWTH HORMONE AND PRODUCTION OF HIGH TITER ANTI-hGH ANTI-SERA IN RABBITS IMPLANTED WITH TRANSFECTED AUTOLOGOUS CELLS

An experiment that explores all of the technical and logistical requirements for performing autologous gene therapy was performed in rabbits. First, skin biopsies were taken from 8 living rabbits. Skin fibroblasts were isolated and placed in primary culture. After one passage in vitro, cells were transfected with either pXGH301 or cotransfected with pXGH5 and pcDNEO. G418 resistant clones were isolated and analyzed for hGH expression. Clones expressing greater than 10 µg/10⁶ cells/day were expanded into roller bottles. Finally, cells from each clone were harvested from roller bottles and prepared for either SRC implantation or IP injection into the same rabbit used as the donor for the skin biopsy.

To isolate rabbit skin fibroblasts, the rabbit is anesthetized with an intramuscular injection of Ketamine-HCl (50 mg/kg body weight) and Xylazine-HCl (5 mg/kg body weight) until sedation is achieved. The animal is shaved in the area, for example, above the right pelvic joint and prepped for surgery using accepted procedures. Two arc-shaped incisions are made approximately 3 cm apart which are joined at the dorsal and ventral areas to form an oval-shaped patch. A serrated forcep is used to remove the epidermis and a small scissor is used to remove the underlying fascia. The dermis and fascia are placed in culture medium and the incision is closed with a 3-0 nylon suture. Rabbits are placed on a heating pad for recovery. Fascial fibroblasts are cultured, transfected, and selected as described in Example 1, 2, and 4.

For implantation of cells into the renal capsule, the rabbits are anesthetized as described above. The animal is placed in a right side lateral recumbent position exposing the area below the left rib cage. The animal is shaved and prepped according to accepted procedures. The left kidney is approached through a 6 cm dorso-ventral incision approximately 2 cm below the twelfth rib. The incision is carried through the abdominal musculature, peritoneum, and peri-renal fat and fascia. A Balfour retractor is used to maintain the abdominal opening while dissection of peri-renal fat and fascia is performed. Using an 11-gauge surgical blade, a 2-3 mm incision is made at the surface of the renal capsule. Using a 20-gauge I.V. catheter, cells to be implanted (typically 100-200 million cells in a volume of 165-660 µl) are drawn into a 1 ml syringe and slowly ejected under the renal capsule. Care is taken to ensure that the cells are released distal to the opening in the renal capsule. The peritoneal cavity is closed by suturing the peritoneum and abdominal muscle with 3-0 adsorbable chromic gut The skin is closed with a 3-0 nylon suture and a sterile gauze bandage and antibiotic ointment (Bactrin) is applied to the wound. Rabbits are placed on a heating pad for recovery. Systemic antibiotics (Tribrissin) are administered after skin biopsy and implantation. For introducing cells by the intraperitoneal (IP) route, cells (700-1100 million in a volume of 3.5-5.5 ml) are injected through a 22-gauge needle. Blood is collected from the middle auricular artery in restrained animals using a 22-gauge needle.

The relevant information on hGH expression levels and numbers of cells introduced into animals is listed in Table 1. In all eight animals, serum hGH levels were readily detectable. By day 14, no hGH could be detected in any animal. The eventual disappearance of hGH in the serum of these animals was expected, since human growth hormone is known to be antigenic in rabbits. Serum samples were therefore assayed for anti-hGH antibodies (quantified by an anti-rabbit IgG ELISA). In each case, the decrease in serum hGH levels coincides with a rise in anti-hGH antibodies.

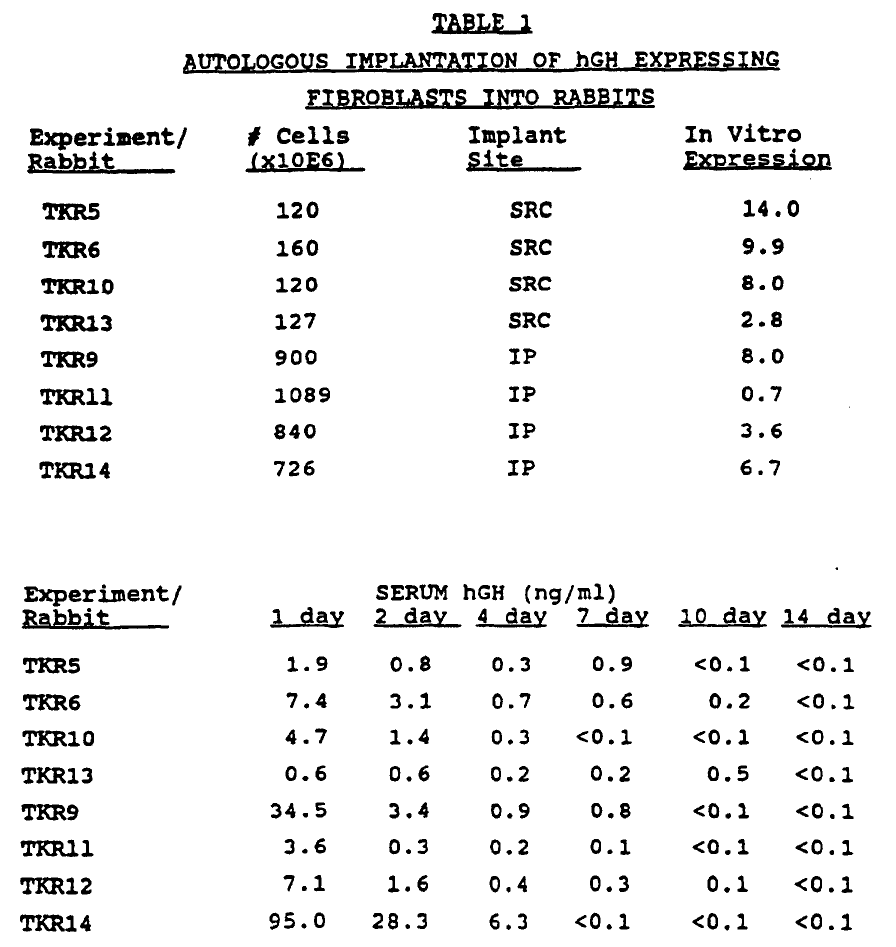

These results indicate that there appears to be no technical barrier to performing autologous gene therapy in rabbits using transfected skin fibroblasts. hGH delivery by genetically engineered autologous cells was successful in all 8 experimental animals. As expected, serum hGH levels decreased concomitantly with a rise in anti-hGH antibodies. High titers (1:40,000) were not uncommon in animals with IP or SRC implants, consistent with the proposal that continuous delivery of proteins by a single implantation treatment can be an efficient method for vertebrate vaccination and the production of high titer antisera against protein antigens.

### EXAMPLE 13. IN VITRO hEPO PRODUCTION BY TRANSFECTED SECONDARY HUMAN AND RABBIT SKIN FIBROBLASTS

### 1. Human Skin Fibroblasts

Fibroblasts were isolated from freshly excised human skin fibroblasts and cultured in DMEM + 15% calf serum. Electroporation (250 volts, 960 µFarads) with 60 µg of an equimolar mixture of pcDNEO and pXEPO1 was performed on passage 1 cells and treated cells were selected in G418-containing medium (300 µg/ml G418). Colonies were isolated and expanded using standard methods. Data derived from an analysis of fifty-six individual clones is shown in Table 2. Cells were maintained in G418 (300 µg/ml G418) in DMEM + 15% calf serum and subcultured at a seeding density of 10,000 cells/cm². Culture medium was changed 24 hr prior to harvesting the cells for passaging. At the time of passage, an aliquot of the culture medium was removed for hEPO assay and the cells were then harvested, counted, and reseeded. hEPO concentration in the medium was determined using a commercially available ELISA (R & D Systems). hEPO levels are expressed as mU/10⁶ cells/24 hr., and expression levels ranged from 69 to 55,591 mU/10⁶ cells/24 hr. 19% of all G418-resistant colonies expressed detectable levels of hEPO.

**TABLE 2**

| hEPO EXPRESSION IN FIFTY-SIX INDEPENDENT SECONDARY HUMAN FIBROBLAST CLONES ISOLATED BY CO-TRANSFECTION WITH pcDNEO AND pXEPO1 | |
|---|---|
| HEPO Expression Level (mU/10⁶cells/24 hr) | Number of Clones |
| <1,000 | 10 |
| 1,000-10,000 | 28 |
| 10,000-50,000 | 17 |
| >50,000 | 1 |

Clonally derived human fibroblasts isolated by co-transfection with pcDneo and pXEPO1 were analyzed for the glycosylation state of secreted hEPO. Media was collected from hEPO producing cells and immunoprecipitated with a mouse monoclonal antibody (Genzyme Corporation) specific for human erythropoietin. The immunoprecipitated material was subject to electrophoresis on a 12.5% polyacrylamide gel and transferred to a PVDF membrane (Millipore). The membrane was probed with the same anti-hEPO monoclonal antibody used for immunoprecipitation and was subsequently treated with an HRP-conjugated sheep anti-mouse IgG antisera (Cappel), followed by luminescent detection (ECL Western blotting detection kit; Amersham) to visualize hEPO through the production of a fluorescent product.

Western blot analysis of hEPO secreted by normal human fibroblasts co-transfected with pEXPO1 and pcDNEO demonstrated that a molecule with a molecular mass of approximately 34 kd reacts with an antibody specific for human erythropoietin. This is the expected size for naturally occurring, fully glycosylated human erythropoietin.

hEPO produced by transfected human fibroblast clones was further analyzed to determine if the secreted material had both N- and O-linked glycosylation characteristic of natural human erythropoietin isolated from urine or recombinant hEPO produced by chinese hamster ovary cells. Western blot analysis was performed on the supernatent from a clonal strain of normal human fibroblasts co-transfected with pXEPO1 and pcDNEO. Samples of the supernatent were first treated with either endoglycosidase-F (New England Nuclear), neuraminidase Genzyme), or with O-glycanase (Genzyme). Treatment with endoglycosidase-F results in a shift in molecular weight from 34 kd to approximately 27 kd. Treatment with neuraminidase results in a barely detectable shift in band position, while treatment with O-glycanase further shifts the size of the immunoreactive band down to approximately 18.5 kd. These results are indistinguishable from those obtained with natural human erythropoietin isolated from urine or recombinant hEPO produced by Chinese hamster ovary cells (Browne, J.K. et al., Cold Spring Harbor Symp. Quant. Biol. 51:693-702 (1986)).

### 2. Rabbit Fibroblasts

Fibroblasts were isolated from freshly excised rabbit skin and cultured in DMEM 10% calf serum. Electroporation (250 volts, 960 µFarads) with 60 µg of an equimolar mixture of pcDNEO and pXEPO1 was performed and treated cells were selected in G418-containing rabbit fibroblast growth medium (1 mg/ml G418; Example 2). Colonies were isolated and expanded using standard methods, and the resulting secondary cell strains were analyzed for hEPO expression. Data derived from forty-nine independent rabbit fibroblast clones is shown in Table 3. Expression levels in these clones ranged from 43 to 2,900,000 mU/10⁶ cells/24 hr., and 72% of all G418-resistant clones expressed detectable levels of hEPO.

**TABLE 3**

| hEPO EXPRESSION IN FORTY-NINE INDEPENDENT SECONDARY RABBIT FIBROBLAST CLONES ISOLATED BY CO-TRANSFECTION WITH pcDNEO AND pEEPO | |
|---|---|
| hEPO Expression Level (mU/10⁶ cells/24 hr) | Number of Clones |
| <1,000 | 1 |
| 1,000-10,000 | 3 |
| 10,000-50,000 | 7 |
| 50,000-500,000 | 19 |
| >500,000 | 19 |

### EXAMPLE 14. CONSTRUCTION OF A PLASMID CONTAINING BOTH THE HUMAN EPO GENE AND THE NEOMYCIN RESISTANCE GENE

A 6.9 kb HindIII fragment extending from positions 11,960-18,869 in the HPRT sequence [Genbank entry HUMHPRTB; Edwards, A. et al., Genomics, 6:593-608 (1990)] and including exons 2 and 3 of the HPRT gene, is subcloned into the HindIII site of pUC12. The resulting clone is cleaved at the unique Xhol site in exon 3 of the HPRT gene fragment and the 1.1 kb SaII-XhoI fragment containing the neo gene from pMClNEO (Stratagene) is inserted, disrupting the coding sequence of exon 3. One orientation, with the direction of neo transcription opposite that of HPRT transcription was chosen and designated pE3Neo. pE3neo has a unique XhoI site at the junction of HPRT sequences and the 5' side of the neo gene. pE3neo is cut with XhoI and made blunt-ended by treatment with the Klenow fragment of E. coli DNA polymerase.

To insert the hEPO gene into the neo selection plasmid pE3Neo, a 5.1 kb EcoRI-HindIII fragment was isolated from plasmid pXEPOl (Example 3; Figure 5). The EcoRI site is located adjacent to the 5' side of the mMT promoter, and the HindIII site is located 5.1 kb away, 3' to the hEPO coding region. The purified Fragment is made blunt-ended by treatment with Klenow fragment of E. coli DNA polymerase and ligated to the XhoI digested and blunt-ended pE3neo fragment described above. After transformation into E. coli, a plasmid with one copy of the mMT-hEPO fragment inserted into pE3neo was identified by restriction enzyme analysis in which the hEPO gene is transcribed in the same orientation as the adjacent neo gene. This plasmid was designated pE3neoEPO. In addition to allowing direct selection of hEPO expressing G418^{r} clones, this fragment may also be used in gene targeting to direct the integration of the hEPO gene to the human HPRT locus.

### EXAMPLE 15. ISOLATION OF HUMAN FIBROBLAST CLONES EXPRESSING THE hEPO GENE AND A SELECTABLE MARKER (pE3neoEPO)

Fibroblasts were isolated from freshly excised human skin fibroblasts and cultured in DMEM + 15% calf serum. Electroporation (250 volts, 960 µFarads) with 60 µg of supercoiled pE3neoEPO was performed on passage 1 cells and treated cells were selected in G418-containing medium (300 µg/ml G418). Colonies were isolated and expanded using standard methods. Data derived from an analysis of twenty-six individual clones is shown in Table 4. Cells were maintained in G418 (300 µg/ml G418) in DMEM + 15% calf serum and subcultured at a seeding density of 10,000 cells/cm². Culture medium was changed 24 hr prior to harvesting the cells for passaging. At the time of passage an aliquot of the culture medium was removed for hEPO assay and the cells were then harvested, counted, and reseeded. hEPO concentration in the medium was determined using a commercially available ELISA (R and D Systems). hEPO levels are expressed as mU hEPO/10⁶ cells/24 hr, and expression levels ranged from 240 to 961,620 mU/10⁶ cells/24 hr. 89% of all G418-resistant clones expressed detectable levels of hEPO.

**TABLE 4**

| hEPO EXPRESSION IN TWENTY-SIX INDEPENDENT SECONDARY HUMAN FIBROBLAST CLONES ISOLATED BY TRANSFECTION WITH pE3neo-EPO | |
|---|---|
| hEPO Expression Level (mU/10⁶ cells/24 hr) | Number of Clones |
| <1,000 | 2 |
| 1,000-10,000 | 2 |
| 10,000-50,000 | 9 |
| 50'000-500,000 | 12 |
| >500,000 | 1 |

hEPO expressing human fibroblast clones are also isolated by electroporation with 60 µg of HindIII digested pE3neoEPO. hEPO expressing rabbit fibroblast clones are isolated using plasmid pE3neoEPO under identical transfection conditions, with the exception that rabbit fibroblast clones are selected in rabbit fibroblast growth medium (Example 2) containing 1 mg/ml G418.

### EXAMPLE 16. EXPRESSION OF BIOLOGICALLY ACTIVE HUMAN ERYTHROPOIETIN IN MICE

The mouse provides a valuable system to study implants of genetically engineered cells for their ability to deliver therapeutically useful proteins to an animal's general circulation. The relative immuneincompetence of nude mice allow xenogeneic implants to retain biologic function and may allow certain primary and secondary rabbit fibroblasts to survive in vivo for extended periods.

For implantation of cells into the subrenal capsule, mice are given intraperitoneal injection of Avertin at a dose of 0.0175 ml/g body weight. The kidney (generally the left kidney) is approached through an 8-10 mm incision made approximately 3 mm below the rib cage. The skin, abdominal musculature, peritoneum, and peri-renal fascia are retracted to expose the kidney. A small forcep is used to pull the kidney out of the abdominal cavity. A 27-gauge hypodermic needle is used to make a small opening in the renal capsule. Using a 20-gauge I.V. catheter, cells to be implanted (typically 3 million cells in a volume of 5-10 µl) are withdrawn into a 1 ml syringe and slowly ejected under the renal capsule. Care is taken to ensure that the cells are released distal to the opening in the renal capsule. The incision is closed with one staple through the musculature and the skin. Blood is collected after placing the mouse in a large beaker containing methoxyflurane until light anesthesia is achieved. The tip of a Pasteur pipette is placed between the eye and the periorbital space to collect blood from the orbital sinus. Serum hEPO levels are determined using a commercially available kit (R and D Systems). An aliquot of blood is also drawn into EDTA coated capillary tubes (Statspin, Norwood, MA) for determination of hematocrit levels.

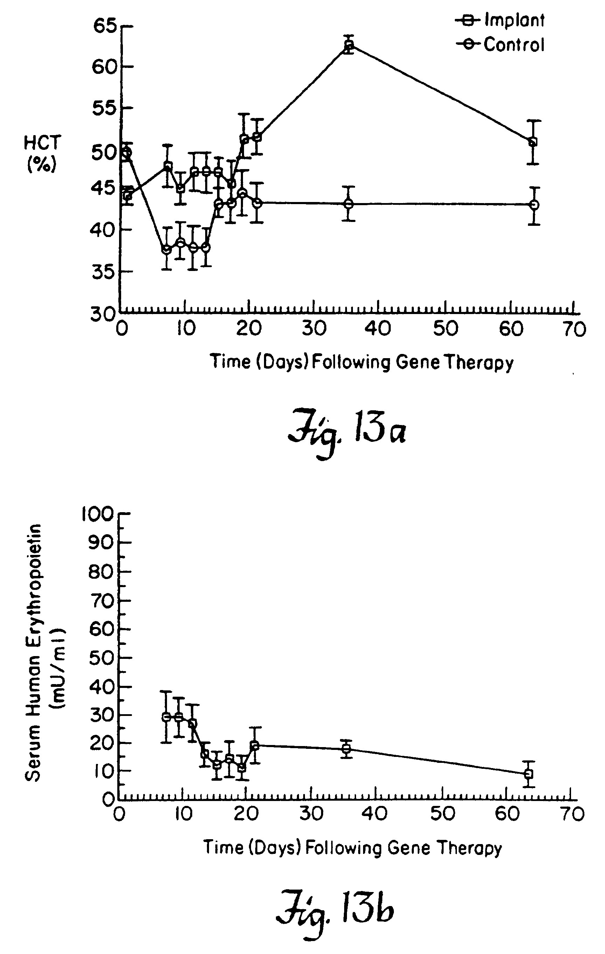

A clonal strain of rabbit skin fibroblasts was isolated by the methods described in Example 13. One clone, designated RF115-D4, was determined to be stably transfected with the human EPO gene and expressed approximately 786,000 mU hEPO/10⁶ cells/24 hr. Three million cells were implanted into the subrenal capsule in each of 15 six nude mice. Approximately 400 µl of blood was drawn on days 1 and 7 after implantation and on every other day thereafter until day 21. During this time an equal volume of saline solution was injected after bleeding to prevent hypotonic shock. Blood was drawn weekly thereafter until day 63. An identical bleeding schedule was used on ten mice that had no cells implanted. Figure 13a shows the effect of these treatments on blood hematocrit (HCT), a commonly used indicator of red blood cell number, was measured in implanted and control animals. In control animals, HCT drops dramatically by day 7, followed by a return to approximately normal levels by day 15. In contrast, animals receiving implants of the hEPO expressing cells showed elevated HCT levels by day 7. HCT remained elevated through day 63, reaching a peak of 64%, or 1.4 times higher than the day 1 level of 45%, on day 35 after implantation. As shown in Figure 13b, immunoreactive hEPO was readily detectable in the blood of implanted animals (the sensitivity of the hEPO ELISA has been determined to be 2 mU/ml by the kit's manufacturer (R and D Systems) and endogenous mouse EPO shows no cross-reactivity with the antibodies used in the ELISA kit). hEPO levels in the implanted animals dropped gradually, from 29 to 9 mU/ml, from days 7 to 63 postimplantation.

This Example clearly demonstrates that normal skin fibroblasts that have been genetically engineered to express and secrete hEPO can: 1) survive in vivo to deliver hEPO to an animals systemic circulation for up to 2 months, and 2) the hEPO produced is biologically functional, serving to prevent the drop in hematocrit observed in the frequently bled control animals, and resulting in a net increase in HCT even when animals were challenged with a bleeding schedule that produces an anemic response in control animals.

### EXAMPLE 17. EXPRESSION OF GLP-1(7-37) FROM SECONDARY HUMAN SKIN FIBROBLASTS STRAINS AFTER TRANSFECTION WITH A GLP-1(7-37) EXPRESSION PLASMID

The portion of GLP-1 from amino acid residues 7 to 37 [GLP-1(7-37); encoded by base pairs 7214 to 7306 in Genbank sequence HUMGLUCG2] has been demonstrated to have insulinotropin activity in vivo. Plasmid pXGLP1 is constructed such that the GLP-1(7-37) moiety is fused at its N-terminus to a 26-amino acid signal peptide derived from human growth hormone for efficient transport through the endoplasmic reticulum. The fusion protein is cleaved immediately C-terminal to residue 26 prior to secretion, such that the secreted product consists solely of residues 7-37 of GLP-l. Expression of the signal peptide: GLP-1(1-37) fusion protein is controlled by the mouse metallothionein promoter.

Plasmid pXGLP1 is constructed as follows: Plasmid PXGH5 [Selden, R.F. et al., Mol. Cell. Biol. 6:3173-3179 (1986)] is digested with SmaI and ligated to a doublestranded oligonucleotide containing a BgIII site (BgIII linkers; New England Biolabs). The ligated product is digested with BgIII and EcoRI and the 0.32 kb fragment corresponding to the 3'-untranslated region of the human growth hormone gene is isolated (with a BgIII linker attached to the SmaI site lying at position 6698 in Genbank entry HUMGHCSA). The hGH fragment can also be isolated by known methods from human genomic DNA using PCR primers designed to amplify the sequence between positions 6698 to 7321 in Genbank entry HUMGHCSA. A 1.45 EcoRI-BgIII fragment containing the mouse metallothionein (mMT) promoter [Hamer, D.H. and Walling, M., J. Mol. Appl. Gen., 1:273- 288 (1982)] is next isolated. The mouse metallothionein promoter may be isolated by known methods from mouse genomic DNA using PCR primers designed from analysis of mMT sequences available from Genbank (i.e. Genbank entries MUSMTI, MUSMTIP, and MUSMTIPRM). Plasmid pUC19 (ATCC #37254) is digested with EcoRI and treated with bacterial alkaline phosphatase. The treated plasmid is ligated with the hGH and mMT fragments described above. The resulting plasmid has a single copy of each the mouse metallothionein promoter and the 3'untranslated region of hGH joined at a BgIII site.

This plasmid, designated pX1 is digested with BgIII and the full-length linear product is purified by gel electrophoresis. Oligonucleotides 11.1 and 11.2 are used to amplify a DNA sequence encoding amino acids 7-37 of GLP-1 from human genomic DNA by PCR. The amplified product (104 bp) is purified and mixed with pXGH5 and oligonucleotides 11.2, 11.3, 11.4, and 11.5 and subject to PCR. Oligonucleotides 11.3 and 11.4 are complementary and correspond to the desired junction between the hGH signal peptide and GLP-1 amino acid residue 7. The 500 base pair amplification product contains 5'-untranslated, exon 1, intron 1, and part of exon 2 sequences from hGH (nucleotides 5168 to 5562 in Genbank entry HUMGHCSA) 15 fused to a sequence encoding GLP-1 residues 7-37 followed by a stop codon. The fragment, by design, is flanked on both ends by BamHI sites.

The fragment is cleaved with BamHI and ligated to the BgIII digest of pX1 described above. Ligation products are analysed to identify those with one copy of the hGH-GLP-1(7-37) fusion product inserted at the BgIII site separating the mMT promoter and the 3'-untranslated hGH sequence in pX1, such that GLP-1 residue 37 is distal to the mMT promoter.

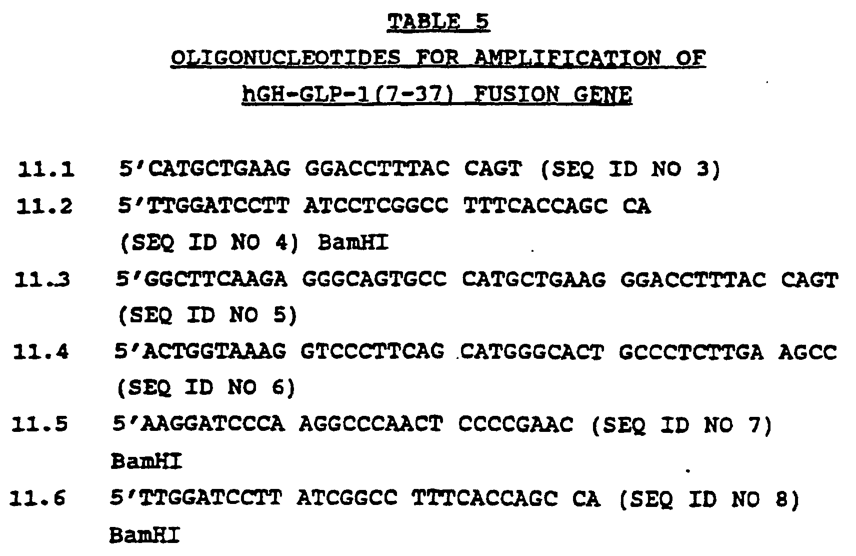

Alternatively, the small sizes of the signal peptide and GLP-1 moieties needed allow complete fusion genes to be prepared synthetically. DNA encoding the signal peptides of the LDL receptor (amino acid residues 1-21), preproglucagon (amino acid residues 1-20), or human growth hormone (amino acid residues 1-26) may be synthesized by known methods and ligated in vitro to similarly synthesized DNA encoding amino acids 7-37 or 7-36 of GLP-1 (followed immediately by a stop codon). The sequences necessary to design and synthesize these molecules are readily available in Genbank entries HUMLDLR01 (human LDL receptor), HUMGLUCG2 (human GLP-1 and glucagon sequences), and HUMGHCSA (human growth hormone). The ligated product may be inserted into a suitable mammalian expression vector for use in human fibroblasts. Plasmid pMSG (Pharmacia LKB Biotechnology, Piscataway, NJ) is suitable for this purpose, having 5' and 3'untranslated sequences, a splice site, a polyA addition site, and an enhancer and promoter for use in human skin fibroblasts. Alternatively, the ligated product may be synthesized with an appropriate 5'-untranslated sequence and inserted into plasmid pX1 described above.

A second insulinotropic GLP-1 derivative, GLP-1(7-36), can be expressed by substituting oligonucleotide 11.6 for oligonucleotide 11.2 described above. All subsequent cloning operations described above for construction of pXGLP1 are followed, such that the final product is lacking the C-terminal glycine residue characteristic of GLP-1(7-37). Alternatively, this terminal glycine residue may be removed in vivo by the activity of a peptidylglycine alpha-amidating enzyme to produce the insulinotropin GLP-1(7-36) amide.

Plasmid pXGLP1 is co-transfected into primary human skin fibroblasts with plasmid pcDNEO exactly as described for pXEPO1 and pcDNEO in Example 13. Clones are selected in G418 containing medium, transferred to 96-well plates, and assayed for GLP-1(7-37) activity or immunoreactivity in cell supernatants. GLP-1(7-37) activity is determined by incubation of cell supernatants with rat insulinoma RINm5F cells and measuring the ability of the supernatants to induce insulin secretion from these cells using a commercially available insulin radioimmuno-assay (Coat-a-Count Insulin, DPC, Los Angeles, CA). GLP-1(7-37) antigen is determined using a commercially available antisera against GLP-1 (Peninsula Laboratories, Belmont, CA). GLP-1(7-37) positive clones are expanded for implantation into nude mice as described in Example 16 and blood samples are taken to monitor serum human GLP-1(7-37) levels.

In vivo activity is monitored in fasting animals by determining the insulinogenic index after intraperitoneal injection of glucose (1 mg glucose per gram of body weight). Typically, implanted and non-implanted groups of 32 mice are fasted overnight, and 28 are injected with glucose. After injection, the 28 mice are arbitrarily assigned to seven groups of four, and blood sampling (for serum glucose and insulin) is performed on each group at 5, 10, 20, 30, 45, 60, or 90 minutes post-injection, with the non-glucose injected group serving as a fasting control. Increases in the postinjection insulinogenic index (the ration of insulin to glucose in the blood) in animals receiving GLP-1(7-37) expressing cells over non-implanted animals provides in vivo support for the insulinotropic activity of the expressed peptide.

### EXAMPLE 18. PRODUCTION OF TRANSFECTED CELL STRAINS BY GENE TARGETING

Gene targeting occurs when transfecting DNA either integrates into or partially replaces chromosomal DNA sequences through a homologous recombinant event. While such events may occur in the course of any given transfection experiment, they are usually masked by a vast excess of events in which plasmid DNA integrates by non-homologous, or illegitimate, recombination.

### a. GENERATION OF A CONSTRUCT USEFUL FOR SELECTION OF GENE TARGETING EVENTS IN HUMAN CELLS

One approach to selecting the targeted events is by genetic selection for the loss of a gene function due to the integration of transfecting DNA. The human HPRT locus encodes the enzyme hypoxanthine-phosphoribosyl transferase. hprt⁻ cells can be selected for by growth in medium containing the nucleoside analog 6-thioguanine (6-TG): cells with the wild-type (HPRT⁺) allele are killed by 6-TG, while cells with mutant (hprt⁻) alleles can survive. Cells harboring targeted events which disrupt HPRT gene function are therefore selectable in 6-TG medium.

To construct a plasmid for targeting to the HPRT locus, the 6.9 kb HindIII fragment extending from positions 11,960-18,869 in the HPRT sequence (Genebank name HUMHPRTB; Edwards, A. et al., Genomics 6:593-608 (1990)) and including exons 2 and 3 of the HPRT gene, is subcloned into the HindIII site of pUC12. The resulting clone is cleaved at the unique XhoI site in exon 3 of the HPRT gene fragment and the 1.1 kb SalI-XhoI fragment containing the neo gene from pMClNeo (Stratagene) is inserted, disrupting the coding sequence of exon 3. One orientation, with the direction of neo transcription opposite that of HPRT transcription was chosen and designated pE3Neo. The replacement of the normal HPRT exon 3 with the neo-disrupted version will result in an hprt⁻, 6-TG resistant phenotype. Such cells will also be G418 resistant.

### b. GENE TARGETING IN AN ESTABLISHED HUMAN FIBROBLAST CELL LINE

As a demonstration of targeting in immortalized cell lines, and to establish that pE3Neo functions properly in gene targeting, the human fibrosarcoma cell line HT1080 (ATCC CCL 121) was transfected with pE3Neo by electroporation.

HT1080 cells were maintained in HAT (hypoxanthine/aminopterin/xanthine) supplemented DMEM with 15% calf serum (Hyclone) prior to electroporation. Two days before electroporation, the cells are switched to the same medium without aminopterin. Exponentially growing cells were trypsinized and diluted in DMEM/15% calf serum, centrifuged, and resuspended in PBS (phosphate buffered saline) at a final cell volume of 13.3 million cells per ml. pE3Neo is digested with HindIII, separating the 8 kb HPRT-neo fragment from the pUC12 backbone, purified by phenol extraction and ethanol precipitation, and resuspended at a concentration of 600 µg/ml. 50 µl (30 µg) was added to the electroporation cuvette (0.4 cm electrode gap; Bio-Rad Laboratories), along with 750 µl of the cell suspension (10 million cells). Electroporation was at 450 volts, 250 µFarads (Bio-Rad Gene Pulser; Bio-Rad Laboratories). The contents of the cuvette were immediately added to DMEM with 15% calf serum to yield a cell suspension of 1 million cells per 25 ml media. 25 ml of the treated cell suspension was plated onto 150 mm diameter tissue culture dishes and incubated at 37°C, 5% CO₂. 24 hrs later, a G418 solution was added directly to the plates to yield a final concentration of 800 µg/ml G418. Five days later the media was replaced with DMEM/15% calf serum/800 µg/ml G418. Nine days after electroporation, the media was replaced with DMEM/15% calf serum/800 µg/ml G418 and 10 µM 6-thioguanine. Colonies resistant to G418 and 6-TG were picked using cloning cylinders 14-16 days after the dual selection was initiated.

The results of five representative targeting experiments in HT1080 cells are shown in Table 6.

**TABLE 6**

| Transfection | Number of Treated Cells | Number of G418^{r} 6-TG^{r} Clones |
|---|---|---|
| 1 | 1 x 10⁷ | 32 |
| 2 | 1 x 10⁷ | 28 |
| 3 | 1 x 10⁷ | 24 |
| 4 | 1 x 10⁷ | 32 |
| 5 | 1 x 10⁷ | 66 |

For transfection 5, control plates designed to determine the overall yield of G418^{r} colonies indicated that 33,700 G418^{r} colonies could be generated from the initial 1 x 10⁷ treated cells. Thus, the ratio of targeted to non-targeted events is 66/33,700, or 1 to 510. In the five experiments combined, targeted events arise at a frequency of 3.6 x 10⁶, or 0.00036% of treated cells.

Restriction enzyme and Southern hybridization experiments using probes derived from the neo and HPRT genes localized the neo gene to the HPRT locus at the predicted site within HPRT exon 3.

### c. GENE TARGETING IN PRIMARY AND SECONDARY HUMAN SKIN FIBROBLASTS

pE3Neo is digested with HindIII, separating the 8 kb HPRT-neo fragment from the pUC12 backbone, and purified by phenol extraction and ethanol precipitation. DNA was resuspended at 2 mg/ml. Three million secondary human foreskin fibroblasts cells in a volume of 0.5 ml were electroporated at 250 volts and 960 µFarads, with 100 µg of HindIII pE3Neo (50 µl). Three separate transfections were performed, for a total of 9 million treated cells. Cells are processed and selected for G418 resistance as described in Example 6, except that 500,000 cells per 150 mm culture dish are plated for G418 selection. After 10 days under selection, the culture medium is replaced with human fibroblast nutrient medium containing 400 µg/ml G418 and 10 µM 6-TG. Selection with the two drug combination is continued for 10 additional days. Plates are scanned microscopically to localize human fibroblast colonies resistant to both drugs. The fraction of G418^{r} t-TG^{r} colonies is 4 per 9 million treated cells. These colonies constitute 0.0001% (or 1 in a million) of all cells capable of forming colonies. Control plates designed to determine the overall yield of G418^{r} colonies indicated that 2,850 G418^{r} colonies could be generated from the initial 9 x 10⁶ treated cells. Thus, the ratio of targeted to non-targeted events is 4/2,850, or 1 to 712. Restriction enzyme and Southern hybridization experiments using probes derived from the neo and HPRT genes were used to localize the neo gene to the HPRT locus at the predicted site within HPRT exon 3 and demonstrate that targeting had occurred in these four clonal cell strains. Colonies resistant to both drugs have also been isolated by transfecting primary cells (1/3.0 x 10⁷).

The results of several pE3Neo targeting experiments are summarized in Table 7. HindIII digested pE3Neo was either transfected directly or treated with exonuclease III to generate 5' single-stranded overhangs prior to transfection (see Example 18c). DNA preparations with single-stranded regions ranging from 175 to 930 base pairs in length were tested. Using pE3neo digested with HindIII alone, 1/799 G418-resistant colonies were identified by restriction enzyme and Southern hybridization analysis as having a targeted insertion of the neo gene at the HPRT locus (a total of 24 targeted clones were isolated). Targeting was maximally stimulated (approximately 10-fold stimulation) when overhangs of 175 bp were used, with 1/80 G418^{r} colonies displaying restriction fragments that are diagnostic for targeting at HPRT (a total of 9 targeted clones were isolated). Thus, using the conditions and recombinant DNA constructs described here, targeting is readily observed in normal human fibroblasts and the overall targeting frequency (the number of targeted clones divided by the total number of clones stably transfected to G418-resistance) can be stimulated by transfection with targeting constructs containing single-stranded overhanging tails, by the method as described in Example 18e.

**TABLE 7**

| TARGETING TO THE HPRT LOCUS IN HUMAN FIBROBLASTS | | | |
|---|---|---|---|
| pE3neo Treatment | Number of Experiments | Number Targeted Per G41B^{r} Colony | Total Number of Targeted Clone |
| HindIII digest | 6 | 1/799 | 24 |
| 175 bp overhang | 1 | 1/80 | 9 |
| 350 bp overhang | 3 | 1/117 | 20 |
| 930 bp overhang | 1 | 1/144 | 1 |

### d. GENERATION OF A CONSTRUCT FOR TARGETED INSERTION OF A GENE OF THERAPEUTIC INTEREST INTO THE HUMAN GENOME AND ITS USE IN GENE TARGETING

A variant of pE3Neo, in which a gene of therapeutic interest is inserted within the HPRT coding region, adjacent to or near the neo gene, can be used to target a gene of therapeutic interest to a specific position in a recipient primary or secondary cell genome. Such a variant of pE3Neo can be constructed for targeting the hGH gene to the HPRT locus.

pXGH5 is digested with EcoRI and the 4.1 kb fragment containing the hGH gene and linked mouse metallothionein (mMT) promoter is isolated. The EcoRI overhangs are filled in with the Klenow fragment from E. coli DNA polymerase. Separately, pE3Neo is digested with XhoI, which cuts at the junction of the neo fragment and HPRT exon 3 (the 3' junction of the insertion into exon 3). The XhoI overhanging ends of the linearized plasmid are filled in with the Klenow fragment from E. coli DNA polymerase, and the resulting fragment is ligated to the 4.1 kb blunt-ended hGH-mMT fragment. Bacterial colonies derived from the ligation mixture are screened by restriction enzyme analysis for a single copy insertion of the hGH-mMT fragment and one orientation, the hGH gene transcribed in the same direction as the neo gene, is chosen and designated pE3Neo/hGH. pE3Neo/hGH is digested with HindIII, releasing the 12.1 kb fragment containing HPRT, neo and mMT-hGH sequences. Digested DNA is treated and transfected into primary or secondary human fibroblasts as described in Example 18c. G418^{r} TG^{r} colonies are selected and analyzed for targeted insertion of the mMT-hGH and neo sequences into the HPRT gene as described in Example 18c. Individual colonies are assayed for hGH expression using a commercially available immunoassay (Nichols Institute).

Secondary human fibroblasts were transfected with pE3Neo/hGH and thioguanine-resistant colonies were analyzed for stable hGH expression and by restriction enzyme and Southern hybridization analysis. Of thirteen TG^{r} colonies analyzed, eight colonies were identified with an insertion of the hGH gene into the endogenous HPRT locus. All eight strains stably expressed significant quantities of hGH, with an average expression level of 22.7 µg/10⁶ cells/24 hours.

### e. MODIFICATION OF DNA TERMINI TO ENHANCE TARGETING

Several lines of evidence suggest that 3'-overhanging ends are involved in certain homologous recombination pathways of E. coli, bacteriophage, S. cerevisiae and Xenopus laevis. In Xenopus laevis oocytes, molecules with 3'-overhanging ends of several hundred base pairs in length underwent recombination with similarly treated molecules much more rapidly after microinjection than molecules with very short overhangs (4 bp) generated by restriction enzyme digestion. In yeast, the generation of 3'-overhanging ends several hundred base pairs in length appears to be a rate limiting step in meiotic recombination. No evidence for an involvement of 3'-overhanging ends in recombination in human cells has been reported, and in no case have modified DNA substrates of any sort been shown to promote targeting (one form of homologous recombination) in any species. In human cells, the effect of 3'-overhanging ends on targeting is untested. The experiment described in the following example suggests that 5'-overhanging ends are most effective for targeting in primary and secondary human fibroblasts.

There have been no reports on the enhancement of targeting by modifying the ends of the transfecting DNA molecules. This example serves to illustrate that modification of the ends of linear DNA molecules, by conversion of the molecules' termini from a double-stranded form to a single-stranded form, can stimulate targeting into the genome of primary and secondary human fibroblasts.

1100 µg of plasmid pE3Neo (Example 18a) is digested with HindIII. This DNA can be used directly after phenol extraction and ethanol precipitation, or the 8 kb HindIII fragment containing only HPRT ana the neo gene can be separated away from the pUC12 vector sequences by gel electrophoresis. ExoIII digestion of the HindIII digested DNA results in extensive exonucleolytic digestion at each end, initiating at each free 3' end, and leaving 5'-overhanging ends. The extent of exonucleolytic action and, hence, the length of the resulting 5'-overhangings, can be controlled by varying the time of ExoIII digestion. ExoIII digestion of 100 µg of HindIII digested pE3Neo is carried out according to the supplier's recommended conditions, for times of 30 sec, 1 min, 1.5 min, 2 min, 2.5 min, 3 min, 3.5 min, 4 min, 4.5 min, and 5 min. To monitor the extent of digestion an aliquot from each time point, containing 1 µg of ExoIII treated DNA, is treated with mung bean nuclease (Promega), under conditions recommended by the supplier, and the samples fractionated by gel electrophoresis. The difference in size between non-treated, HindIII digested pE3Neo and the same molecules treated with ExoIII and mung bean nuclease is measured. This size difference divided by two gives the average length of the 5'-overhang at each end of the molecule. Using the time points described above and digestion at 30°, the 5'-overhangs produced should range from 100 to 1,000 bases.

60 µg of ExoIII treated DNA (total HindIII digest of pE3NEO) from each time point is purified and electroporated into primary or secondary human fibroblasts under the conditions described in Example 18c. The degree to which targeting is enhanced by each ExoIII treated preparation is quantified by counting the number of G418^{r} 6-TG^{r} colonies and comparing these numbers to targeting with HindIII digested pE3Neo that was not treated with ExoIII.

The effect of 3'-overhanging ends can also be quantified using an analogous system. In this case HindIII digested pE3Neo is treated with bacteriophase T7 gene 6 exonuclease (United States Biochemicals) for varying time intervals under the supplier's recommended conditions. Determination of the extent of digestion (average length of 3'-overhang produced per end) and electroporation conditions are as described for ExoIII treated DNA. The degree to which targeting is enhanced by each T7 gene 6 exonuclease treated preparation is quantified by counting the number of G418^{r} 6-TG^{r} colonies and comparing these numbers to targeting with HindIII digested pE3NEO that was not treated with T7 gene 6 exonuclease.

Other methods for generating 5' and 3' overhanging ends are possible, for example, denaturation and annealing of two linear molecules that partially overlap with each other will generate a mixture of molecules, each molecule having 3'-overhangs at both ends or 5'-overhangs at both ends, as well as reannealed fragments indistinguishable from the starting linear molecules. The length of the overhangs is determined by the length of DNA that is not in common between the two DNA fragments.

### f. CONSTRUCTION OF TARGETING PLASMIDS FOR PLACING THE HUMAN ERYTHROPOIETIN GENE UNDER THE CONTROL OF THE MOUSE METALLOTHIONEIN PROMOTER IN PRIMARY AND SECONDARY HUMAN FIBROBLASTS

The following serves to illustrate one embodiment of the present invention, in which the normal positive and negative regulatory sequences upstream of the human erythropoietin (EPO) gene are altered to allow expression of human erythropoietin in primary or secondary human fibroblast strains, which do not express EPO in significant quantities as obtained.

A region lying exclusively upstream of the human EPO coding region can be amplified by PCR. Three sets of primers useful for this purpose were designed after analysis of the published human EPO [Genbank designation HUMERPA; Lin, F-K., et al., Proc. Natl. Acad. Sci., USA 82:7580-7584 (1985)]. These primer pairs can amplify fragments of 609, 603, or 590 bp.

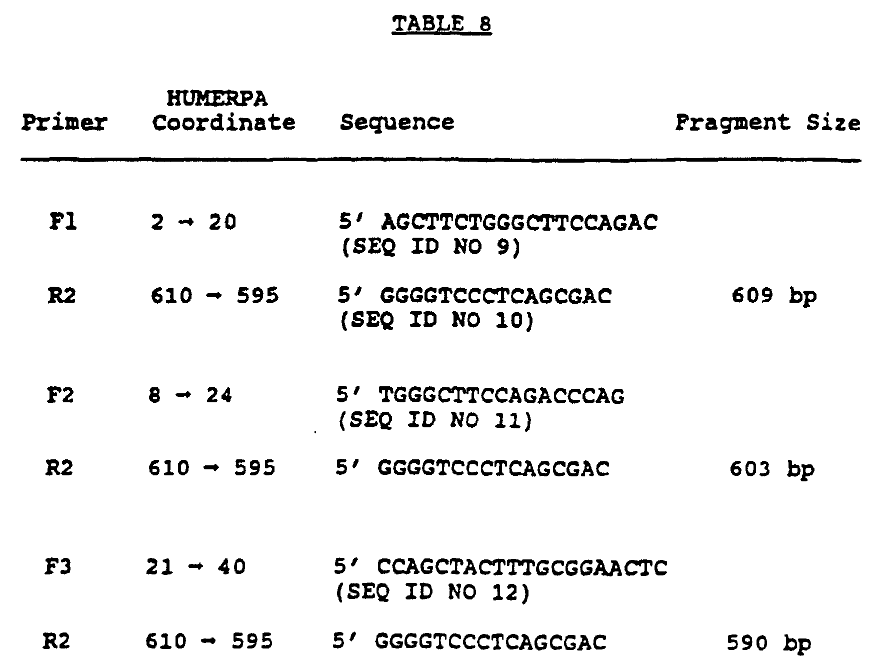

The three fragments overlap substantially and are interchangeable for the present purposes. The 609 bp fragment, extending from -623 to -14 relative to the translation start site (HUMERPA nucleotide positions 2 to 610), is ligated at both ends with ClaI linkers. The resulting ClaI-linked fragment is digested with ClaI and inserted into the ClaI site of pBluescriptIISK/+ (Stratagene), with the orientation such that HUMERPA nucleotide position 610 is adjacent to the Sail site in the plasmid polylinker). This plasmid, p5'EPO, can be cleaved, separately, at the unique FspI or SfiI sites in the EPO upstream fragment (HUMERPA nucleotide positions 150 and 405, respectively) and ligated to the mouse metallotheionein promoter. Typically, the 1.8 kb EcoRI-BglII from the mMT-I gene [containing no mMT coding sequences; Hamer, D.H. and walling M., J. Mol. Appl. Gen. 1:273 288 (1982); this fragment can also be isolated by known methods from mouse genomic DNA using PCR primers 5 designed from analysis of mMT sequences available from Genbank; i.e., MUSMTI, MUSMTIP, MUSMTIPRM] is made blunt-ended by known methods and ligated with SfiI digested (also made blunt-ended) or FspI digested p5'EPO. The orientations of resulting clones are analyzed and those in which the former mMT BglII site is proximal to the SalI site in the plasmid polylinker are used for targeting primary and secondary human fibroblasts. This orientation directs mMT transcription towards HUMERPA nucleotide position 610 in the final construct. The resulting plasmids are designated p5'EPO-mMTF and p5'EPO-mMTS for the mMT insertions in the FspI and SfiI sites, respectively.

Additional upstream sequences are useful in cases where it is desirable to modify, delete and/or replace negative regulatory elements or enhancers that lie upstream of the initial target sequence. In the case of EPO, a negative regulatory element that inhibits EPO expression in extrahepatic and extrarenal tissues [Semenza, G.L. et al., Mol. Cell. Biol. 10:930-938 (1990)] can be deleted. A series of deletions within the 6 kb fragment are prepared. The deleted regions may be replaced with an enhancer with broad host-cell activity [e.g. an enhancer from the Cytomegalovirus (CMV)].

The orientation of the 609 bp 5'EPO fragment in the pBluescriptIISK/+ vector was chosen since the HUMERPA sequences are preceded on their 5' end by a BamHI (distal) and HindIII site (proximal). Thus, a 6 kb BamHI-HindIII fragment normally lying upstream of the 609 bp fragment [Semenza, G. L. et al., Mol. Cell. Biol. 10:930-938 (1990)] can be isolated from genomic DNA by known methods. For example, a bacteriophage, cosmid, or yeast artificial chromosome library could be screened with the 609 bp PCR amplified fragment as a probe. The desired clone will have a 6 kb BamHI-HindIII fragment and its identity can be confirmed by comparing its restriction map from a restriction map around the human EPO gene determined by known methods. Alternatively, constructing a restriction map of the human genome upstream of the EPO gene using the 609 bp fragment as a probe can identify enzymes which generate a fragment originating between HUMERPA coordinates 2 and 609 and extending past the upstream BamHI site; this fragment can be isolated by gel electrophoresis from the appropriate digest of human genomic DNA and ligated into a bacterial or yeast cloning vector. The correct clone will hybridize to the 609 bp 5'EPO probe and contain a 6 kb BamHI-HindIII fragment. The isolated 6 kb fragment is inserted in the proper orientation into p5'EPO, p5'EPO-mMTF, or p5'EPO-mMTS (such that the HindIII site is adjacent to HUMERPA nucleotide position 2). Additional upstream sequences can be isolated by known methods, using chromosome walking techniques or by isolation of yeast artificial chromosomes hybridizing to the 609 bp 5'EPO probe.

The cloning strategies described above allow sequences upstream of EPO to be modified in vitro for subsequent targeted transfection of primary and secondary human fibroblasts. The strategies describe simple insertions of the mMT promoter, as well as deletion of the negative regulatory region, and deletion of the negative regulatory region and replacement with an enhancer with broad host-cell activity.

### g. TARGETING TO SEQUENCES FLANKING THE HUMAN EPO GENE AND ISOLATION OF TARGETED PRIMARY, SECONDARY AND IMMORTALIZED HUMAN FIBROBLASTS BY SCREENING

For targeting, the plasmids are cut with restriction enzymes that free the insert away from the plasmid backbone. In the case of p5'EPO-mMTS, HindIII and Sail digestion releases a targeting fragment of 2.4 kb, comprised of the 1.8 kb mMT promoter flanked on the 5' and 3' sides by 405 bp and 204 base pairs, respectively, of DNA for targeting this construct to the regulatory region of the EPO gene. This DNA or the 2.4 kb targeting fragment alone is purified by phenol extraction and ethanol precipitation and transfected into primary or secondary human fibroblasts under the conditions described in Example 18c. Transfected cells are plated onto 150 mm dishes in human fibroblast nutrient medium. 48 hours later the cells are plated into 24 well dishes at a density of 10,000 cells/cm² [approximately 20,000 cells per well; if targeting occurs at a rate of 1 event per 10⁶ clonable cells (Example 18c, then about 50 wells would need to be assayed to isolate a single expressing colony]. Cells in which the transfecting DNA has targeted to the homologous region upstream of EPO will express EPO under the control of the mMT promoter. After 10 days, whole well supernatants are assayed for EPO expression using a commercially available immunoassay kit (Amgen). Clones from wells displaying EPO synthesis are isolated using known methods, typically by assaying fractions of the heterogenous populations of cells separated into individual wells or plates, assaying fractions of these positive wells, and repeating as needed, ultimately isolating the targeted colony by screening 96-well microtiter plates seeded at one cell per well. DNA from entire plate lysates can also be analyzed by PCR for amplification of a fragment using a mMT specific primer in conjunction with a primer lying upstream of HUMERPA nucleotide position 1. This primer pair should amplify a DNA fragment of a size precisely predicted based on the DNA sequence. Positive plates are trypsinized and replated at successively lower dilutions, and the DNA preparation and PCR steps repeated as needed to isolate targeted cells.

The targeting schemes herein described may also be used to activate hGH expression in immortalized human cells (for example, HT1080 fibroblasts, HeLa, MCF-7 breast cancer cells, K-562 leukemia cells, KB carcinoma cells or 2780AD ovarian carcinoma cells) for the purposes of producing hGH for conventional pharmacologic delivery.

### h. TARGETING TO SEQUENCES FLANKING THE HUMAN EPO GENE AND ISOLATION OF TARGETED PRIMARY, SECONDARY AND IMMORTALIZED HUMAN FIBROBLASTS BY A POSITIVE OR A COMBINED POSITIVE/NEGATIVE SELECTION SYSTEM

The strategy for constructing p5'EPO-mMTF, p5'EPO-mMTS, and derivatives of such with the additional upstream 6 kb BamHI-HindIII fragment can be followed with the additional step of inserting the neo gene adjacent to the mMT promoter. In addition, a negative selection marker, for example, gpt [from pMSG (Pharmacia) or another suitable source], can be inserted adjacent to the HUMERPA sequences in the pBluescriptIISK/+ polylinker. In the former case, G418^{r} colonies are isolated and screened by PCR amplification or restriction enzyme and Southern hybridization analysis of DNA prepared from pools of colonies to identify targeted colonies. In the latter case, G418^{r} colonies are placed in medium containing 6-thioxanthine to select against the integration of the gpt gene [Besnard, C. et al., Mol. Cell. Biol. 7:4139-4141 (1987)]. In addition, the HSV-TK gene can be placed on the opposite side of the insert as gpt, allowing selection for neo and against both gpt and TK by growing cells in human fibroblast nutrient medium containing 400 µg/ml G418, 100 µM 6-thioxanthine, and 25 µg/ml gancyclovir. The double negative selection should provide a nearly absolute selection for true targeted events and Southern blot analysis provides an ultimate confirmation.

The targeting schemes herein described may also be used to activate hEPO expression in immortalized human cells (for example, HT1080 fibroblasts, HeLa, MCF-7 breast cancer cells, K-562 leukemia cells, KB carcinoma cells or 2780AD ovarian carcinoma cells) for the purposes of producing hEPO for conventional pharmacologic delivery.

### i. CONSTRUCTION OF TARGETING PLASMIDS FOR PLACING THE HUMAN GROWTH HORMONE GENE UNDER THE CONTROL OF THE MOUSE METALLOTHIONEIN PROMOTER IN PRIMARY HUMAN FIBROBLASTS

The following example serves to illustrate an embodiment in which the normal regulatory sequences upstream of the human growth hormone gene are altered to allow expression of human growth hormone in primary or secondary human fibroblast strains.

Targeting molecules similar to those described in Example 18f for targeting to the EPO gene regulatory region are generated using cloned DNA fragments derived from the 5' end of the human growth hormone N gene. An approximately 1.8 kb fragment spanning HUMGHCSA (Genbank Entry) nucleotide positions 3787-5432 (the positions of two EcoNI sites which generate a convenient sized fragment for cloning or for diagnostic digestion of subclones involving this fragment) is amplified by PCR primers designed by analysis of the HUMGHCSA sequence in this region. This region extends from the middle of hGH gene N intron 1 to an upstream position approximately 1.4 kb 5' to the translational start site. pUC12 is digested with EcoRI and BamHI, treated with Klenow to generate blunt ends, and recircularized under dilute conditions, resulting in plasmids which have lost the EcoRI and BamHI sites. This plasmid is designated pUC12XEB. HindIII linkers are ligated onto the amplified hGH fragment and the resulting fragment is digested with HindIII and ligated to HindIII digested pUC12XEB. The resulting plasmid, pUC12XEB-5'hGH, is digested with EcoRI and BamHI, to remove a 0.5 kb fragment lying immediately upstream of the hGH transcriptional initiation site. The digested DNA is ligated to the 1.8 kb EcoRI-BglII from the mMT-I gene [containing no mMT coding sequences; Hamer, D.H. and Walling, M., J. Mol. Appl. Gen. 1:273-288 (1982); the fragment can also be isolated by known methods from mouse genomic DNA using PCR primers designed from analysis of mMT sequences available from Genbank; i.e., MUSMTI, MUSMTIP, MUSMTIPRM]. This plasmid p5'hGH-mMT has the mMT promoter flanked on both sides by upstream hGH sequences.

The cloning strategies described above allow sequences upstream of hGH to be modified in vitro for subsequent targeted transfection of primary and secondary human fibroblasts. The strategy described a simple insertion of the mMT promoter. Other strategies can be envisioned, for example, in which an enhancer with broad host-cell specificity is inserted upstream of the inserted mMT sequence.

### j. TARGETING TO SEQUENCES FLANKING THE HUMAN hGH GENE AND ISOLATION OF TARGETED PRIMARY, SECONDARY AND IMMORTALIZED HUMAN FIBROBLASTS BY SCREENING

For targeting, the plasmids are cut with restriction enzymes that free the insert away from the plasmid backbone. In the case of p5'hGH-mMT, HindIII digestion releases a targeting fragment of 2.9 kb, comprised of the 1.8 kb mMT promoter flanked on the 5' end 3' sides by DNA for targeting this construct to the regulatory region of the hGH gene. This DNA or the 2.9 kb targeting fragment alone is purified by phenol extraction and ethanol precipitation and transfected into primary or secondary human fibroblasts under the conditions described in Example 6. Transfected cells are plated onto 150 mm dishes in human fibroblast nutrient medium. 48 hours later the cells are plated into 24 well dishes at a density of 10,000 cells/cm² [approximately 20,000 cells per well; if targeting occurs at a rate of 1 event per 10⁶ clonable cells (Example 18c), then about 50 wells would need to be assayed to isolate a single expressing colony]. Cells in which the transfecting DNA has targeted to the homologous region upstream of hGH will express hGH under the control of the mMT promoter. After 10 days, whole well supernatants are assayed for hGH expression using a commercially available immunoassay kit (Nichols). Clones from wells displaying hGH synthesis are isolated using known methods, typically by assaying fractions of the heterogenous populations of cells separated into individual wells or plates, assaying fractions of these positive wells, and repeating as needed, ultimately isolated the targeted colony by screening 96-well microtiter plates seeded at one cell per well. DNA from entire plate lysates can also be analyzed by PCR for amplification of a fragment using a mMT specific primer in conjunction with a primer lying downstream of HUMGHCSA nucleotide position 5,432. This primer pair should amplify a DNA fragment of a size precisely predicted based on the DNA sequence. Positive plates are trypsinized and replated at successively lower dilutions, and the DNA preparation and PCR steps repeated as needed to isolate targeted cells.

The targeting schemes herein described may also be used to activate hHGH expression in immortalized human cells (for example, HT1080 fibroblasts, HeLa, MCF-7 breast cancer cells, K-562 leukemia cells, KB carcinoma cells or 2780AD ovarian carcinoma cells) for the purposes of producing hGH for conventional pharmacologic delivery.

### k. TARGETING TO SEQUENCES FLANKING THE HUMAN hGH GENE AND ISOLATION OF TARGETED PRIMARY, SECONDARY AND IMMORTALIZED HUMAN FIBROBLASTS BY A POSITIVE OR A COMBINED POSITIVE/NEGATIVE SELECTION SYSTEM

The strategy for constructing p5'hGH-mMT can be followed with the additional step of inserting the neo gene adjacent to the mMT promoter. In addition, a negative selection marker, for example, gpt [from pMSG (Pharmacia) or another suitable source], can be inserted adjacent to the HUMGHCSA sequences in the pUC12 polylinker. In the former case, G418^{r} colonies are isolated and screened by PCR amplification or restriction enzyme and Southern hybridization analysis of DNA prepared from pools of colonies to identify targeted colonies. In the latter case, G418^{r} colonies are placed in medium containing thioxanthine to select against the integration of the gpt gene (Besnard, C. et al., Mol. Cell. Biol. 7: 4139-4141 (1987)]. In addition, the HSV-TK gene can be placed on the opposite side of the insert as gpt, allowing selection for neo and against both gpt and TK by growing cells in human fibroblast nutrient medium containing 400 µg/ml G418, 100 µM 6-thioxanthine, and 25 µg/ml gancyclovir. The double negative selection should provide a nearly absolute selection for true targeted events. Southern hybridization analysis is confirmatory.

The targeting schemes herein described may also be used to activate hGH expression in immortalized human cells (for example, HT1080 fibroblasts, HeLa, MCF-7 breast cancer cells, K-562 leukemia cells, KB carcinoma cells or 2780AD ovarian carcinoma cells) for the purposes of producing hGH for conventional pharmacologic delivery.

### EXAMPLE 19. CONSTRUCTION OF TARGETING PLASMIDS WHICH RESULT IN CHIMERIC TRANSCRIPTION UNITS IN WHICH HUMAN GROWTH HORMONE AND ERYTHROPOIETIN SEQUENCES ARE FUSED

The following serves to illustrate two further embodiments in which the normal regulatory sequences upstream of the human EPO gene are altered to allow expression of hEPO in primary or secondary fibroblast strains which do not express EPO in detectable quantities in their untransfected state as obtained. In these embodiments, the products of the targeting events are chimeric transcription units in which the first exon of the human growth hormone gene is positioned upstream of EPO exons 2-5. The product of transcription, splicing and translation is a protein in which amino acids 1-4 of the hEPO signal peptide are replaced with amino acid residues 1-3 of hGH. The two embodiments differ with respect to both the relative positions of the foreign regulatory sequences that are inserted and the specific pattern of splicing that needs to occur to produce the final, processed transcript.

Plasmid pXEPO-10 is designed to replace exon 1 of hEPO with exon 1 of hGH by gene targeting to the endogenous hEPO gene on human chromosome 7. Plasmid pXEPO-10 is constructed as follows. First, the intermediate plasmid pT163 is constructed by inserting the 6 kb HindIII-BamHI fragment (see Example 18f) lying upstream of the hEPO coding region into HindIII-BamHI digested pBluescriptII SK+ (Stratagene, LaJolla, CA). The product of this ligation is digested with XhoI and HindIII and ligated to the 1.1 kb HindIII-XhoI fragment from pMClneoPolyA [Thomas, K. R. and Capecchi, M. R. Cell 51: 503-512 (1987) available from Strategene, LaJolla, CA] to create pT163. Oligonucleotides 13.1 - 13.4 are utilized in polymerase chain reactions to generate a fusion fragment in which the mouse metallothionein 1 (mMT-I) promoter - hGH exon 1 sequences are additionally fused to hEPO intron 1 sequences. First, oligonucleotides 13.1 and 13.2 are used to amplify the approximately 0.73 kb mMT-I promoter - hGH exon 1 fragment from pXGH5 (Figure 1). Next, oligonucleotides 13.3 and 13.4 are used to amplify the approximately 0.57 kb fragment comprised predominantly of hEPO intron 1 from human genomic DNA. Finally, the two amplified fragments are mixed and further amplified with oligonucleotides 13.1 and 13.4 to generate the final fusion fragment (fusion fragment 3) flanked by a SalI site at the 5' side of the mMT-I moiety and an XhoI site at the 3' side of the hEPO intron 1 sequence. Fusion fragment 3 is digested with XhoI and Sail and ligated to XhoI digested pT163. The ligation mixture is transformed into *E. coli* and a clone containing a single insert of fusion fragment 3 in which the XhoI site is regenerated at the 3' side of hEPO intron 1 sequences is identified and designated pXEPO-10. 
13.1 5' AAAAGTCGAC GGTACCTTGG TTTTTAAAAC CAGCCTGGAG
SalI kpnI
(SEQ ID NO 13)
 
13.2 5′ CCTAGCGGCA ATGGCTACAG GTGAGTACTC GCGGGCTGGG CG
(SEQ ID NO 14)
 
13.3 5′ CGCCCAGCCC GCGAGTACTC ACCTGTAGCC ATTGCCGCTA GG
(SEQ ID NO 15)
 
13.4 5′ TTTTCTCGAG CTAGAACAGA TAGCCAGGCT GAGAG
XhoI
(SEQ ID NO 16)
 The non-boldface region of oligo 13.1 is identical to the mMT-I promoter, with the natural KpnI site as its 5' boundary. The boldface type denotes a Sail site tail to convert the 5' boundary to a Sall site. The boldface region of oligos 13.2 and 13.3 denote hGH sequences, while the non-boldface regions are intron 1 sequences from the hEPO gene. The non-boldface region of oligo 13.4 is identical to last 25 bases of hEPO intron 1. The boldface region includes an XhoI site tail to convert the 3' boundary of the amplified fragment to an XhoI site.

Plasmid pXEPO-11 is designed to place, by gene targeting, the mMT-I promoter and exon 1 of hGH upstream of the hEPO structural gene and promoter region at the endogenous hEPO locus on human chromosome 7. Plasmid pXEPO-11 is constructed as follows. Oligonucleotides 13.1 and 13.5 - 13.7 are utilized in polymerase chain reactions to generate a fusion fragment in which the mouse metallothionein I (mMT-I) promoter - hGH exon 1 sequences are additionally fused to hEPO sequences from -1 to -630 relative to the hEPO coding region. First, oligonucleotides 13.1 and 13.5 are used to amplify the approximately 0.73 kb mMT-I promoter - hGH exon 1 fragment from pXGH5 (Figure 1). Next, oligonucleotides 13.6 and 13.7 are used to amplify, from human genomic DNA, the approximately 0.62 kb fragment comprised predominantly of hEPO sequences from -1 to -620 relative to the hEPO coding region. Both oligos 13.5 and 13.6 contain a 10 bp linker sequence located at the hGH intron 1 - hEPO promoter region, which corresponds to the natural hEPO intron 1 splice donor site. Finally, the two amplified fragments are mixed and further amplified with oligonucleotides 13.1 and 13.7 to generate the final fusion fragment (fusion fragment 6) flanked by a SalI site at the 5' side of the mMT-I moiety and an XhoI site at the 3' side of the hEPO promoter region. Fusion fragment 6 is digested with XhoI and SalI and ligated to XhoI digested pT163. The ligation mixture is transformed into *E. coli* and a clone containing a single insert of fusion fragment 6 in which the XhoI site is regenerated at the 3' side of hEPO promoter sequences is identified and designated pXEPO-11. 
13.5 5′ CCTAGCGGCA ATGGCTACAG GTGAGTACTC AAGCTTCTGG
HindIII
GCTTCCAGAC CCAG (SEQ ID NO 17)
 
13.6 5′ CTGGGTCTGG AAGCCCAGAA GCTTGAGTAC TCACCTGTAG
HindIII
CCATTGCCGC TAGG (SEQ ID NO 18)
 
13.7 5′ TTTTCTCGAG CTCCGCGCCT GGCCGGGGTC CCTC
XhoI
(SEQ ID NO 19)
 The boldface regions of oligos 13.5 and 13.6 denote hGH sequences. The italicized regions correspond to the first 10 base pairs of hEPO intron 1. The remainder of the oligos correspond to hEPO sequences from -620 to -597 relative to the hEPO coding region. The non-boldface region of oligo 13.7 is identical to bases -1 to -24 relative to the hEPO coding region. The boldface region includes an XhoI site tail to convert the 3' boundary of the amplified fragment to an XhoI site.

Plasmid pXEPO-10 can be used for gene targeting by digestion with BamHI and XhoI to release the 7.3 kb fragment containing the mMT-I/hGH fusion flanked on both sides by hEPO sequences. This fragment (targeting fragment 1) contains no hEPO coding sequences, having only sequences lying between -620 and approximately -6620 upstream of the hEPO coding region and hEPO intron 1 sequences to direct targeting to the human EPO locus. Targeting fragment 1 is transfected into primary or secondary human skin fibroblasts using conditions similar to those described in Example 18c. G418-resistant colonies are picked into individual wells of 96-well plates and screened for EPO expression by an ELISA assay (R&D Systems, Minneapolis MN). Cells in which the transfecting DNA integrates randomly into the human genome cannot produce EPO. Cells in which the transfecting DNA has undergone homologous recombination with the endogenous hEPO intron 1 and hEPO upstream sequences contain a chimeric gene in which the mMT-I promoter and non-transcribed sequences and the hGH 5' untranslated sequences and hGH exon 1 replace the normal hEPO promoter and hEPO exon 1 (see Figure 7). Non-hEPO sequences in targeting fragment 1 are joined to hEPO sequences downstream of hEPO intron 1. The replacement of the normal hEPO regulatory region with the mMT-I promoter will activate the EPO gene in fibroblasts, which do not normally express EPO. The replacement of hEPO exon 1 with hGH exon 1 results in a protein in which the first 4 amino acids of the hEPO signal peptide are replaced with amino acids 1-3 of hGH, creating a functional, chimeric signal peptide which is removed by post-translation processing from the mature protein and is secreted from the expressing cells.

Plasmid pXEPO-11 can be used for gene targeting by digestion with BamHI and XhoI to release the 7.4 kb fragment containing the mMT-I/hGH fusion flanked on both sides by hEPO sequences. This fragment (targeting fragment 2) contains no hEPO coding sequences, having only sequences lying between -1 and approximately -6620 upstream of the hEPO coding region to direct targeting to the human EPO locus. Targeting fragment 2 is transfected into primary or secondary human skin fibroblasts using conditions similar to those described in Example 18g. G418-resistant colonies are picked into individual wells of 96-well plates and screened for EPO expression by an ELISA assay (R&D Systems, Minneapolis, MN). Cells in which the transfecting DNA integrates randomly into the human genome cannot produce EPO. Cells in which the transfecting DNA has undergone homologous recombination with the endogenous hEPO promoter and upstream sequences contain a chimeric gene in which the mMT-I promoter and non-transcribed sequences, hGH 5' untranslated sequences and hGh exon 1, and a 10 base pair linker comprised of the first 10 bases of hEPO intron 1 are inserted at the HindIII site lying at position -620 relative to the hEPO coding region (see Figure 8). The localization of the mMT-I promoter upstream of the normally silent hEPO promoter will direct the synthesis, in primary or secondary skin fibroblasts, of a message reading (5' to 3') non-translated metallothionein and hGH sequences, hGH exon 1, 10 bases of DNA identical to the first 10 base pairs of hEPO intron 1, and the normal hEPO promoter and hEPO exon 1 (-620 to +13 relative to the EPO coding sequence). The 10 base pair linker sequence from hEPO intron 1 acts as a splice donor site to fuse hGH exon 1 to the next downstream splice acceptor site, that lying immediately upstream of hEPO exon 2. Processing of the resulting transcript will therefore splice out the hEPO promoter, exon 1, and intron 1 sequences. The replacement of hEPO exon 1 with hGH exon 1 results in a protein in which the first 4 amino acids of the hEPO signal peptide are replaced with amino acids 1-3 of hGH, creating a functional, chimeric signal peptide which is removed by post-translation processing from the mature protein and is secreted from the expressing cells.

A series of constructs related to pXEPO-10 and pXEPO-11 may be constructed, using known methods. In these constructs, the relative positions of the mMT-I promoter and hGH sequences, as well as the position at which the mMT-I/hGH sequences are inserted into hEPO upstream sequences, are varied to create alternative chimeric transcription units that facilitate gene targeting, result in more efficient expression of the fusion transcripts, or have other desirable properties. Such constructs will give similar results, such that an hGH-hEPO fusion gene is placed under the control of an exogenous promoter by gene targeting to the normal hEPO locus. For example, the 6 kb HindIII-BamHI fragment upstream of the hEPO gene (See Example 18f) has numerous restriction enzyme recognition sequences that can be utilized as sites for insertion of the *neo* gene and the mMT-I promoter/hGH fusion fragment. One such site, a BglII site lying approximately 1.3 kb upstream of the HindIII site, is unique in this region and can be used for insertion of one or more selectable markers and a regulatory region derived from another gene that will serve to activate EPO expression in primary, secondary, or immortalized human cells.

First, the intermediate plasmid pT164 is constructed by inserting the 6 kb HindIII-BamHI fragment (Example 18f) lying upstream of the hEPO coding region into HindIII-BamHI digested pBluescriptII SK+ (Stratagene, LaJolla, CA). Plasmid pMClneoPolyA [Thomas, K.R. and Capecchi, M.R. Cell 51:503-512 (1987); available from Stratagene, LaJolla, CA] is digested with BamHI and XhoI, made blunt-ended by treatment with the Klenow fragment of *E. coli* DNA polymerase, and the resulting 1.1 kb fragment is purified. pT164 is digested with BglII and made blunt-ended by treatment with the Klenow fragment of *E. coli* DNA polymerase. The two preceding blunt-ended fragments are ligated together and transformed into competent *E. coli.* Clones with a single insert of the 1.1 kb neo fragment are isolated and analyzed by restriction enzyme analysis to identify those in which the BglII site recreated by the fusion of the blunt XhoI and BglII sites is localized 1.3 kb away from the unique HindIII site present in plasmid pT164. The resulting plasmid, pT165, can now be cleaved at the unique BglII site flanking the 5' side of the neo transcription unit.

Oligonucleotides 13.8 and 13.9 are utilized in polymerase chain reactions to generate a fragment in which the mouse metallothionein I (mMT-I) promoter - hGH exon 1 sequences are additionally fused to a 10 base pair fragment comprising a splice donor site. The splice donor site chosen corresponds to the natural hEPO intron 1 splice donor site, although a larger number of splice donor sites or consensus splice donor sites may be used. The oligonucleotides (13.8 and 13.9) are used to amplify the approximately 0.73 kb mMT-I promoter - hGH exon 1 fragment from pXGH5 (Figure 1). The amplified fragment (fragment 7) is digested with BglII and ligated to BglII digested pT165. The ligation mixture is transformed into *E. coli* and a clone, containing a single insert of fragment 7 in which the KpnI site in the mMT-I promoter is adjacent to the 5' end of the neo gene and the mMT-I promoter is oriented such that transcription is directed towards the unique HindIII site, is identified and designated pXEPO-12. 
13.8 5' AAAAAGATCT GGTACCTTGG TTTTTAAAAC CAGCCTGGAG
BglII KpnI
(SEQ ID NO 20)
 The non-boldface region of oligo 13.8 is identical to the mMT-I promoter, with the natural KpnI site as its 5' boundary. The boldface type denotes a BglII site tail to convert the 5' boundary to a BglII site. 
13.9 5′ TTTTAGATCT GAGTACTCAC CTGTAGCCAT TGCCGCTAGG
BglII
(SEQ ID NO 21)
 The boldface region of oligos 13.9 denote hGH sequences. The italicized region corresponds to the first 10 base pairs of hEPO intron 1. The underlined BglII site is added for plasmid construction purposes.

Plasmid pXEPO-12 can be used for gene targeting by digestion with BamHI and HindIII to release the 7.9 kb fragment containing the neo gene and the mMT-I/hGH fusion flanked on both sided by hEPO sequences. This fragment (targeting fragment 3) contains no hEPO coding sequences, having only sequences lying between approximately -620 and approximately -6620 upstream of the hEPO coding region to direct targeting upstream of the human EPO locus. Targeting fragment 3 is transfected into primary, secondary, or immortalized human skin fibroblasts using conditions similar to those described in Examples 18b and 18c. G418-resistant colonies are picked into individual wells of 96-well plates and screened for EPO expression by an ELISA assay (R&D Systems, Minneapolis MN). Cells in which the transfecting DNA integrates randomly into the human genome cannot produce EPO. Cells in which the transfecting DNA has undergone homologous recombination with the endogenous hEPO promoter and upstream sequences contain a chimeric gene in which the mMT-I promoter and non-transcribed sequences, hGH 5' untranslated sequences, and hGH exon 1, and a 10 base pair linker comprised of the first 10 bases of hEPO intron 1 are inserted at the BglII site lying at position approximately -1920 relative to the hEPO coding region. The localization of the mMT-I promoter upstream of the normally silent hEPO promoter will direct the synthesis, in primary, secondary, or immortalized human fibroblasts (or other human cells), of a message reading: (5' to 3') nontranslated metallothionein and hGH sequences, hGH exon 1, 10 bases of DNA identical to the first 10 base pairs of hEPO intron 1, and hEPO upstream region and hEPO exon 1 (from approximately -1920 to +13 relative to the EPO coding sequence). The 10 base pair linker sequence from hEPO intron 1 acts as a splice donor site to fuse hGH exon 1 to a downstream splice acceptor site, that lying immediately upstream of hEPO exon 2. Processing of the resulting transcript will therefore splice out the hEPO upstream sequences, promoter region, exon 1, and intron 1 sequences. When using pXEPO-10, -11 and -12, post-transcriptional processing of the message may be improved by using in vitro mutagenesis to eliminate splice acceptor sites lying in hEPO upstream sequences between the mMT-I promoter and hEPO exon 1, which reduce level of productive splicing events needed create the desired message. The replacement of hEPO exon 1 with hGH exon 1 results in a protein in which the first 4 amino acids of the hEPO signal peptide are replaced with amino acids 1-3 of hGH, creating a functional, chimeric signal peptide which is removed by post-translation processing from the mature protein and is secreted from the expressing cells.

### EXAMPLE 20. STABLE TRANSFECTION OF NORMAL HUMAN MAMMARY EPITHELIAL CELLS WITH PLASMIDS CONTAINING THE hGH AND/OR neo GENES

Human mammary epithelial cells (HMEC) are obtained by digestion of ductal fragments dissociated from breast tissue. Breast tissue is trimmed of fat and minced into 2 - 4 mm³ pieces. Minced tissue is washed with Hanks Basic Salt Solution (HBSS) and then placed in enzyme solution in trypsinizing flasks (Bellco, Vineland, NJ) at approximately 50 ml enzyme solution per 10 g tissue. Enzyme solution consists of Collagenase A (Boehringer Mannheim, Indianapolis, IN) at 1 mg/ml and hyaluronidase (Sigma, St. Louis, MO) at 100 units/ml dissolved in Mammary Epithelial Basal Media (MEBM, Clonetics, San Diego, CA) supplemented with 5% calf serum, 1% penicillin-streptomycin (Gibco, Grand Island, NY), and 10 µg/ml bovine insulin (Sigma). Digestion is carried out at 37°C and 5% CO₂ on an orbital shaker (180 rpm) overnight. Digested tissue is further dissociated by repeated passage through a wide bore pipette. Ductal fragments are washed in 3 changes of HBSS (10 - 40 ml) by spinning at 1000 rpm for 10 minutes in 15 or 50 ml conical centrifuge tubes. The pellet is either resuspended in freezing media (MEBM + 20% calf serum + 10% DMSO) and frozen under liquid nitrogen or further digested to obtain single cells. To obtain single cells, trypsin (0.05% trypsin-EDTA, Gibco) is added to the ductal pellet (10 ml trypsin per 0.5 ml pellet) and incubated on an orbital shaker at 37°C for 30 minutes (200 rpm). Trypsinization is stopped with the addition of 2 ml calf serum and cells are spun down at 1200 rpm for 10 minutes. The cell pellet is resuspended in growth media, counted and seeded into uncoated culture vessels at 10,000 cells/cm². HMEC are routinely cultured in serum-free growth media, MEGM, composed of MEBM supplemented with bovine insulin (10 µg/ml, Sigma), epidermal growth factor (10 ng/ml, Collaborative Research, Waltham, MA), bovine transferrin (5 µg/ml, Sigma), hydrocortisone (0.14 µM, Sigma), and bovine pituitary extract (35 µg/ml, Sigma).

Subconfluent HMEC at early passage are trypsinized, resuspended in HBSS + 15% calf serum, and counted. Cells are then pelleted, resuspended in 10 ml of electroporation buffer (EPB) and pelleted once again. Cells are resuspended at 3 x 10⁶ cells/ml EPB + bovine serum albumin (BSA, Sigma). Supercoiled plasmid DNA is added to a sterile cuvette with a 0.4 cm electrode gap (Bio Rad, Melville, NY). 0.5 ml of the cell suspension is added to the cuvette and mixed with the DNA using a sterile transfer pipette (Costar, Cambridge, MA). For electroporation, a Gene Pulser apparatus (Bio Rad, Melville, NY) is set at a capacitance of 960 µF and 150 - 400 V. Following electroporation, cells are removed with a transfer pipette and added to 15 ml polypropylene conical centrifuge tubes containing MEGM. The cell suspension is added to tissue culture dishes at 1.5 x 10⁶ cells per 100 mm dish for selection. Cells are re-fed after 48 hours with MEGM containing 50 µg/ml G418 (Geneticin, Gibco). Selection dishes are incubated for 14 - 28 days until distinct colonies of 2 mm² or larger are observed. During the selection period cultures are re-fed twice weekly with MEGM + 50 µg/ml G418 after one week without refeeding. Colonies are isolated with cloning cylinders and transferred into 96 or 24 well plates.

HMEC were transfected with pXGH301 (120 µg), pcDNEO (5 - 60 µg), or cotransfected with pXGH5 and pcDNEO (100 µg each) at voltages ranging from 150 - 400 V. Average hGH expression for 23 hGH expressing clones was 2.2 µg hGH/10⁶ cells/24 hours. Table 9 summarizes the number of population doublings achieved for a sample of transfected and non-transfected clones. As the data indicate, stably transfected human mammary epithelial cell clones can be propagated for more than 20 population doublings and may be used further as cellular implants for in vivo protein delivery and gene therapy.

**TABLE 9**

| POPULATION DOUBLING CAPACITY OF HUMAN MAMMARY EPITHELIAL CELL CLONES | | |
|---|---|---|
| Condition | Number of clones Achieving: | |
| | 10 - 20 mpd | > 20 mpd |
| Non-transfected | 12 | 10 |
| pcDNEO | 2 | 13 |
| pXGH301 | 1 | 5 |
| pcDNEO + pXGH5 | 12 | 15 |

### EXAMPLE 21. TARGETING AND ACTIVATION OF THE HUMAN EPO LOCUS IN AN IMMORTALIZED HUMAN FIBROBLAST LINE

The targeting construct pXEPO-13 was made to test the hypothesis that the endogenous hEPO gene could be activated in a human fibroblast cell. First, plasmid pT22.1 was constructed, containing 63 bp of genomic hEPO sequence upstream of the first codon of the hEPO gene fused to the mouse metallothionein-1 promoter (mMT-I). Oligonucleotides 22.1 to 22.4 were used in PCR to fuse mMT-I and hEPO sequences. The properties of these primers are as follows: 22.1 is a 21 base oligonucleotide homologous to a segment of the mMT-I promoter beginning 28 bp upstream of the mMT-I KpnI site; 22.2 and 22.3 are 58 nucleotide complementary primers which define the fusion of hEPO and mMT-I sequences such that the fusion contains 28 bp of hEPO sequence beginning 35 bases upstream of the first codon of the hEPO gene, and mMT-I sequences beginning at base 29 of oligonucleotide 22.2, comprising the natural BglII site of mMT-I and extending 30 bases into mMT-I sequence; 22.4 is 21 nucleotides in length and is homologous to hEPO sequence beginning 725 bp downstream of the first codon of the hEPO gene. These primers were used to amplify a 1.4 kb DNA fragment comprising a fusion of mMT-I and hEPO sequences as described above. The resulting fragment was digested with KpnI (the PCR fragment contained two KpnI sites: a single natural KpnI site in the mMT-I promoter region and a single natural KpnI site in the hEPO sequence), and purified. The plasmid pXEPO1 (Figure 5) was also digested with KpnI, releasing a 1.4 kb fragment and a 6.4 kb fragment. The 6.4 kb fragment was purified and ligated to the 1.4 kb KpnI PCR fusion fragment. The resulting construct was called pT22.1. A second intermediate, pT22.2, was constructed by ligating the approximately 6 kb HindIII-BamHI fragment lying upstream of the hEPO structural gene (see Example 18f) to BamHI and HindIII digested pBSIISK+ (Stratagene, LaJolla, CA). A third intermediate, pT22.3, was constructed by first excising a 1.1 kb XhoI/BamHI fragment from pMCINEOpolyA (Stratagene,, LaJolla, CA) containing the neomycin phosphotransferase gene. The fragment was then made blunt-ended with the Klenow fragment of DNA polymerase I (New England Biolabs). This fragment was then ligated to the HincII site of pBSIISK+ (similarly made blunt with DNA polymerase I) to produce pT22.3. A fourth intermediate, pT22.4, was made by purifying a 1.1 kb XhoI/HindIII fragment comprising the neo gene from pT22.3 and ligating this fragment to XhoI and HindIII digested pT22.2. pT22.4 thus contains the neo gene adjacent to the HindIII side of the BamHI-HindIII upstream hEPO fragment. Finally, pXEPO-13 was generated by first excising a 2.8 kb EcoRI/AccI fragment from pT22.1. The EcoRI site of this fragment defines the 5' boundary of the mMT-I promoter, while the AccI site of this fragment lies within hEPO exon 5. Thus, the AccI/EcoRI fragment contains a nearly complete hEPO expression unit; missing only a part of exon 5 and the natural polyadenylation site. This 2.8 kb EcoRI/AccI fragment was purified, made blunt-ended by treatment with the Klenow fragment of DNA polymerase I, and ligated to XhoI digested, blunt-ended, pT22.4.

HT1080 cells were transfected with PvuI-BamHI digested pXEPO-13. pXEPO-13 digested in this way generates three fragments; a 1 kb vector fragment including a portion of the *amp* gene, a 1.7 kb fragment of remaining vector sequences and an 8.9 kb fragment containing hEPO, neo and mMT-I sequences. The 8.9 kb BamHI/PvuI fragment contained the following sequences in order from the BamHI site: 6.0 kb of upstream hEPO genomic sequence, the 1.1 kb *neo* transcription unit, the 0.7 kb mMT-I promoter and the 2.8 kb hEPO coding sequence truncated within exon 5. 45µg of pEXPO-13 digested in this way was used in an electroporation of 12 million cells (electroporation conditions were described in Example 18b). This electroporation was repeated a total of eight times, resulting in electroporation of a total of 96 million cells. Cells were mixed with media to provide a cell density of 1 million cells per ml and 1 ml aliquots were dispensed into a total of 96, 150mm tissue culture plates (Falcon) each containing a minimum of 35 ml of DMEM/15% calf serum. The following day, the media was aspirated and replaced with fresh medium containing 0.8 mg/ml G418 (Gibco). After 10 days of incubation, the media of each plate was sampled for hEPO by ELISA analysis (R & D Systems). Six of the 96 plates contained at least 10 mU/ml hEPO. One of these plates, number 18, was selected for purification of hEPO expressing colonies. each of the 96, 150 mm plates contained approximately 600 G418 resistant colonies (an estimated total of 57,600 G418 resistant colonies on all 96 plates). The approximately 600 colonies on plate number 18 were trypsinized and replated at 50 cells/ml into 364 well plates (Sterilin). After one week of incubation, single colonies were visible at approximately 10 colonies per large well of the 364 well plates (Sterilin). After one week of incubation, single colonies were visible at approximately 10 colonies per large well of the 364 well plate (these plates are comprised of 16 small wells within each of the 24 large wells). Each well was screened for hEPO expression at this time. Two of the large wells contained media with at least 20 mU/ml hEPO. Well number A2 was found to contain 15 colonies distributed among the 16 small wells. The contents of each of these small wells were trypsinized and transferred to 16 individual wells of a 96 well plate. following 7 days of incubation the media from each of these wells was sampled for hEPO ELISA analysis. Only a single well, well number 10, contained hEPO. This cell strain was designated HT165-18A2-10 and was expanded in culture for quantitative hEPO analysis, RNA isolation and DNA isolation. Quantitative measurement of hEPO production resulted in a value of 2,500 milliunits/million cells/24 hours.

A 0.2 kb DNA probe extending from the AccI site in hEPO exon 5 to the BglII site in the 3' untranslated region was used to probe genomic DNA from HT165-18A2-10 cells. The targeting construct, pXEPO-13, truncated at the AccI site in exon 5 does not contain these AccI/BglII sequences and, therefore, is diagnostic for targeting at the hEPO locus. Only cell strains that have recombined in a homologous manner with natural hEPO sequences would produce an hEPO mRNA containing sequence homologous to the AccI/BglII sequences. HT16518A2-10 was found to express an mRNA of the predicted size hybridizing with the 32-P labeled AccI/BglII hEPO probe on Northern blots. Restriction enzyme and Southern blot analysis confirmed that the *neo* gene and nMT-I promoter were targeted to one of the two hEPO alleles in HT165-18A2-10 cells.

These results demonstrate that homologous recombination may be used to target a regulatory region to a gene that is normally silent in human fibroblasts, resulting in the functional activation of that gene.

22.1 5′ CACCTAAAAT GATCTCTCTG G (SEQ ID NO 22)

22.2 5′ CGCGCCGGGT GACCACACCG GGGGCCCTAG ATCTGGTGAA
GCTGGAGCTA CGGAGTAA (SEQ ID NO 23)

22.3 5’ TTACTCCGTA GCTCCAGCTT CACCAGATCT AGGGCCCCCG
GTGTGGTCAC CCGGCGCG (SEQ ID NO 24)

22.4 5′ GTCTCACCGT GATATTCTCG G (SEQ ID NO 25)

## Claims

1. A DNA construct that can alter the expression of a targeted gene in a vertebrate cell following homologous recombination with a target site present in DNA of the cell, the DNA construct comprising:
(a) a targeting sequence homologous to the target site,
(b) an exogenous regulatory region for altering said expression,
(c) an exon located downstream of said exogenous regulatory region, and
(d) a splice donor site at the 3' end of the exon;
wherein the construct is such so that following said homologous recombination the DNA of the cell includes the construct-derived exon in addition to all endogenous exons of the targeted gene and, following transcription and splicing to remove RNA between an RNA sequence transcribed from the construct-derived exon and an RNA sequence transcribed from endogenous exon 2 of the targeted gene, RNA can be provided in which said RNA sequence transcribed from the construct-derived exon is positioned adjacent to said RNA sequence transcribed from endogenous exon 2 of the targeted gene.

2. A construct according to claim 1 that is in linear form.

3. A construct according to claim 2 having overhanging ends.

4. A construct according to claim 2 having 3' overhanging ends.

5. A construct according to claim 2 having 5' overhanging ends.

6. A construct according to claim 5, wherein each overhang of an overhanging end is from 100 to 1000 bases long.

7. A construct according to any preceding claim, wherein the exogenous regulatory region comprises one or more of the following: a promoter, an enhancer, a scaffold attachment region, a negative regulatory element, a transcriptional initiation site and/or a regulatory protein binding site.

8. A construct according to any preceding claim, wherein the exogenous regulatory region comprises a cytomegalovirus enhancer.

9. A construct according to any preceding claim, wherein the exogenous regulatory region comprises a promoter (e.g. a mouse metallothionein promoter).

10. A construct according to claim 9, wherein following the homologous recombination referred to in claim 1, the construct-derived promoter is positioned upstream of the promoter of the targeted gene.

11. A construct according to any preceding claim, wherein the construct-derived exon is different from exon 1 of the targeted gene.

12. A construct according to claim 11, wherein the construct-derived exon encodes a sequence that is removed by post-translational processing in said cell.

13. A construct according to any preceding claim, wherein the targeted gene referred to in claim 1 encodes a therapeutic product.

14. A construct according to claim 13, wherein the therapeutic product is Factor VIII, Factor IX, erythropoietin, alpha-1 antitrypsin, calcitonin, glucocerebrosidase, growth hormone, low density lipoprotein (LDL) receptor, apolipoprotein E, IL-2 receptor or an antagonist thereof, insulin, insulinotropin, globin, an immunoglobulin, a catalytic antibody, an interleukin, an insulin-like growth factor, superoxide dismutase, an immune response modifier, parathyroid hormone, an interferon, a nerve growth factor, a tissue plasminogen activator, or a colony stimulating factor.

15. A construct according to claim 13 or 14, wherein the therapeutic product is growth hormone.

16. A construct according to claim 15, wherein the therapeutic product is human growth hormone.

17. A construct according to claim 13 or 14, wherein the therapeutic product is glucocerebrosidase.

18. A construct according to claim 13 or 14, wherein the therapeutic product is erythropoietin.

19. A construct according to claim 18, wherein the therapeutic product is human erythropoietin.

20. A construct according to any preceding claim wherein the construct-derived exon comprises a coding sequence that is the same as that of exon 1 of a growth hormone gene.

21. A construct according to any preceding claim, wherein the targeted gene does not express a gene product, does not express it at significant levels, or expresses it at a level that would benefit from enhancement.

22. A construct according to any preceding claim, wherein the construct can be used to increase expression of a gene product.

23. A construct according to any of claims 1 to 2 1, wherein the construct can be used to decrease expression of a gene product.

24. A construct according to any preceding claim, wherein the construct is non-retroviral.

25. A construct according to any preceding claim, wherein the cell is a mammalian cell.

26. A construct according to any preceding claim, wherein the cell is a human cell.

27. A construct according to any preceding claim, wherein the cell is a fibroblast, a keratinocyte, an epithelial cell, a glial cell, a neural cell, a blood cell, a hepatocyte, a muscle cell; or a precursor of any of the foregoing.

28. A construct according to any preceding claim comprising a selectable marker.

29. A construct according to any preceding claim comprising two selectable markers.

30. A construct according to any preceding claim comprising an inducible promoter.

31. A construct according to any preceding claim comprising a constitutive promoter.

32. An *in vitro* method comprising transfecting a vertebrate cell (optionally an immortalised vertebrate cell) with a construct according to any of claims 1 to 31 and allowing homologous recombination as described in claim 1 to occur.

33. A method according to claim 32 comprising using said cell to express a gene product.

34. A method according to claim 32 or 33 comprising:
1) providing a population of primary cells,
2) introducing into said cells or secondary cells derived therefrom, a construct according to any of claims 1 to 31 and allowing homologous recombination to occur as described in claim 1, so as to produce primary or secondary transfected cells,
3) maintaining said transfected cells under conditions appropriate for their propagation,
4) identifying a transfected primary or secondary cell, and
5) producing a colony from the cell identified in 4) above by maintaining it under appropriate culture conditions and for sufficient time for its propagation.

35. A vertebrate cell comprising DNA having all of the endogenous exons of a given gene, as well as an exogenous exon, a splice donor site at the 3' end of the exogenous exon and an exogenous regulatory region; wherein transcription of said gene is under control of the exogenous regulatory region and, following transcription and splicing to remove RNA between an RNA sequence transcribed from the exogenous exon and an RNA sequence transcribed from endogenous exon 2 of the given gene, RNA can be provided in which an RNA sequence transcribed from the exogenous exon is positioned adjacent to the RNA sequence transcribed from endogenous exon 2 of the given gene.

36. A cell according to claim 35 produced by a method according to any of claims 32 to 34.

37. A cell according to claim 35 or claim 36, wherein said cell is a primary cell, a secondary cell or an immortal cell.

38. A cell according to any of claims 35 or 37, that is a transfected HT 1080 cell, a HeLa cell, an MCF-7 cell, a breast cancer cell, a K-562 leukemia cell, a KB carcinoma cell, or a 2780AD ovarian carcinoma cell.

39. A clonal population of cells comprising a plurality of cells according to any of claims 35 to 38.

40. A heterogenous strain of cells comprising a plurality of cells according to any of claims 35 to 38.

41. A device comprising a plurality of cells according to any of claims 35 to 40 and a barrier that permits a gene product of said cells to pass into a recipient's circulation or tissues, but which prevents an immune response from said recipient causing rejection of said cells.

42. The device according to claim 41, wherein the device is for intraomental implantation.

43. The use of a construct according to any of claims 1 to 31, a method according to any of claims 32 to 34, or of a cell according to any of claims 35 to 40 for the production of a protein *in vitro.*

44. A construct according to any of claims 1 to 31, or a cell according to any of claims 35 to 40, or a device according to claim 41; for use in therapy.

45. A cell or device for use in therapy, as described in claim 44, wherein the therapy comprises intraomental implantation of the cell or device.

46. The use of a construct according to any of claims 1 to 31, or of a cell according to any of claims 3 5 to 40, or of a device according to claim 41, in the preparation of a medicament for treating a disorder that is treatable by altering the expression of a targeted gene.

47. The use of a cell or of a device, as described in claim 46, wherein the cell or device is used in the preparation of a medicament for intraomental implantation.

48. The use according to claim 46 or 47, wherein the disorder is treatable by increasing expression of the gene product.

49. The use according to claim 46 or 47, wherein the disorder is treatable by decreasing expression of the gene product.

50. The use of a construct according to any of claims 1 to 31, of a cell according to any of claims 35 to 40, or of a device according to claim 41, in the preparation of a medicament for gene therapy.

51. The use of a cell or device, as described in claim 50, wherein the cell or device is used in the preparation of a medicament for intraomental implantation.

## Patentansprüche

1. Ein DNA Konstrukt, das nach homologer Rekombination mit einer Zielregion in der Zell-DNA die Expression eines Zielgens in einer Wirbeltierzelle verändern kann,
wobei das DNA Konstrukt
(a) eine zur Zielregion homologe Zielsequenz,
(b) eine exogene regulatorische Region zur Veränderung besagter Expression,
(c) ein stromabwärts von besagter exogener regulatorischer Region gelegenes Exon, und
(d) eine Splicedonorstelle am 3' Ende des Exons umfasst;
wobei das Konstrukt derart gestaltet ist, dass die DNA der Zelle nach besagter homologer Rekombination zusätzlich zu allen endogenen Exons des Zielgenes das vom Konstrukt abgeleitete Exon enthält und, wobei nach Transkription und Splicing zur Entfernung von RNA zwischen einer transkribierten RNA Sequenz, die vom Konstrukt abgeleiteten Exon transkribiert wird und einer RNA Sequenz, die vom endogenen Exon 2 des Zielgens transkribiert wird, RNA zur Verfügung gestellt werden kann, in welcher besagte RNA Sequenz, die vom Konstrukt abgeleiteten Exon transkribiert wird, benachbart zu der RNA Sequenz positioniert ist, die vom endogenen Exon 2 des Zielgens transkribiert wird.

2. Ein Konstrukt gemäß Anspruch 1, das linear ist.

3. Ein Konstrukt gemäß Anspruch 2 mit überhängenden Enden.

4. Ein Konstrukt gemäß Anspruch 2 mit 3' überhängenden Enden.

5. Ein Konstrukt gemäß Anspruch 2 mit 5' überhängenden Enden.

6. Ein Konstrukt gemäß Anspruch 5, worin jeder Überhang eines überhängenden Endes von 100 bis 1000 Basen lang ist.

7. Ein Konstrukt gemäß einem der vorangegangenen Ansprüche, worin die exogene regulatorische Region einen oder mehrere der folgenden umfasst: einen Promotor, einen Verstärker, eine Region, die sich an ein "Scaffold" anlegen kann, ein negatives regulatorisches Element, eine Transkriptionsinitiationsstelle und/oder eine Bindungsstelle für ein regulatorisches Protein.

8. Ein Konstrukt gemäß einem der vorangegangenen Ansprüche, worin die exogene regulatorische Region einen Cytomegalovirus Verstärker umfasst.

9. Ein Konstrukt gemäß einem der vorangegangenen Ansprüche, worin die exogene regulatorische Region einen Promotor (z.B. ein Maus Metallothionein Promotor) umfasst.

10. Ein Konstrukt gemäß Anspruch 9, worin nach der homologen Rekombination gemäß Anspruch 1 der konstruktabgeleitete Promotor stromaufwärts vom Promotor des Zielgens positioniert ist.

11. Ein Konstrukt gemäß einem der vorangegangenen Ansprüche, worin das konstruktabgeleitete Exon vom Exon 1 des Zielgens verschieden ist.

12. Ein Konstrukt gemäß Anspruch 11, worin das konstruktabgeleitete Exon eine Sequenz kodiert, die durch post-translationale Prozessierung in besagter Zelle entfernt wird.

13. Ein Konstrukt gemäß einem der vorangegangenen Ansprüche, worin das in Anspruch 1 genannte Zielgen ein therapeutisches Produkt kodiert.

14. Ein Konstrukt gemäß Anspruch 13, worin das therapeutische Produkt Faktor VIII, Faktor IX, Erythropoetin, alpha-1 Antitrypsin, Calcitonin, Glucocerebrosidase, Wachstumshormon, der Rezeptor des Lipoproteins mit geringer Dichte (LDL), Apolipoprotein E, IL-2 Rezeptor oder ein Antagonist davon, Insulin, Insulinotropin, Globin, ein Immunoglobin, ein katalytischer Antikörper, ein Interleukin, ein Insulin ähnlicher Wachstumsfaktor, Superoxid Dismutase, eine die Immunantwort modifizierende Substanz, Parathyhormon, ein Interferon, ein Nervenwachstumsfaktor, ein Gewebeplasminogenaktivator oder ein Kolonie stimulierender Faktor.

15. Ein Konstrukt gemäß Anspruch 13 oder 14, worin das therapeutische Produkt das Wachstumshormon ist.

16. Ein Konstrukt gemäß Anspruch 15, worin das therapeutische Produkt menschliches Wachstumshormon ist.

17. Ein Konstrukt gemäß Anspruch 13 oder 14, worin das therapeutische Produkt Glucocerebrosidase ist.

18. Ein Konstrukt gemäß Anspruch 13 oder 14, worin das therapeutische Produkt Erythropoetin ist.

19. Ein Konstrukt gemäß Anspruch 18, worin das therapeutische Produkt menschliches Erythropoietin ist.

20. Ein Konstrukt gemäß einem der vorangegangenen Ansprüche, worin das konstruktabgeleitete Exon eine kodierende Sequenz umfasst, die dem Exon 1 des Gens von Wachstumshormon entspricht.

21. Ein Konstrukt gemäß einem der vorangegangenen Ansprüche, worin die Zielsequenz kein Genprodukt exprimiert, ein solches nicht in signifikanten Mengen exprimiert, oder in einer Menge exprimiert, die von einer Verstärkung vorteilhaft unterstützt würde.

22. Ein Konstrukt gemäß einem der vorangegangenen Ansprüche, worin das Konstrukt zur Erhöhung der Expression eines Genprodukts verwendet werden kann.

23. Ein Konstrukt gemäß einem der Ansprüche 1 bis 21, worin das Konstrukt zur Verringerung der Expression eines Genprodukts verwendet werden kann.

24. Ein Konstrukt gemäß einem der vorangegangenen Ansprüche, worin das Konstrukt nicht retroviral ist.

25. Ein Konstrukt gemäß einem der vorangegangenen Ansprüche, worin die Zelle eine Säugetierzelle ist.

26. Ein Konstrukt gemäß einem der vorangegangenen Ansprüche, worin die Zelle eine menschliche Zelle ist.

27. Ein Konstrukt gemäß einem der vorangegangenen Ansprüche, worin die Zelle ein Fibroblastenzelle, ein Keratinozyt, eine Epithelialzelle, eine Gliazelle, eine Nervenzelle, eine Blutzelle, ein Hepatozyt, eine Muskelzelle; oder ein Vorläufer der Vorgenannten ist.

28. Ein Konstrukt gemäß einem der vorangegangenen Ansprüche, das einen selektierbaren Marker umfasst.

29. Ein Konstrukt gemäß einem der vorangegangenen Ansprüche enthaltend zwei selektierbare Marker.

30. Ein Konstrukt gemäß einem der vorangegangenen Ansprüche, das einen induzierbaren Promotor umfasst.

31. Ein Konstrukt gemäß einem der vorangegangenen Ansprüche umfassend einen konstitutiven Promotor.

32. Ein *in vitro* Verfahren umfassend die Transfektion einer Wirbeltierzelle (optional einer immortalisierten Wirbeltierzelle) mit einem Konstrukt gemäß einem der Ansprüche 1 bis 31 und anschließender homologer Rekombination wie in Anspruch 1 beschrieben.

33. Ein Verfahren gemäß Anspruch 32, umfassend die Verwendung der besagten Zelle zur Expression eines Genprodukts.

34. Ein Verfahren gemäß Anspruch 32 oder 33 umfassend:
1) das zur Verfügung stellen einer Population von primären Zellen,
2) das Einführen eines Konstrukts gemäß einem der Ansprüche 1 bis 31 in besagte Zellen oder davon abgeleitete sekundäre Zellen, und anschließende homologe Rekombination wie in Anspruch 1 beschrieben, so dass primäre oder sekundäre transfizierte Zellen hergestellt werden,
3) die Aufbewahrung besagter transfizierter Zellen unter Bedingungen, die zu deren Vermehrung geeignet sind,
4) das Identifizieren einer transfizierten primären oder sekundären Zelle und
5) das Herstellen einer Kolonie aus der in 4) identifizierten Zelle durch die Inkubation dieser Zelle unter geeigneten Kulturbedingungen und für einen zur Vermehrung ausreichenden Zeitraum.

35. Eine Wirbeltierzelle umfassend eine DNA, die alle endogenen Exons eines gegebenen Gens sowie ein exogenes Exon, eine Splicedonorstelle am 3' Ende des exogenen Exons und eine exogene regulatorische Region aufweist; wobei die Transkription des besagten Gens unter der Kontrolle der exogenen regulatorischen Region steht und, nach Transkription und Splicing zur Entfernung von RNA zwischen einer RNA Sequenz, die vom exogenen Exon transkribiert wird und einer RNA Sequenz, die vom endogenen Exon 2 des gegebenen Gens transkribiert wird, RNA zur Verfügung gestellt werden kann, in welcher eine RNA Sequenz, die vom exogenen Exon transkribiert wurde, in benachbarter Position zu einer RNA Sequenz steht, die vom endogenen Exon 2 des gegebenen Gens transkribiert wurde.

36. Eine Zelle gemäß Anspruch 35, hergestellt nach einem Verfahren gemäß einem der Ansprüche 32 bis 34.

37. Eine Zelle gemäß Anspruch 35 oder Anspruch 36, worin diese Zelle eine primäre Zelle, eine sekundäre Zelle oder eine immortalisierte Zelle ist.

38. Eine Zelle gemäß einem der Ansprüche 35 oder 37, die eine transfizierte HT 1080 Zelle, eine HeLa-Zelle, eine MCF-7 Zelle, eine Brustkrebszelle, eine K-562 Leukämiezelle, eine KB Karzinomzelle oder eine 2780AD ovarielle Karzinomzelle ist.

39. Eine klonale Zellpopulation umfassend eine Vielzahl von Zellen gemäß einem der Ansprüche 35 bis 38.

40. Ein heterogener Zellstamm umfassend eine Vielzahl von Zellen gemäß einem der Ansprüche 35 bis 38.

41. Eine Vorrichtung umfassend eine Vielzahl von Zellen gemäß einem der Ansprüche 35 bis 40 und eine Barriere, die es einem Genprodukt dieser Zellen erlaubt, in den Kreislauf oder Gewebe eines Empfängers einzudringen, welche aber eine Immunantwort des Empfängers verhindert, die die Abstoßung dieser Zellen bewirkt.

42. Die Vorrichtung gemäß Anspruch 41, worin die Vorrichtung ein intraomentales Implantat ist.

43. Die Verwendung eines Konstrukts gemäß einem der Ansprüche 1 bis 31, ein Verfahren gemäß einem der Ansprüche 32 bis 34, oder einer Zelle gemäß einem der Ansprüche 35 bis 40 zur Herstellung eines Proteins *in vitro.*

44. Ein Konstrukt gemäß einem der Ansprüche 1 bis 31, oder eine Zelle gemäß einem der Ansprüche 35 bis 40, oder eine Vorrichtung gemäß Anspruch 41 zur therapeutischen Verwendung.

45. Eine Zelle oder Vorrichtung zur therapeutischen Verwendung, wie in Anspruch 44 beschrieben, wobei die Therapie die intraomentale Implantation der Zelle oder der Vorrichtung umfasst.

46. Die Verwendung eines Konstrukts gemäß einem der Ansprüche 1-31, oder einer Zelle gemäß einem der Ansprüche 35 bis 40 oder einer Vorrichtung gemäß Anspruch 41 zur Herstellung eines Medikaments zur Behandlung einer Krankheit, die durch die Veränderung der Expression eines Zielgens behandelbar ist.

47. Die Verwendung einer Zelle oder einer Vorrichtung, wie in Anspruch 46 beschrieben, worin die Zelle oder Vorrichtung zur Herstellung eines Medikaments zur intraomentalen Implantation verwendet wird.

48. Die Verwendung gemäß Anspruch 46 oder 47, worin die Krankheit durch die Erhöhung der Expression eines Genprodukts behandelbar ist.

49. Die Verwendung gemäß Anspruch 46 oder 47, worin die Krankheit durch die Verringerung der Expression des Genprodukts behandelbar ist.

50. Die Verwendung eines Konstrukts gemäß einem der Ansprüche 1 bis 31, einer Zelle gemäß einem der Ansprüche 35 bis 40 oder einer Vorrichtung gemäß Anspruch 41 zur Herstellung eines Medikaments zur Gentherapie.

51. Die Verwendung einer Zelle oder Vorrichtung wie in Anspruch 50 beschrieben, worin die Zelle oder Vorrichtung zur Herstellung eines Medikaments zur intraomentalen Implantation verwendet wird.

## Revendications

1. Produit d'assemblage d'ADN, qui peut altérer l'expression d'un gène cible dans une cellule de vertébré après recombinaison homologue avec un site cible présent dans l'ADN de la cellule, lequel produit d'assemblage d'ADN comprend :
(a) une séquence cible homologue du site cible,
(b) une région régulatrice exogène pour altérer ladite expression,
(c) un exon localisé en aval de ladite région régulatrice exogène, et
(d) un site donneur d'épissage à l'extrémité 3' de l'exon ;
le produit d'assemblage étant tel qu'après ladite recombinaison homologue, l'ADN de la cellule comprenne l'exon issu du produit d'assemblage en plus de tous les exons endogènes du gène cible et, qu'après transcription et épissage pour éliminer l'ARN entre une séquence d'ARN transcrite depuis l'exon issu du produit d'assemblage et une séquence d'ARN transcrite à partir de l'exon endogène 2 du gène cible, puisse être fourni un ARN dans lequel ladite séquence d'ARN transcrite depuis l'exon issu du produit d'assemblage est situé en une position adjacente à ladite séquence d'ARN transcrite depuis l'exon endogène 2 du gène cible.

2. Produit d'assemblage selon la revendication 1, qui est sous forme linéaire.

3. Produit d'assemblage selon la revendication 2, ayant des extrémités débordantes.

4. Produit d'assemblage selon la revendication 2, ayant des extrémités débordantes en 3'.

5. Produit d'assemblage selon la revendication 2, ayant des extrémités débordantes en 5'.

6. Produit d'assemblage selon la revendication 5, dans lequel chaque débordement d'une extrémité débordante a une longueur de 100 à 1000 bases.

7. Produit d'assemblage selon l'une quelconque des revendications précédentes, dans lequel la région régulatrice exogène comprend un ou plusieurs des suivants: un promoteur, un activateur, une région de rattachement à la charpente, un élément régulateur négatif, un site d'initiation de transcription et/ou un site de fixation à une protéine régulatrice.

8. Produit d'assemblage selon l'une quelconque des revendications précédentes, dans lequel la région régulatrice exogène comprend un activateur de cytomégalovirus.

9. Produit d'assemblage selon l'une quelconque des revendications précédentes, dans lequel la région régulatrice exogène comprend un promoteur (par exemple un promoteur de métallothionéine de souris).

10. Produit d'assemblage selon la revendication 9, dans lequel, après la recombinaison homologue indiquée dans la revendication 1, le promoteur issu du produit d'assemblage est positionné en amont du promoteur du gène cible.

11. Produit d'assemblage selon l'une quelconque des revendications précédentes, dans lequel l'exon issu du produit d'assemblage est différent de l'exon 1 du gène cible.

12. Produit d'assemblage selon la revendication 11, dans lequel l'exon issu du produit d'assemblage code une séquence qui est éliminée par traitement post-traductionnel dans ladite cellule.

13. Produit d'assemblage selon l'une quelconque des revendications précédentes, dans lequel le gène cible auquel on se réfère dans la revendication 1 code un produit thérapeutique.

14. Produit d'assemblage selon la revendication 13, dans lequel le produit thérapeutique est le Facteur VIII, le Facteur IX, l'érythropoïétine, l'α-1-antitrypsine, la calcitonine, la glucocérébrosidase, l'hormone de croissance, le récepteur de lipoprotéine basse densité (LDL), l'apolipoprotéine E, le récepteur de IL-2 ou un antagoniste de celui-ci, l'insuline, l'insulinotropine, la globine, une immunoglobuline, un anticorps catalytique, une interleukine, un facteur de croissance de type insuline, la superoxyde dismutase, un agent modifiant la réponse immunitaire, l'hormone parathyroïdienne, un interféron, un facteur de croissance des nerfs, un activateur du plasminogène tissulaire, ou un facteur stimulant les colonies.

15. Produit d'assemblage selon la revendication 13 ou 14, dans lequel le produit thérapeutique est l'hormone de croissance.

16. Produit d'assemblage selon la revendication 15, dans lequel le produit thérapeutique est l'hormone de croissance humaine.

17. Produit d'assemblage selon la revendication 13 ou 14, dans lequel le produit thérapeutique est la glucocérébrosidase.

18. Produit d'assemblage selon la revendication 13 ou 14, dans lequel le produit thérapeutique est l'érythropoïétine.

19. Produit d'assemblage selon la revendication 18, dans lequel le produit thérapeutique est l'érythropoïétine humaine.

20. Produit d'assemblage selon l'une quelconque des revendications précédentes, dans lequel l'exon issu du produit d'assemblage comprend une séquence codante qui est la même que celle de l'exon 1 d'un gène d'hormone de croissance.

21. Produit d'assemblage selon l'une quelconque des revendications précédentes, dans lequel le gène cible n'exprime pas de produit génique, ne l'exprime pas à des taux significatifs, ou l'exprime à un taux qui va tirer avantage d'une activation.

22. Produit d'assemblage selon l'une quelconque des revendications précédentes, lequel peut être utilisé pour augmenter l'expression d'un produit génique.

23. Produit d'assemblage selon l'une quelconque des revendications 1 à 21, lequel peut être utilisé pour diminuer l'expression d'un produit génique.

24. Produit d'assemblage selon l'une quelconque des revendications précédentes, lequel est non-rétroviral.

25. Produit d'assemblage selon l'une quelconque des revendications précédentes, dans lequel la cellule est une cellule de mammifère.

26. Produit d'assemblage selon l'une quelconque des revendications précédentes, dans lequel la cellule est une cellule humaine.

27. Produit d'assemblage selon l'une quelconque des revendications précédentes, dans lequel la cellule est un fibroblaste, un kératinocyte, une cellule épithéliale, une cellule gliale, une cellule neurale, une cellule sanguine, un hépatocyte, une cellule musculaire ; ou un précurseur de l'un quelconque des précédents.

28. Produit d'assemblage selon l'une quelconque des revendications précédentes, comprenant un marqueur sélectionnable.

29. Produit d'assemblage selon l'une quelconque des revendications précédentes, comprenant deux marqueurs sélectionnables.

30. Produit d'assemblage selon l'une quelconque des revendications précédentes, comprenant un promoteur inductible.

31. Produit d'assemblage selon l'une quelconque des revendications précédentes, comprenant un promoteur constitutif.

32. Procédé in vitro comprenant les étapes consistant à transfecter une cellule de vertébré (éventuellement d'une cellule de vertébré immortalisée) avec un produit d'assemblage selon l'une quelconque des revendications 1 à 31 et à laisser se produire une recombinaison homologue telle que décrite dans la revendication 1.

33. Procédé selon la revendication 32, comprenant l'utilisation de ladite cellule pour exprimer un produit génique.

34. Procédé selon la revendication 32 ou 33, comprenant les étapes consistant à :
1) se procurer une population de cellules primaires,
2) introduire dans lesdites cellules ou dans les cellules secondaires en dérivant, un produit d'assemblage selon l'une quelconque des revendications 1 à 31, et laisser se produire une recombinaison homologue telle que décrite dans la revendication 1, de façon à produire des cellules transfectées primaires ou secondaires,
3) maintenir lesdites cellules transfectées dans des conditions appropriées pour leur propagation,
4) identifier une cellule primaire ou secondaire transfectée, et
5) produire une colonie à partir de la cellule identifiée en 4) ci-dessus en la maintenant dans des conditions de culture appropriées et pendant un temps suffisant pour sa propagation.

35. Cellule de vertébré comprenant un ADN ayant la totalité des exons endogènes d'un gène donné, ainsi qu'un exon exogène, un site donneur d'épissage à l'extrémité 3' de l'exon exogène et une région régulatrice exogène ; dans laquelle la transcription dudit gène est sous le contrôle de la région régulatrice exogène et, après transcription et épissage pour retirer l'ARN entre une séquence d'ARN transcrite depuis l'exon exogène et une séquence d'ARN transcrite depuis l'exon endogène 2 du gène donné, peut être mis à disposition un ARN dans lequel une séquence d'ARN transcrite depuis l'exon exogène est en position adjacente à la séquence d'ARN transcrite depuis l'exon endogène 2 du gène donné.

36. Cellule selon la revendication 35, produite par un procédé selon l'une quelconque des revendications 32 à 34.

37. Cellule selon la revendication 35 ou la revendication 36, laquelle est une cellule primaire, une cellule secondaire ou une cellule immortelle.

38. Cellule selon l'une quelconque des revendications 35 et 37, qui est une cellule HT 1080 transfectée, une cellule HeLa, une cellule MCF-7, une cellule de cancer du sein, une cellule de leucémie K-562, une cellule de carcinome KB, ou une cellule de carcinome ovarien 2780AD.

39. Population clonale de cellules comprenant une pluralité de cellules selon l'une quelconque des revendications 35 à 38.

40. Souche hétérogène de cellules comprenant une pluralité de cellules selon l'une quelconque des revendications 35 à 38.

41. Dispositif comprenant une pluralité de cellules selon l'une quelconque des revendications 35 à 40 et une barrière qui permet à un produit génique desdites cellules de passer dans la circulation ou les tissus d'un receveur, mais qui empêche une réponse immunitaire provenant dudit receveur provoquant un rejet desdites cellules.

42. Dispositif selon la revendication 41, lequel est destiné à une implantation intra-omentale.

43. Utilisation d'un produit d'assemblage selon l'une quelconque des revendications 1 à 31, d'un procédé selon l'une quelconque des revendications 32 à 34 ou d'une cellule selon l'une quelconque des revendications 35 à 40 pour la production d'une protéine in vitro.

44. Produit d'assemblage selon l'une quelconque des revendications 1 à 31, ou cellule selon l'une quelconque des revendications 35 à 40, ou dispositif selon la revendication 41 ; pour une utilisation en thérapie.

45. Cellule ou dispositif pour une utilisation en thérapie, telle (tel) que décrit(e) dans la revendication 44, pour laquelle (lequel) la thérapie comprend une implantation intra-omentale de la cellule ou du dispositif.

46. Utilisation d'un produit d'assemblage selon l'une quelconque des revendications 1 à 31, ou d'une cellule selon l'une quelconque des revendications 35 à 40, ou d'un dispositif selon la revendication 41, dans la préparation d'un médicament pour traiter un trouble qui peut être traité par altération de l'expression d'un gène cible.

47. Utilisation d'une cellule ou d'un dispositif, telle que décrite dans la revendication 46, dans laquelle la cellule ou le dispositif est utilisé dans la préparation d'un médicament destiné à une implantation intra-omentale.

48. Utilisation selon la revendication 46 ou 47, dans laquelle le trouble peut être traité par augmentation de l'expression du produit génique.

49. Utilisation selon la revendication 46 ou 47, dans laquelle le trouble peut être traité par diminution de l'expression du produit génique.

50. Utilisation d'un produit d'assemblage selon l'une quelconque des revendications 1 à 31, d'une cellule selon l'une quelconque des revendications 35 à 40, ou d'un dispositif selon la revendication 41, dans la préparation d'un médicament destiné à une thérapie génique.

51. Utilisation d'une cellule ou d'un dispositif, telle que décrite dans la revendication 50, dans laquelle la cellule ou le dispositif est utilisé dans la préparation d'un médicament destiné à une implantation intra-omentale.
